# EUROPEAN PATENT APPLICATION

(11) **EP 4 847 511 A1**
(43) Date of publication of application: **02.12.2026**
(21) Application number: 24926327.8
(22) Date of filing: 27.02.2024
(51) Int. Cl.: C07D 487/06, C07D 471/06, C07D 519/00, A61K 31/519, A61K 31/4745, A61P 35/00

(54) **COMPOUND WITH TRICYCLIC STRUCTURE, PHARMACEUTICAL COMPOSITION THEREOF AND USE THEREOF**

(71) Applicant: Shanghai Yingli Pharmaceutical Co., Ltd, Shanghai 201203 (CN)
(72) Inventor: XU, Zusheng, Shanghai 201201 (CN); XIA, Lin, Shanghai 201203 (CN); YANG, Haoran, Shanghai 201203 (CN)
(74) Representative: Vogelbruch, Keang
(86) International application number: PCT/CN2024/078673
(87) International publication number: WO 2025/179441

(57) **Abstract**

Disclosed in the present invention are a compound with a tricyclic structure, a pharmaceutical composition thereof and the use thereof. The compound with a tricyclic structure of the present invention comprises a compound with a tricyclic structure as shown in formula (I), a pharmaceutically acceptable salt thereof, a solvate thereof, a solvate of the pharmaceutically acceptable salt thereof, a crystal form thereof, a stereoisomer thereof, a tautomer thereof or an isotopically labeled compound thereof. The compound with a tricyclic structure as shown in formula (I) of the present invention has a relatively good inhibitory effect on cells of various PI3K-meditated diseases, and is expected to be used for treating and/or preventing the various PI3K-meditated diseases.

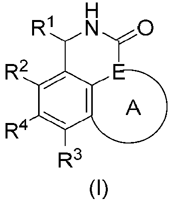

## Description

### TECHNICAL FIELD

The present invention relates to a compound with a tricyclic structure (tricyclic fused ring structure), a pharmaceutical composition thereof and the use thereof.

### BACKGROUND

Cancer has become a frequent and common disease that poses a serious threat to human health and life. PI3K/AKt/mTOR signaling pathway is one of the main mechanisms that regulate the cell cycle and apoptosis, and dysregulation of one component of the pathway can lead to tumor development. Receptor tyrosine kinase (RTK) regulates the activation of the PI3K/AKt signaling pathway in response to activation of growth factors, including Insulin-like Growth Factors (IGF), Epidermal Growth Factor (EGF) and Hepatocyte Growth Factors (HGF), activate RTK by phosphorylating tyrosine residues, while PI3K binds to phosphorylated tyrosine residues to activate the catalytic subunit of PI3K. p110α, the catalytic subunit of type IA PI3K, activates PI3K by binding to p85α, which further phosphorylates phosphatidylinositol 4,5-bisphosphate (PIP2) to phosphatidylinositol 3,4,5-trisphosphate (PIP3). PIP3, as an important second messenger and mediator, recruits AKt from the cytoplasm to the membrane through its interactions with the PH domains of AKt. It mediates the membrane translocation of Akt, 3-phosphoinositide-dependent protein kinase 1 (PDK1) and 3-phosphoinositide-dependent protein kinase 2 (PDK2) phosphorylate the Thr308 and Ser473 sites, respectively, which is essential for Akt activation. Fully activated AKt regulates cell proliferation and apoptosis through its upstream and downstream regulation. Dysregulation of the PI3K signaling pathway is involved in almost all human cancers. PIK3CA (encoding the PI3K catalytic subunit α) is one of the most commonly mutated oncogenes in human tumors. Mutations in the oncogene PIK3CA have been found in about 2-5% of human solid tumors, with mutation rates of about 32%, 27%, 25%, 8%, and 4% in colon, glioblastoma, gastric, breast, and lung cancers, respectively, and 11% in esophageal squamous carcinoma and 6% in esophageal adenocarcinoma, amoung other gastrointestinal tumors.

### SUMMARY

The technical problem to be solved by the present invention is the lack of effective drugs acting as PI3K inhibitor for clinical therapy in the prior art. To this end, the present invention provides a compound with a tricyclic fused ring structure, a pharmaceutical composition thereof and the use thereof, wherein the compound with a tricyclic fused ring structure is expected to be used for treating and/or preventing various PI3K-meditated diseases.

The present invention solves the aforementioned technical problem through the following technical solutions. The present invention provides a compound with a tricyclic fused ring structure as shown in formula I, a pharmaceutically acceptable salt thereof, a solvate thereof, a solvate of the pharmaceutically acceptable salt thereof, a crystal form thereof, a stereoisomer thereof, a tautomer thereof or an isotopically labeled compound thereof: 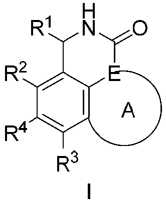 wherein,
E is C or N;
ring A is a 4- to 10-membered cycloalkene containing 1-3 heteroatoms each independently selected from O, N and S, or a 5- to 12-membered heteroaromatic ring containing 1-3 heteroatoms each independently selected from O, N and S substituted by one or more C₁₋₆ alkyl;
R¹ is phenyl substituted by at least two R¹⁻¹; R¹⁻¹ is independently halogen or C₁₋₆ alkyl;
R² is C₆₋₁₀ aryl substituted by one or more R²⁻¹, or benzofuranyl substituted by one or more R²⁻²; each R²⁻¹ and R²⁻² is independently halogen, C₁₋₆ alkyl, or C₁₋₆ alkyl substituted by one or more halogen;
R³ is 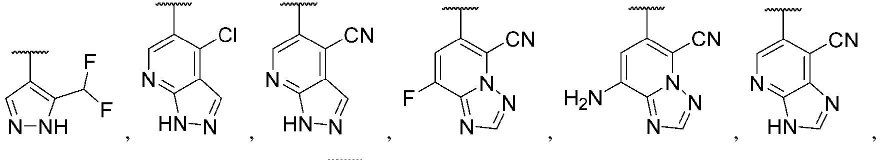 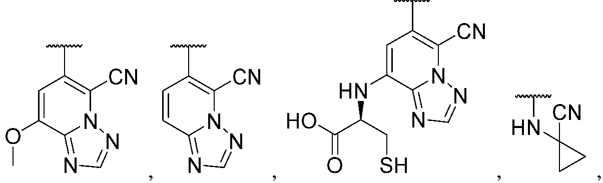 Cl, F, Br, -NH₂, H, 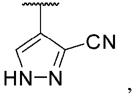 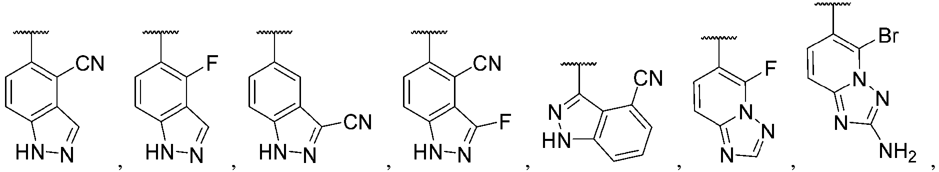 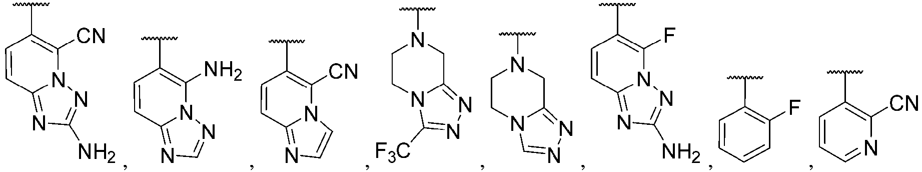 or 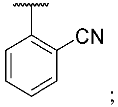
R⁴ is hydrogen or halogen;
said compound with a tricyclic fused ring structure as shown in formula I is not any one of the following compounds:
   N-(7-bromo-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
   N-(7-amino-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
   N-(7-bromo-1-(2-bromo-5-fluorophenyl)-3-oxo-2,3,5,6-tetrahydro-1H-pyrrolo[3,2,1-ij]quinazolin-9-yl)-3-fluoro-5-(trifluoromethyl)benzamide;
   N-(7-bromo-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
   N-(7-amino-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
   N-(7-amino-1-(2-chloro-5-(trifluoromethyl)phenyl)-3-oxo-2,3,5,6-tetrahydro-1H-pyrrolo[3,2,1-ij]quinazolin-9-yl)-3-fluoro-5-(trifluoromethyl)benzamide;
   N-(7-amino-1-(2,5-dichloro-4-fluorophenyl)-3-oxo-2,3,5,6-tetrahydro-1H-pyrrolo[3,2,1-ij]quinazolin-9-yl)-3-fluoro-5-(trifluoromethyl)benzamide;
   N-(5-(2-chloro-5-fluorophenyl)-2-methyl-3-oxo-2,3,4,5-tetrahydropyrazolo[3,4,5-de]isoquinolin-6-yl)-3-fluoro-5-(trifluoromethyl)benzamide;
   N-(5-(2,5-dichlorophenyl)-2-methyl-3-oxo-2,3,4,5-tetrahydropyrazolo[3,4,5-de]isoquinolin-6-yl)-3-fluoro-5-(trifluoromethyl)benzamide;
   N-(5-(2-chloro-5-fluorophenyl)-8-(5-cyano-[1,2,4]triazolo[1,5-a]pyridin-6-yl)-2-methyl-3-oxo-2,3,4,5-tetrahydropyrazolo[3,4,5-de]isoquinolin-6-yl)-3-fluoro-5-(trifluoromethyl)benzamide;
   N-(8-(5-cyano-[1,2,4]triazolo[1,5-a]pyridin-6-yl)-5-(2,5-dichlorophenyl)-2-methyl-3-oxo-2,3,4,5-tetrahydropyrazolo[3,4,5-de]isoquinolin-6-yl)-3-fluoro-5-(trifluoromethyl)benzamide;
   N-(1-(2-chloro-5-fluorophenyl)-7-(5-cyano-[1,2,4]triazolo[1,5-a]pyridin-6-yl)-3-oxo-2,3,5,6-tetrahydro-1H-pyrrolo[3,2,1-ij]quinazolin-9-yl)-3-fluoro-5-(trifluoromethyl)benzamide;
   N-(7-(5-cyano-[1,2,4]triazolo[1,5-a]pyridin-6-yl)-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
   N-(7-(5-cyano-[1,2,4]triazolo[1,5-a]pyridin-6-yl)-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
   N-(1-(2-chloro-5-fluorophenyl)-7-(5-cyano-[1,2,4]triazolo[1,5-a]pyridin-6-yl)-3-oxo-2,3,5,6-tetrahydro-1H-pyrrolo[3,2,1-ij]quinazolin-9-yl)-3-fluoro-5-(trifluoromethyl)benzamide;
   N-(5-(2,5-dichloro-4-fluorophenyl)-2-methyl-3-oxo-2,3,4,5-tetrahydropyrazolo[3,4,5-de]isoquinolin-6-yl)-3-fluoro-5-(trifluoromethyl)benzamide;
   N-(1-(2-chloro-5-fluorophenyl)-7-(3-cyano-1H-pyrazol-4-yl)-3-oxo-2,3,5,6-tetrahydro-1H-pyrrolo[3,2,1-ij]quinazolin-9-yl)-3-fluoro-5-(trifluoromethyl)benzamide;
   N-(1-(2-chloro-5-fluorophenyl)-7-(5-fluoro-[1,2,4]triazolo[1,5-a]pyridin-6-yl)-3-oxo-2,3,5,6-tetrahydro-1H-pyrrolo[3,2,1-ij]quinazolin-9-yl)-3-fluoro-5-(trifluoromethyl)benzamide;
   N-(7-(2-cyanopyridin-3-yl)-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
   N-(1-(2-chloro-5-fluorophenyl)-7-(2-cyanopyridin-3-yl)-3-oxo-2,3,5,6-tetrahydro-1H-pyrrolo[3,2,1-ij]quinazolin-9-yl)-3-fluoro-5-(trifluoromethyl)benzamide;
   N-(7-(5-amino-[1,2,4]triazolo[1,5-a]pyridin-6-yl)-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
   N-(1-(2-chloro-5-fluorophenyl)-7-(3-cyano-1H-pyrazol-4-yl)-3-oxo-2,3,5,6-tetrahydro-1H-pyrrolo[3,2,1-ij]quinazolin-9-yl)-3-fluoro-5-(trifluoromethyl)benzamide;
   N-(7-(5-amino-[1,2,4]triazolo[1,5-a]pyridin-6-yl)-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
   N-(1-(2-chloro-5-fluorophenyl)-7-(4-cyano-1H-indazol-5-yl)-3-oxo-2,3,5,6-tetrahydro-1H-pyrrolo[3,2,1-ij]quinazolin-9-yl)-3-fluoro-5-(trifluoromethyl)benzamide.

In one embodiment, the structure of the compound with a tricyclic fused ring structure as shown in formula I is as shown in formula II: 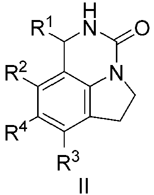 wherein,
R¹ is phenyl substituted with at least two R¹⁻¹; R¹⁻¹ is independently halogen or C₁₋₆ alkyl;
R² is C₆₋₁₀ aryl substituted by one or more R²⁻¹, or benzofuranyl substituted by one or more R²⁻²; each R²⁻¹ and R²⁻² is independently halogen, C₁₋₆ alkyl, or C₁₋₆ alkyl substituted by one or more halogen;
R³ is 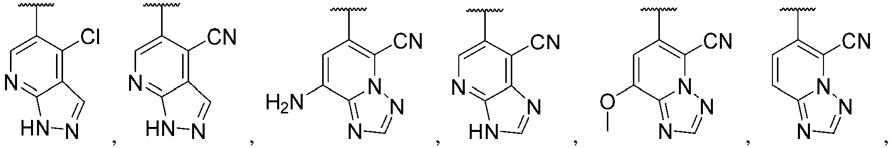 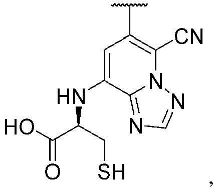 F, Br, -NH₂, 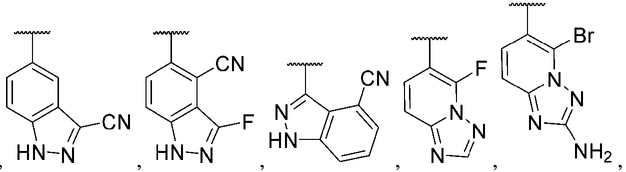 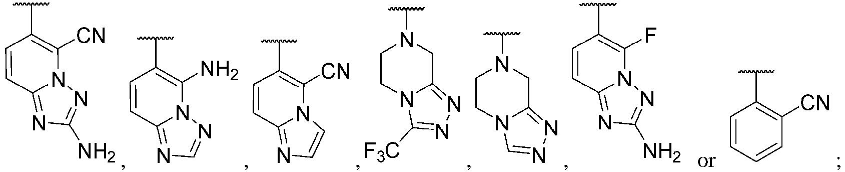
R⁴ is hydrogen or halogen.

In one embodiment, the structure of the compound with a tricyclic fused ring structure as shown in formula I is as shown in formula III: 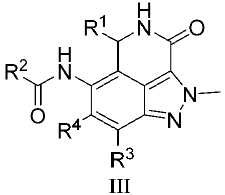 wherein,
R¹ is phenyl substituted by at least two R¹⁻¹; R¹⁻¹ is independently halogen or C₁₋₆ alkyl;
R² is C₆₋₁₀ aryl substituted by one or more R²⁻¹, or benzofuranyl substituted by one or more R²⁻²; each R²⁻¹ and R²⁻² is independently halogen, C₁₋₆ alkyl, or C₁₋₆ alkyl substituted by one or more halogen;
R³ is 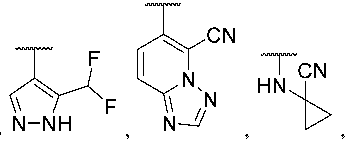 Cl, F, H, 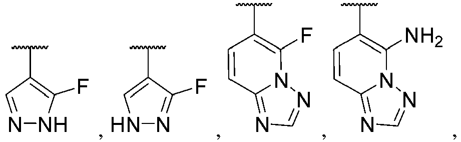 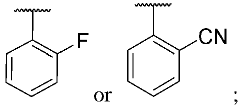
R⁴ is hydrogen or halogen.

In one embodiment, when the definition of R¹⁻¹, R²⁻¹, R²⁻² and R⁴ refers to "halogen", said halogen is fluorine, chlorine, bromine or iodine.

In one embodiment, when the definition of ring A, R¹⁻¹, R²⁻¹ and R²⁻² refers to "C₁₋₆ alkyl", said C₁₋₆ alkyl is C₁₋₄ alkyl, further selected from methyl, ethyl, n-propyl, isopropyl, n-butyl, sec-butyl, isobutyl or tert-butyl. In one embodiment, when ring A is "a 4- to 10-membered cycloalkene containing 1-3 heteroatoms each independently selected from O, N and S", said ring A is "a 4- to 6-membered cycloalkene containing 1-3 N heteroatoms", for example, "a 5-membered cycloalkene containing one N heteroatom".

In one embodiment, when ring A is "a 5- to 12-membered heteroaromatic ring containing 1-3 heteroatoms each independently selected from O, N and S" substituted by one or more C₁₋₆ alkyl, said ring A is "a 5- to 6-membered heteroaromatic ring containing 1-3 heteroatoms each independently selected from O, N and S" substituted by one or more C₁₋₆ alkyl, for example, a pyrazole ring substituted by one or more C₁₋₆ alkyl.

In one embodiment, R¹ is

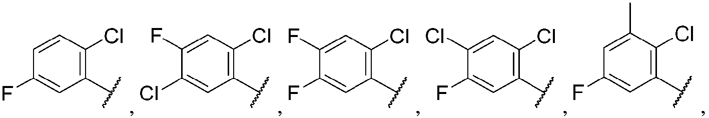

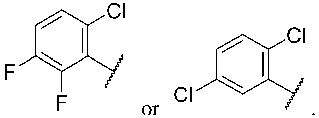

In one embodiment, R² is

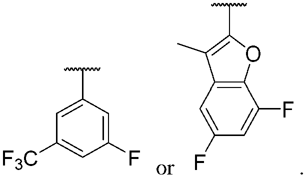

In one embodiment, R⁴ is hydrogen or fluorine.

In one embodiment, said compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof is any one of the following structures:

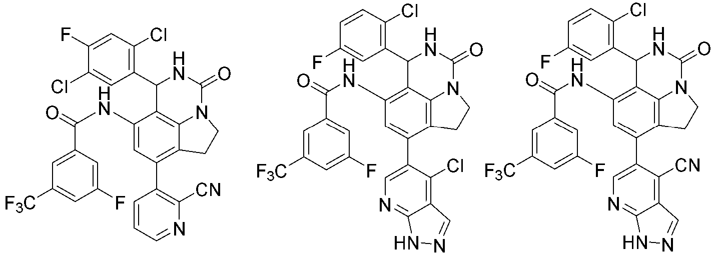

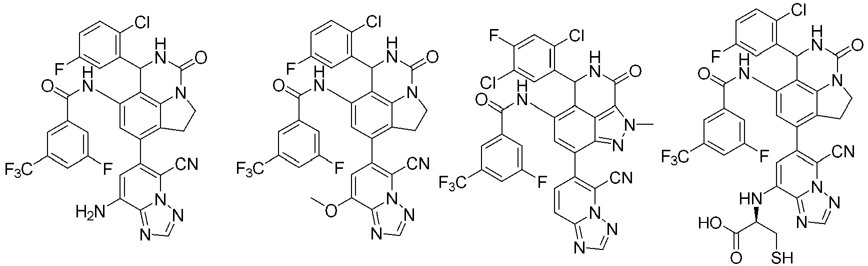

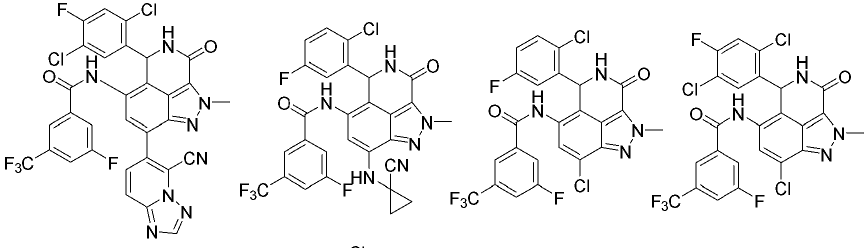

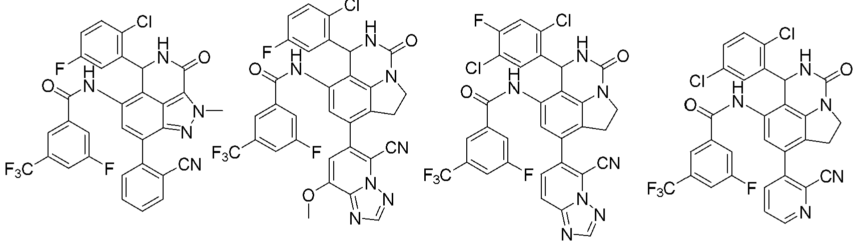

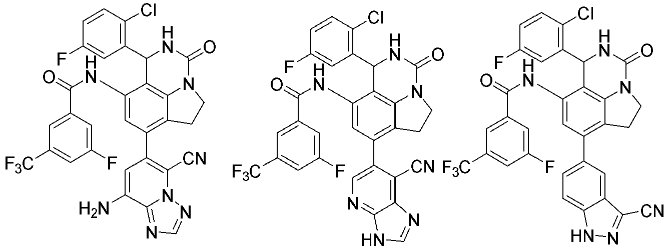

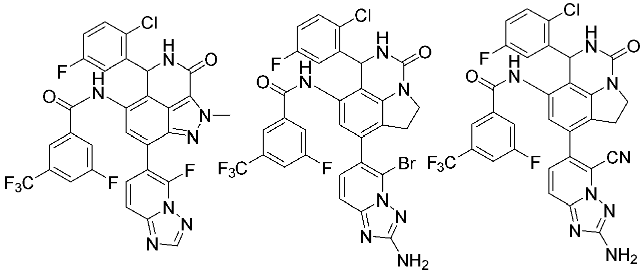

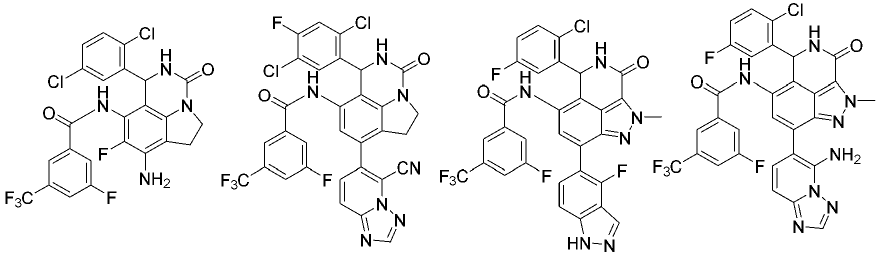

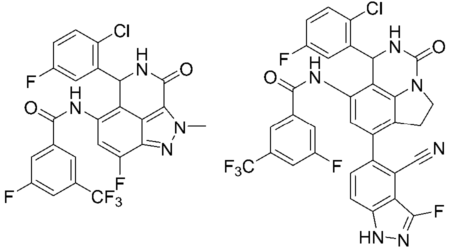

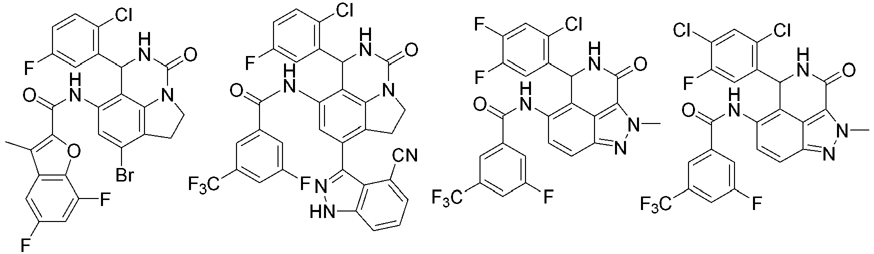

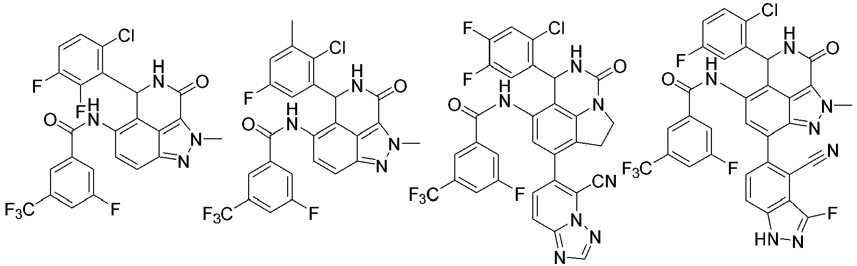

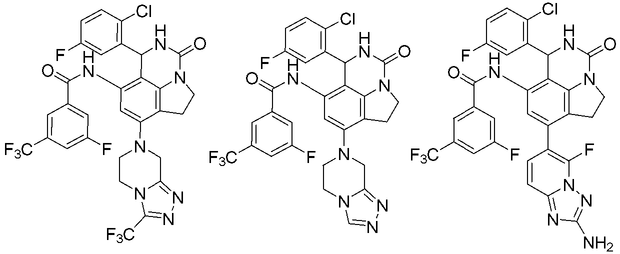

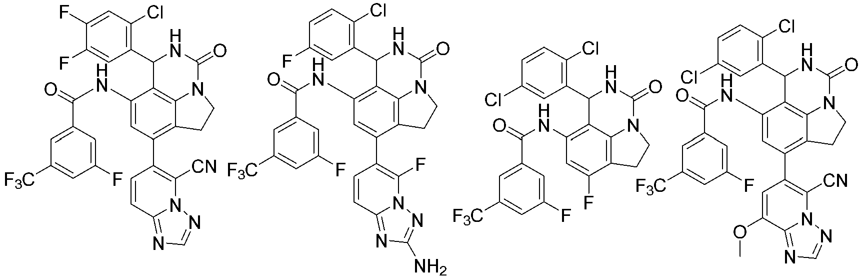

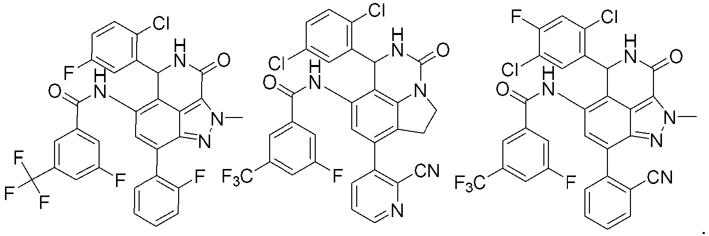

In one embodiment, said compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof is any one of the following structures:
compound 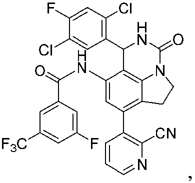 which has a retention time of 0.693 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 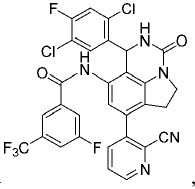 which has a retention time of 1.326 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 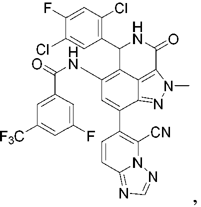 which has a retention time of 3.379 min under the following conditions: equipments: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 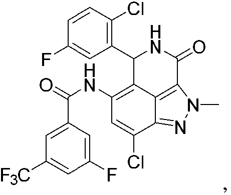 which has a retention time of 1.543 min under the following conditions: equipments: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 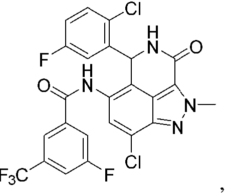 which has a retention time of 3.246 min under the following conditions: equipments: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 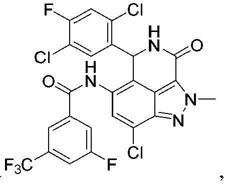 which has a retention time of 3.833 min under the following conditions: equipments: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 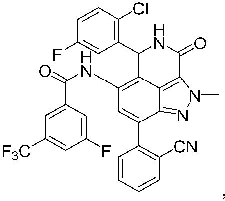 which has a retention time of 0.987 min under the following conditions: equipments: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 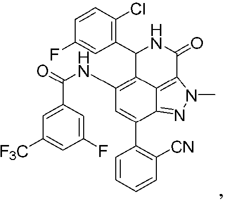 which has a retention time of 3.033 min under the following conditions: equipments: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 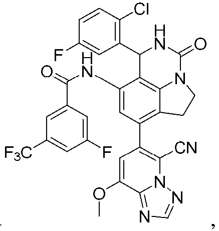 which has a retention time of 1.235 min under the following conditions: equipments: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 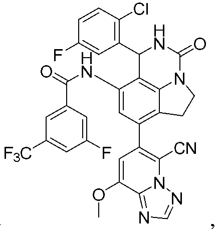 which has a retention time of 4.026 min under the following conditions: equipments: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 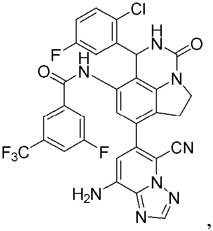 which has a retention time of 3.381 min under the following conditions: equipments: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 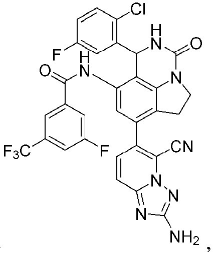 which has a retention time of 1.35 min under the following conditions: equipments: SFC-150 (Waters); chromatographic column: (R, R)Whelk-O1 4.6*100 mm 5 µm; flow rate: 3.0 mL/min; back pressure: 2000 psi; detection wavelength: 254 nm;
compound 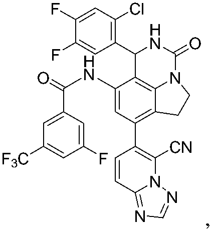 which has a retention time of 2.617 min under the following conditions: instrument: SFC-150 (Waters); chromatographic column: (R, R)Whelk-O1 4.6*100mm 5um; mobile phase: CO₂/EtOH = 70/30; flow rate: 2.5 mL/min; back pressure: 100 bar; detection wavelength: 254 nm;
compound 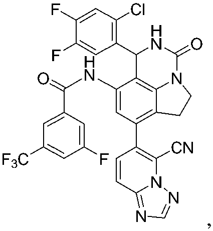 which has a retention time of 3.677 min under the following conditions: instrument: AD-3, 0.46 cm I.D. * 5 cm L; mobile phase: CO₂/EtOH = 70/30; flow rate: 2.5 mL/min; back pressure: 100 bar; detection wavelength: 254 nm;
compound 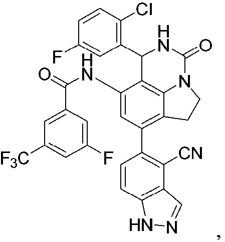 which has a retention time of 1.344 min under the following conditions: equipments: SFC-150 (Waters); chromatographic column: AD-3, 0.46 cm I.D. *5 cm. L; mobile phase: CO₂/EtOH = 70/30; flow rate: 2.5 mL/min; back pressure: 100 bar; detection wavelength: 254 nm;
compound 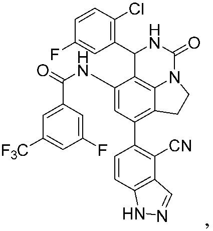 which has a retention time of 3.123 min under the following conditions: equipments: SFC-150 (Waters); chromatographic column: AD-3, 0.46 cm I.D.* 5 cm. L; mobile phase: CO₂/EtOH = 70/30; flow rate: 2.5 mL/min; back pressure: 100 bar; detection wavelength: 254 nm;
compound 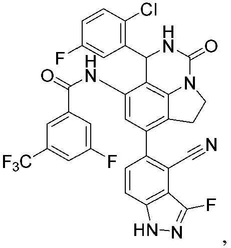 which has a retention time of 2.456 min under the following conditions: equipments: SFC-150 (Waters); chromatographic column: AD-3, 0.46 cm I.D.* 5 cm. L; mobile phase: CO₂/EtOH = 70/30; flow rate: 2.5 mL/min; back pressure: 100 bar; detection wavelength: 254 nm;
compound 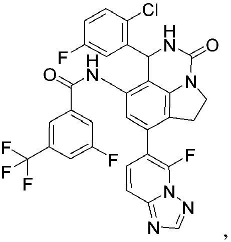 which has a retention time of 1.486 min under the following conditions: equipments: AD-3, 0.46 cm I.D.*5 cm. L; mobile phase: MeOH [0.2%NH₃(7M in MeOH)]; flow rate: 2.5 mL/min; back pressure: 100 bar; detection wavelength: 254 nm;
compound 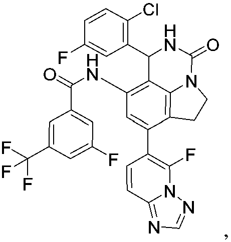 which has a retention time of 3.136 min under the following conditions: equipments: AD-3, 0.46 cm I.D.*5 cm. L; mobile phase: MeOH [0.2%NH₃(7M in MeOH)]; flow rate: 2.5 mL/min; back pressure: 100 bar; detection wavelength: 254 nm;
Compound 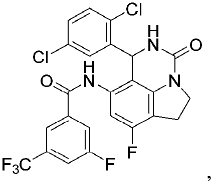 which has a retention time of 1.305 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; detection wavelength: 214 nm;
compound 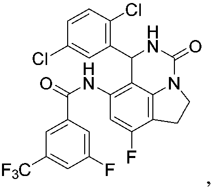 which has a retention time of 1.712 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; detection wavelength: 214 nm;
compound 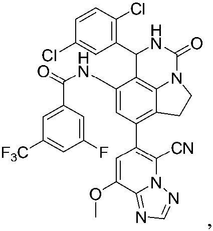 which has a retention time of 0.938 min under the following conditions: equipments: SFC-150 (Waters); chromatographic column: OD-3 4.6*100 mm 3 µm; mobile phase: MeOH[0.2%NH₃(7M in MeOH)]; flow rate: 3.0 mL/min; back pressure: 2000 psi; detection wavelength: 254 nm;
compound 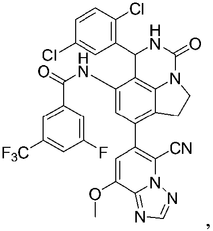 which has a retention time of 1.306 min under the following conditions: equipments: SFC-150 (Waters); chromatographic column: OD-3 4.6*100mm 3 µm; mobile phase: MeOH[0.2%NH₃(7M in MeOH)]; flow rate: 3.0 mL/min; back pressure: 2000 psi; detection wavelength: 254 nm;
compound 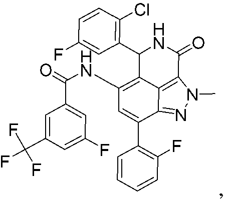 which has a retention time of 2.002 min under the following conditions: equipments: SFC-150 (Waters); chromatographic column: AD-3; mobile phase: CO₂/EtOH = 70/30; flow rate: 2.5 mL/min; back pressure: 2000 psi; detection wavelength: 254 nm;
compound 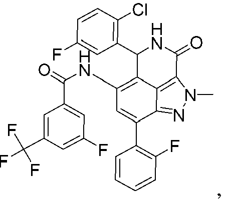 which has a retention time of 3.317 min under the following conditions: equipments: SFC-150 (Waters); chromatographic column: AD-3; mobile phase: CO₂/EtOH = 70/30; flow rate: 2.5 mL/min; back pressure: 2000 psi; detection wavelength: 254 nm;
compound 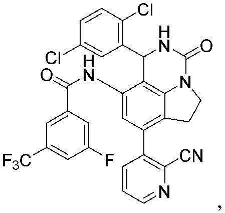 which has a retention time of 2.387 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 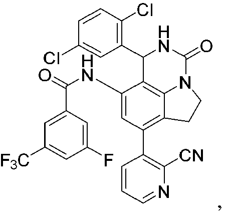 which has a retention time of 4.086 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 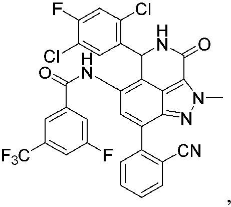 which has a retention time of 2.223 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 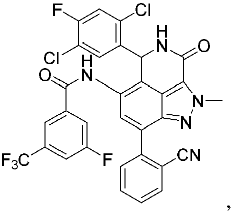 which has a retention time of 3.679 min under the following conditions: equipments: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm.

In one embodiment, the aforementioned compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof is any one of the following structures:

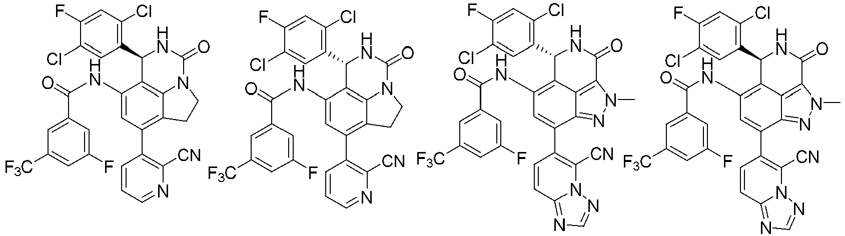

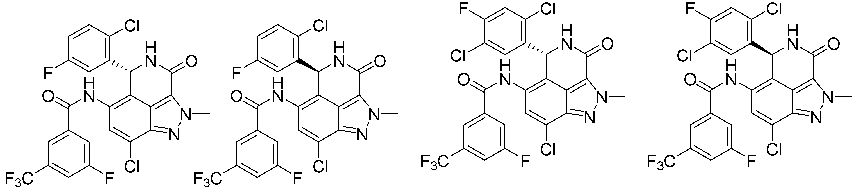

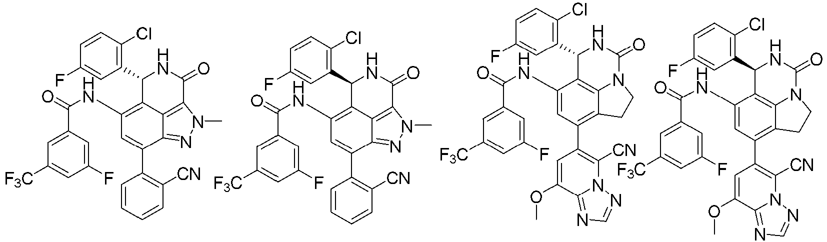

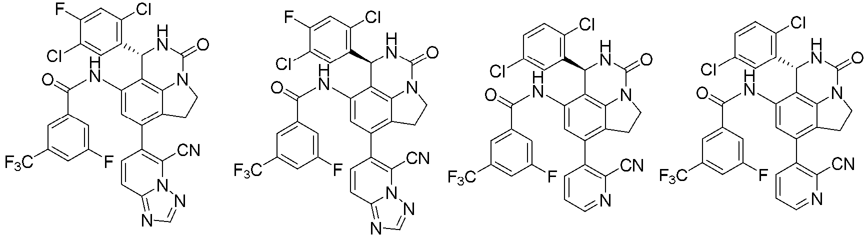

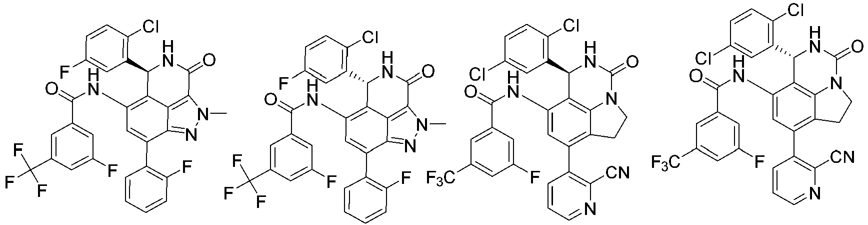

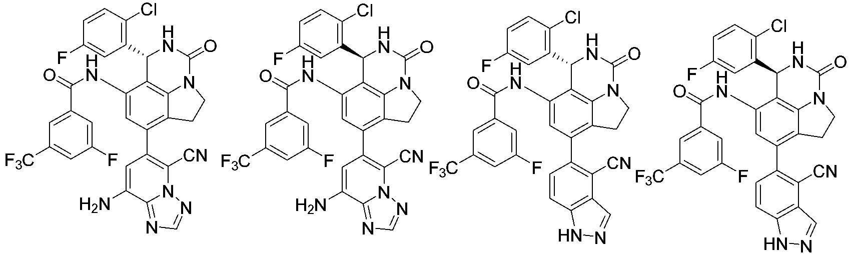

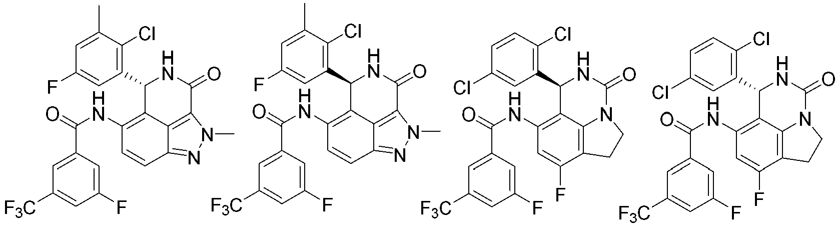

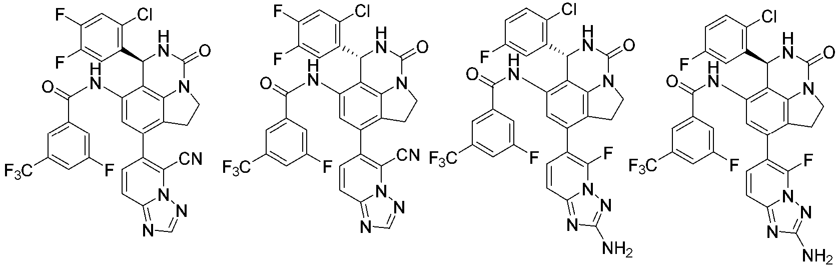

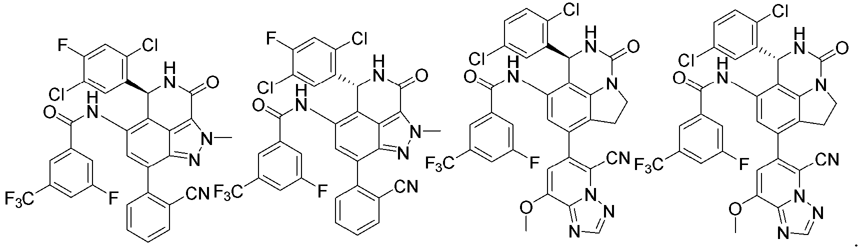

The present invention further provides a pharmaceutical composition, comprising a substance A and pharmaceutical adjuvant; wherein, said substance A is a therapeutically effective amount of the aforementioned compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof.

The present invention further provides an use of a substance A as a PI3K inhibitor, wherein, said substance A is the aforementioned compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof.

The present invention further provides an use of a substance A in the preparation of a medicament, wherein, said medicament is used for treating or preventing PI3K-mediated diseases; said substance A is the aforementioned compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof.

The term "pharmaceutically acceptable salt" refers to a salt prepared from a compound of the present disclosure and a relatively non-toxic, pharmaceutically acceptable acid or base.When the compounds of the present disclosure contain relatively acidic functional groups, base addition salts can be obtained by contacting the neutral form of such compounds with a sufficient amount of a pharmaceutically acceptable base in a pure solution or a suitable inert solvent. Pharmaceutically acceptable base addition salts include, but are not limited to, lithium salt, sodium salt, potassium salt, calcium salt, aluminum salt, magnesium salt, zinc salt, bismuth salt, ammonium salt and diethanolamine salt.When the compounds of the present disclosure contain relatively basic functional groups, acid addition salts can be obtained by contacting the neutral form of such compounds with a sufficient amount of a pharmaceutically acceptable acid in a pure solution or a suitable inert solvent. The said pharmaceutically acceptable acids include inorganic acids, which include, but are not limited to, hydrochloric acid, hydrobromic acid, hydroiodic acid, nitric acid, carbonic acid, phosphoric acid, phosphorous acid, sulfuric acid, etc. The said pharmaceutically acceptable acids also include organic acids, which include, but are not limited to, acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citrate, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acids (e.g., glutamic acid, arginine), etc. When the compounds of the present disclosure contain both relatively acidic and relatively basic functional groups, they can be converted into either base addition salt or acid addition salt. For details, reference may be made to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, eds., Wiley-VCH, 2002).

The term "tautomer" refers to functional group isomers formed by the rapid migration of a certain atom between two positions within a molecule. For example, acetone and 1-propen-2-ol be converted into each other through the rapid migration of a hydrogen atom between the oxygen atom and the α-carbon atom.

The term "isotopically labeled compound" refers to a compound in which one or more atoms are replaced by one or more atoms with a specific atomic mass or mass number. Examples of isotopes that can be incorporated into the compounds of the present disclosure include, but are not limited to, isotopes of hydrogen, carbon, nitrogen, oxygen, fluorine, sulfur, and chlorine (e.g., ²H, ³H, ¹³C, ¹⁴C, ¹⁵N, ¹⁸O, ¹⁷O, ¹⁸F, ³⁵S, and ³⁶Cl). The isotopic compounds of the present disclosure can generally be prepared by replacing non-isotopically labeled reagents with isotopically labeled reagents in accordance with the methods described herein.

The term "halogen" refers to fluorine, chlorine, bromine or iodine.

The term "alkyl" refers to a straight-chain or branched-chain alkyl group having a specified number of carbon atoms. Examples of alkyl groups include methyl, ethyl, n-propyl, isopropyl, n-butyl, tert-butyl, isobutyl, sec-butyl, n-pentyl, n-hexyl, n-heptyl, n-octyl and similar alkyl groups thereof.

The term "aryl" refers to an aromatic group composed solely of carbon atoms, wherein each ring exhibits aromaticity. Examples include phenyl or naphthyl.

The term "cyano" refers to a -CN group.

The term "oxo" refers to a =O group.

The above preferred conditions may be combined arbitrarily to obtain preferred embodiments of the present disclosure without departing from the general knowledge in the art.

All the reagents and raw materials used in the present disclosure are commercially available.

The positive effect of the present invention is that the present invention provides a compound with a tricyclic fused ring structure, a pharmaceutical composition thereof and the use thereof, wherein the compound with a tricyclic fused ring structure has a relatively good inhibitory effect on cells of various PI3K-meditated diseases, and is expected to be used for treating and/or preventing various PI3K-meditated diseases.

### DETAILED DESCRIPTION OF THE EMBODIMENTS

The present invention is further illustrated by the following examples, which are not intended to limit the present invention. Experimental procedures without specified conditions in the following examples were performed in accordance with conventional procedures and conditions, or in accordance with instructions.

In the present disclosure, room temperature refers to the ambient temperature, ranging from 10°C to 35°C. Overnight refers to a duration of 8 to 15 hours. Reflux refers to the reflux temperature of the solvent under atmospheric pressure.

| | |
|---|---|
| Pd₂(dba)₃ | tris(dibenzylideneacetone)dipalladium |
| Pd(dppf)Cl₂ | 1,1'-Bis(diphenylphosphino)ferrocene]dichloropalladium(II) |
| HATU | 2-(7-Azabenzotriazol-1-yl)-N,N,N',N'-tetramethyluronium hexafluorophosphate |
| DIEA | diisopropylethylamine |
| PPA | Polyphosphoric acid |

Eaton's reagent Phosphorus pentoxide methanesulfonic acid

### Example 1 Synthetic route of compound 1(1-p1 and 1-p2)

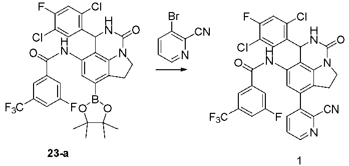

### Synthesis of compound 1

To a 50 mL three-necked flask were added compound 23-a (350 mg, 0.523 mmol), 2-cyano-3-bromopyridine (210 mg, 1.105 mmol), potassium carbonate (216.84 mg, 1.569 mmol), Pd(dppf)Cl₂ (38.04 mg, 0.05 mmol), 1,4-dioxane (15 mL) and water (3 mL) successively. The mixture was stirred at 100 °C for 1 hour under nitrogen protection. The reaction mixture was concentrated under reduced pressure and purified by column chromatography (dichloromethane:methanol = 20:1 to 10:1) to afford compound 1 (120 mg, 0.187 mmol, 35.76%). LC-MS (ESI): m/z 645.3 (M+H)⁺. ¹H NMR (400 MHz, DMSO-*d*₆) δ 10.20 (s, 1H), 8.79 (s, 1H), 8.31 (d, *J* = 9.3 Hz, 1H), 8.03-7.80 (m, 4H), 7.61 (d, *J* = 9.1 Hz, 1H), 7.52 (d, *J* = 2.3 Hz, 1H), 7.25 (d, *J* = 7.8 Hz, 1H), 6.96 (s, 1H), 6.13 (d, *J* = 2.2 Hz, 1H), 4.03 (qd, *J* = 7.8, 7.0, 5.6 Hz, 2H), 3.24 (m, *J* = 13.6, 9.7, 7.0 Hz, 2H).

Chiral resolution conditions: chromatographic column: OJ-H, 4.6*100 mm 5 µm; mobile phase: MeOH[0.2%NH₃(7 M in MeOH)]; flow rate: 3.0 mL/min; back pressure: 2000 psi; detection wavelength: 254 nm.

Compound 1-p1 (Retention time 0.693min): LC-MS (ESI): m/z 645.3 (M+H)⁺. ¹H NMR (400 MHz, DMSO-*d6*) δ 10.30 (s, 1H), 8.79 (s, 1H), 8.34 (d, J = 9.3 Hz, 1H), 8.03-7.80 (m, 4H), 7.61 (d, J = 9.1 Hz, 1H), 7.52 (d, J = 2.3 Hz, 1H), 7.26 (d, J = 7.8 Hz, 1H), 6.96 (s, 1H), 6.13 (d, J = 2.2 Hz, 1H), 4.03 (qd, J = 7.8, 7.0, 5.6 Hz, 2H), 3.26 (m, 2H).

Compound 1-p2 (Retention time 1.326min): LC-MS (ESI): m/z 645.3 (M+H)⁺. ¹H NMR (400 MHz, DMSO-*d6*) δ 10.30 (s, 1H), 8.79 (s, 1H), 8.34 (d, J = 9.3 Hz, 1H), 8.03-7.80 (m, 4H), 7.61 (d, J = 9.1 Hz, 1H), 7.52 (d, J = 2.3 Hz, 1H), 7.26 (d, J = 7.8 Hz, 1H), 6.96 (s, 1H), 6.13 (d, J = 2.2 Hz, 1H), 4.03 (qd, J = 7.8, 7.0, 5.6 Hz, 2H), 3.26 (m, 2H).

### Example 2 Synthetic route of compound 2 and compound 3

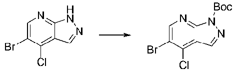

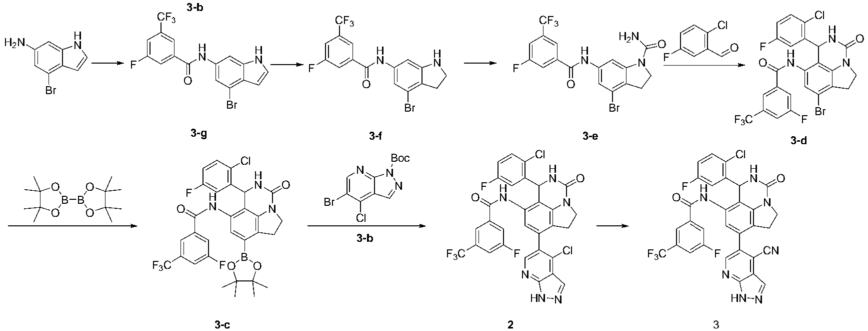

### Synthesis of compound 3-g

To a 100 mL three-necked flask were added 4-bromo-1H-indol-6-amine (1000 mg, 4.738 mmol), triethylamine (1.317 mL, 9.476 mmol) and dichloromethane (40 mL) successively. The mixture was cooled to 0 °C, followed by the addition of 3-fluoro-5-(trifluoromethyl)benzoyl chloride (1180.73 mg, 5.212 mmol). The reaction was stirred at 0 °C for 30 min and then warmed to room temperature for further 1 h. The reaction mixture was concentrated and purified by column chromatography (eluent: petroleum ether/ethyl acetate = 5:1 to 3:1) to afford compound 3-g (1809 mg, 4.509 mmol, 95.18%).

### Synthesis of compound 3-f

To a 100 mL three-necked flask were added compound 3-g (802 mg, 1.999 mmol), acetic acid (30 mL) and sodium cyanoborohydride (376.89 mg, 5.998 mmol) successively. The reaction was carried out at room temperature for 18 h. The reaction mixture was concentrated, then ethyl acetate and saturated aqueous sodium bicarbonate solution were added. The organic phase was concentrated and purified by column chromatography (eluent: petroleum ether/ethyl acetate = 10:1 to 3:1) to afford compound 3-f (350 mg, 0.868 mmol, 43.42%).

### Synthesis of compound 3-e

To a 100 mL three-necked flask were added compound 3-f (320 mg, 0.794 mmol) and acetic acid (10 mL) successively. The mixture was cooled to 0 °C, and potassium cyanate (128.77 mg, 1.587 mmol) was added. The reaction was stirred at 0 °C for half an hour. The reaction mixture was concentrated, followed by addition of water and saturated aqueous sodium bicarbonate solution. The mixture was filtered, and the filter cake was washed with water and then dried to afford compound 3-e (280 mg, 0.628 mmol, 79.06%).

### Synthesis of compound 3-d

To a 100 mL three-necked flask was added polyphosphoric acid (20 mL). The mixture was heated to 105 °C, then compound 3-e (150 mg, 0.336 mmol) and 2-chloro-5-fluorobenzaldehyde (53.28 mg, 0.336 mmol) were added successively. The reaction was continued at this temperature for 30 min. Dichloromethane and saturated aqueous sodium bicarbonate solution were added, the organic phase was concentrated and purified by column chromatography (eluent: dichloromethane/methanol = 20:1 to 10:1) to afford compound 3-d (47 mg, 0.080 mmol, 23.83%).

LC-MS (ESI): m/z 587.9 (M+H)⁺; ¹H NMR (400 MHz, DMSO-*d*₆) δ 10.16 (s, 1H), 7.95 (dt, J = 8.6, 1.9 Hz, 1H), 7.89-7.77 (m, 2H), 7.54 (d, J = 2.6 Hz, 1H), 7.32 (dd, J = 8.8, 5.2 Hz, 1H), 7.09 (ddd, J = 8.8, 8.0, 3.1 Hz, 1H), 6.95 (s, 1H), 6.90 (dd, J = 9.2, 3.1 Hz, 1H), 6.14-5.97 (m, 1H), 4.16 - 3.87 (m, 2H), 3.17 (ddd, J = 9.6, 6.9, 4.6 Hz, 2H).

### Synthesis of compound 3-c

Compound 3-d (0.5 g, 0.852 mmol), potassium acetate (250.85 mg, 2.556 mmol), bis(pinacolato)diboron (540.89 mg, 2.130 mmol) and [1,1'-bis(diphenylphosphino)ferrocene]dichloropalladium(II) (62.34 mg, 0.085 mmol) were placed in a reaction flask and dissolved in 1,4-dioxane (7 mL). The mixture was heated to 100 °C under nitrogen atmosphere and stirred for 2 hours. Potassium acetate (83.6 mg, 0.852 mmol), bis(pinacolato)diboron (216.0 mg, 0.852 mmol) and [1,1'-bis(diphenylphosphino)ferrocene]dichloropalladium(II) (62.34 mg, 0.085 mmol) were added, and the reaction was continued for another 2 hours. After concentration, the crude product was purified by normal phase pre-TLC (eluent: DCM/MeOH = 10:1) to afford the target compound 3-c (400 mg, 74.06%) as a yellowish-brown solid. LC-MS (ESI): m/z 634.1(M+H) ⁺.

### Synthesis of compound 3-b

Methyl 5-bromo-6-fluoro-1H-indazole-4-carboxylate (100 mg, 0.430 mmol) was dissolved in dichloromethane (5 mL). Triethylamine (0.18 mL, 1.290 mmol) and di-tert-butyl dicarbonate (140 mg, 0.645 mmol) were added, and the mixture was stirred at room temperature overnight. The reaction mixture was concentrated under reduced pressure and purified by column chromatography (petroleum ether/ethyl acetate ~ 20/1) to afford compound 3-b (100 mg, 69.9%). LCMS (ESI): m/z 332.0 (M+H)⁺.

### Synthesis of compound 2

Compound 3-c (150 mg, 0.237 mmol), compound 3-b (78.7 mg, 0.237 mmol), potassium carbonate (98 mg, 0.710 mmol) and Pd(dppf)Cl₂ (17.3 mg, 0.024 mmol) were added to a reaction flask, followed by addition of 1,4-dioxane (2 mL) and water (0.4 mL). The mixture was purged with nitrogen and stirred at 100 °C for 2 hours. The reaction mixture was diluted with ethyl acetate and water. The organic phase was separated, and the aqueous phase was extracted with ethyl acetate. The combined organic phases were washed with water and saturated aqueous sodium chloride solution, dried over anhydrous Na₂SO₄, filtered, concentrated under reduced pressure and purified by column chromatography (dichloromethane/methanol ~ 20/1) to afford compound 2 (50 mg, 1.9%). LCMS (ESI): m/z 659.0 (M+H)⁺. ¹H NMR (400 MHz, DMSO-*d*₆) δ 14.15 (s, 1H), 10.26 (s, 1H), 8.53 (s, 1H), 8.32 (s, 1H), 7.95 (d, J = 8.6 Hz, 1H), 7.88 (d, J = 14.8 Hz, 2H), 7.59 (d, J = 2.6 Hz, 1H), 7.37 (dd, J = 8.9, 5.1 Hz, 1H), 7.13 (td, J = 8.4, 3.1 Hz, 1H), 6.94 (dd, J = 9.2, 3.1 Hz, 1H), 6.79 (s, 1H), 6.17 (d, J = 2.5 Hz, 1H), 4.01 (td, J = 11.0, 10.5, 7.5 Hz, 2H), 3.10 (q, J = 8.2 Hz, 2H).

### Synthesis of compound 3

Compound 3-a (240 mg, 0.576 mmol) was dissolved in DMAC (1 mL). Zinc cyanide (13.4 mg, 0.114 mmol), zinc powder (1 mg, 0.015 mmol), Pd₂(dba)₃ (10.4 mg, 0.011 mmol) and DPPF (12.6 mg, 0.023 mmol) were added successively. The mixture was purged with nitrogen and reacted under microwave irradiation at 170 °C for 1 hour. The reaction mixture was diluted with ethyl acetate and water. The organic phase was separated, and the aqueous phase was extracted with ethyl acetate. The combined organic phases were washed with water and saturated brine, dried over anhydrous Na₂SO₄, filtered, concentrated under reduced pressure and purified by column chromatography (dichloromethane/methanol ~ 20/1) to afford compound 3 (13 mg). ESI: (m/z) = 650.0 [M+H]⁺; ¹H NMR (400 MHz, DMSO-*d*₆) δ 14.14 (s, 1H), 10.33 (s, 1H), 8.85 (s, 1H), 8.47 (s, 1H), 7.96 (dd, J = 8.3, 2.2 Hz, 1H), 7.93-7.86 (m, 2H), 7.64 (d, J = 2.6 Hz, 1H), 7.38 (dd, J = 8.8, 5.1 Hz, 1H), 7.14 (td, J = 8.4, 3.1 Hz, 1H), 6.98-6.91 (m, 2H), 6.19 (d, J = 2.6 Hz, 1H), 4.04 (dtd, J = 28.3, 10.4, 6.7 Hz, 2H), 3.23 (ddd, J = 24.5, 10.2, 6.7 Hz, 2H).

### Example 3 Synthetic route of compound 4, 5 and 6

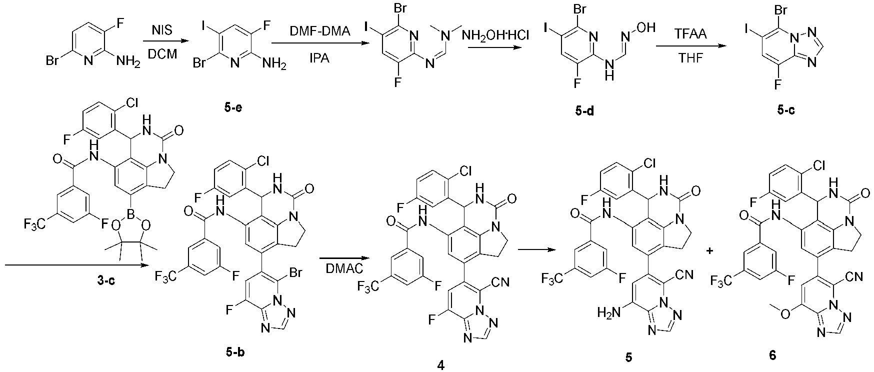

### Synthesis of compound 5-e

6-Bromo-3-fluoropyridin-2-amine (10 g, 52.356 mmol) was dissolved in dichloromethane (120 mL). N-Iodosuccinimide (14.14 g, 62.827 mmol) was added, and the mixture was stirred at room temperature for 48 hours. The reaction mixture was concentrated under reduced pressure, then diluted with ethyl acetate and water. The organic phase was separated, and the aqueous phase was extracted with ethyl acetate. The combined organic phases were washed with water and saturated aqueous sodium chloride solution, dried over anhydrous Na₂SO₄, filtered, concentrated under reduced pressure and purified by column chromatography (mobile phase: petroleum ether/ethyl acetate ~ 10/1) to afford compound 5-e (15.56 g, 93.79%). ESI: (m/z) = 317.0 [M+H]⁺; ¹H NMR (400 MHz, DMSO-*d*₆) δ 7.79 (d, J = 10.0 Hz, 1H), 6.87 (s, 2H).

### Synthesis of compound 5-d

Compound 5-e (15.56 g, 49.10 mmol) was dissolved in isopropanol (160 mL). N,N-Dimethylformamide dimethyl acetal (11.70 g, 98.20 mmol) was added, and the mixture was stirred at 80 °C for 6 hours. The reaction mixture was cooled to 50 °C, and hydroxylamine hydrochloride (6.82 g, 98.20 mmol) was added. The reaction was continued at 50 °C overnight. The mixture was filtered directly, and the filter cake was washed with ethanol twice to afford compound 5-d (15.86 g, 89.76%). ESI: (m/z) = 360.0 [M+H]⁺. ¹H NMR (400 MHz, DMSO-*d*₆) δ 10.63 (s, 1H), 8.71 (d, J = 9.5 Hz, 1H), 8.17 (d, J = 9.5 Hz, 1H), 7.63 (d, J = 9.5 Hz, 1H).

### Synthesis of compound 5-c

Compound 5-d (15.86 g, 44.07 mmol) was dissolved in Eaton's reagent (160 mL), and the mixture was stirred at 80 °C for 3.5 hours. The reaction mixture was diluted with ethyl acetate and slowly added dropwise to ice-cooled saturated aqueous sodium bicarbonate solution to adjust to weakly basic. The mixture was extracted with ethyl acetate, the aqueous phase was extracted with ethyl acetate twice, followed by extraction with dichloromethane/methanol ~ 10:1 twice. The organic phases were combined separately, washed with water, dried over anhydrous Na₂SO₄, filtered, concentrated under reduced pressure and purified by column chromatography (mobile phase: petroleum ether/ethyl acetate ~ 3:1) to afford compound 5-c (14 g, 92.9%). ESI: (m/z) = 342.0 [M+H]⁺.

### Synthesis of compound 5-b

Compound 3-c (8 g, 12.62 mmol), compound 5-c (4.1 g, 11.99 mmol), potassium carbonate (5.23 g, 37.866 mmol) and Pd(dppf)Cl₂ (920 mg, 1.262 mmol) were added to a reaction flask, followed by addition of 1,4-dioxane (70 mL) and water (14 mL). The mixture was purged with nitrogen and stirred at 100 °C for 3 hours. The mixture was extracted with ethyl acetate and water, and concentrated under reduced pressure. Purification by column chromatography (mobile phase: dichloromethane/methanol ~ 20:1) afforded compound 5-b (5.05 g, 55.43%). LCMS (ESI): m/z 721.0 (M+H)⁺;

### Synthesis of compound 4

Compound 5-b (5.05 g, 6.996 mmol), zinc cyanide (1.23 g, 10.494 mmol), zinc powder (50 mg, 0.70 mmol), Xantphos (1.62 g, 2.798 mmol) and palladium acetate (0.31 g, 1.399 mmol) were added to a reaction flask, followed by addition of N,N-dimethylacetamide (50 mL). The mixture was purged with nitrogen and stirred at 120 °C for 1 hour. The mixture was extracted with ethyl acetate and water, and concentrated under reduced pressure. Purification by column chromatography (mobile phase: dichloromethane/methanol ~ 20:1) afforded compound 4 (3.4 g). LCMS (ESI): m/z 668.0 (M+H)⁺;

### Synthesis of compound 5

Compound 4 (100 mg, 0.150 mmol), aqueous ammonia (2 mL) and methanol (1 mL) were added to a reaction flask. The mixture was stirred at room temperature overnight. The solvent was evaporated under reduced pressure, and the residue was extracted with ethyl acetate and water. The organic phase was concentrated and purified by preparative chromatography to afford compound 5 (25 mg) and compound 6 (27 mg).

Compound 5: LCMS (ESI): m/z 665.0 (M+H)⁺;
¹H NMR (400 MHz, DMSO-*d*₆) δ 10.30 (s, 1H), 8.59 (s, 1H), 7.96 (d, J = 8.5 Hz, 1H), 7.88 (d, J = 14.8 Hz, 2H), 7.62 (d, J = 2.6 Hz, 1H), 7.46 (s, 2H), 7.37 (dd, J = 8.9, 5.1 Hz, 1H), 7.13 (td, J = 8.4, 3.0 Hz, 1H), 6.93 (dd, J = 9.2, 3.1 Hz, 1H), 6.87 (s, 1H), 6.59 (s, 1H), 6.17 (d, J = 2.5 Hz, 1H), 4.13-3.94 (m, 2H), 3.22 (q, J = 7.7 Hz, 2H).

Compound 6: LCMS (ESI): m/z 680.0 (M+H)⁺; ¹H NMR (400 MHz, DMSO-*d*₆) δ 10.35 (s, 1H), 8.69 (s, 1H), 7.96 (d, J = 8.5 Hz, 1H), 7.94-7.87 (m, 2H), 7.66 (d, J = 2.7 Hz, 1H), 7.38 (dd, J = 8.8, 5.2 Hz, 1H), 7.33 (s, 1H), 7.14 (ddd, J = 8.9, 8.0, 3.1 Hz, 1H), 6.98 (s, 1H), 6.94 (dd, J = 9.1, 3.1 Hz, 1H), 6.23-6.16 (m, 1H), 4.14 (s, 3H), 4.12-3.96 (m, 2H), 3.42-3.36 (m, 1H), 3.23 (ddd, J = 16.5, 10.4, 6.0 Hz, 1H).

### Example 4 Synthetic route of compound 7

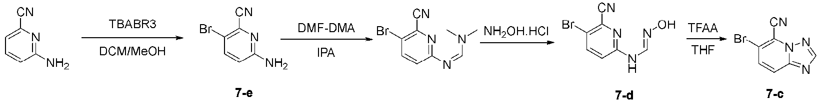

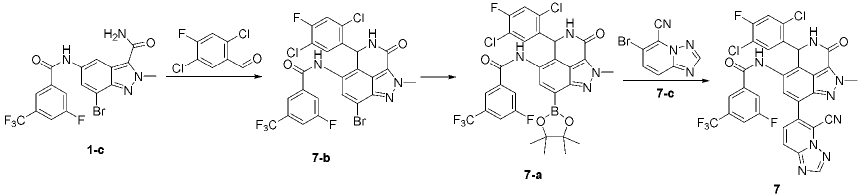

### Synthesis of compound 7-e

2-Amino-6-cyanopyridine (15 g, 125.91 mmol) was dissolved in dichloromethane (60 mL) and methanol (60 mL). Tetrabutylammonium tribromide (69.82 g, 144.8 mmol) was added, and the mixture was stirred at room temperature for 16 hours. The reaction mixture was diluted with ethyl acetate and water. The organic phase was separated, and the aqueous phase was extracted with ethyl acetate. The combined organic phases were washed with water and saturated aqueous sodium bicarbonate solution, dried over anhydrous Na₂SO₄, filtered, and concentrated under reduced pressure. Purification by column chromatography (mobile phase: petroleum ether/ethyl acetate ~ 3:1) afforded compound 7-e (10.2 g, 40.11%).

### Synthesis of compound 7-d

Compound 7-e (10.2 g, 51.51 mmol) was dissolved in isopropanol (150 mL). N,N-Dimethylformamide dimethyl acetal (7.98 g, 66.96 mmol) was added, and the mixture was stirred at 80 °C for 3 hours. The reaction mixture was cooled to 50 °C, and hydroxylamine hydrochloride (4.65 g, 66.96 mmol) was added. The reaction was continued at 50 °C overnight. The mixture was filtered directly, and the filter cake was washed with ethanol to afford compound 7-d (10 g, 80.52%). ESI: (m/z) = 241.0 [M+H]⁺.

### Synthesis of compound 7-c

Compound 7-d (5 g, 20.743 mmol) was dissolved in tetrahydrofuran (50 mL). The reaction mixture was cooled to 0 °C, and trifluoroacetic anhydride (8.71 g, 41.485 mmol) was added dropwise. The mixture was stirred at 60 °C overnight. The reaction mixture was diluted with ethyl acetate and water. The organic phase was separated, and the aqueous phase was extracted with ethyl acetate. The combined organic phases were washed with water and saturated aqueous sodium bicarbonate solution, dried over anhydrous Na₂SO₄, filtered, and concentrated under reduced pressure. Purification by column chromatography (mobile phase: petroleum ether/ethyl acetate ~ 2:1) afforded compound 7-c (1.1 g, 23.76%). ESI: (m/z) = 223.0 [M+H]⁺. ¹H NMR (400 MHz, DMSO-*d*₆) δ 8.73 (s, 1H), 8.20 (d, J = 9.5 Hz, 1H), 8.06 (d, J = 9.5 Hz, 1H).

### Synthesis of compound 7-b

Compound 1-c (1 g, 2.178 mmol) was dissolved in Eaton's reagent (10 mL). 2,5-dichloro-4-fluorobenzaldehyde (0.5 g, 2.613 mmol) was added, and the mixture was stirred at 80 °C for 2 hours. The reaction mixture was diluted with ethyl acetate and water. The organic phase was separated, and the aqueous phase was extracted with ethyl acetate. The combined organic phases were washed with water and saturated aqueous sodium bicarbonate solution, dried over anhydrous Na₂SO₄, filtered, and concentrated under reduced pressure. Purification by column chromatography (mobile phase: petroleum ether/ethyl acetate ~ 3:1) afforded compound 7-b (280 mg, 20.29%). ESI: (m/z) = 633.0 [M+H]⁺.

### Synthesis of compound 7-a

Compound 7-b (280 mg, 0.442 mmol) was dissolved in 1,4-dioxane (5 mL). Bis(pinacolato)diboron (168 mg, 0.662 mmol), potassium acetate (130 mg, 1.325 mmol) and Pd(dppf)Cl₂ (64.6 mg, 0.088 mmol) were added, and the mixture was stirred at 100 °C for 2 hours. The reaction mixture was used directly in the next step without further purification.

### Synthesis of compound 7

Compound 7-a (260 mg, 0.434 mmol), compound 7-c (145 mg, 0.651 mmol), potassium carbonate (180 mg, 1.302 mmol) and Pd(dppf)Cl₂ (31.76 mg, 0.043 mmol) were added to a reaction flask, followed by addition of 1,4-dioxane (5 mL) and water (1 mL). The mixture was purged with nitrogen and stirred at 100 °C for 1 hour. The mixture was extracted with ethyl acetate and water, and concentrated under reduced pressure. Purification by normal-phase and reversed-phase column chromatography afforded compound 7 (80 mg). LCMS (ESI): m/z 697.3 (M+H)⁺;

### Example 5 Synthetic route of compound 8

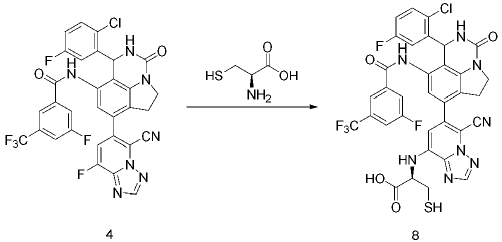

### Synthesis of compound 8

Compound 4 (100 mg, 0.15 mmol) was placed in a reaction flask, followed by addition of L-cysteine (18 mg, 0.15 mmol) and DMAC (3 mL). The mixture was stirred at room temperature overnight, filtered, and purified by preparative chromatography to afford compound 8 (26 mg). LCMS (ESI): m/z 769.5 (M+H)⁺; ¹H NMR (400 MHz, DMSO-*d*₆) δ 10.37 (s, 1H), 8.74 (s, 1H), 7.99-7.87 (m, 4H), 7.85 (d, J = 5.9 Hz, 1H), 7.65 (t, J = 3.1 Hz, 1H), 7.38 (ddd, J = 8.9, 5.2, 2.1 Hz, 1H), 7.19-7.10 (m, 1H), 7.00 (d, J = 5.1 Hz, 1H), 6.93 (dt, J = 9.0, 3.0 Hz, 1H), 6.19 (dd, J = 5.8, 2.5 Hz, 1H), 4.10-3.96 (m, 2H), 3.88 (ddd, J = 14.1, 8.6, 3.1 Hz, 1H), 3.55-3.42 (m, 4H), 2.00 (q, J = 7.3 Hz, 1H).

### Example 6 Synthetic route of compound 9 (9-p1 and 9-p2)

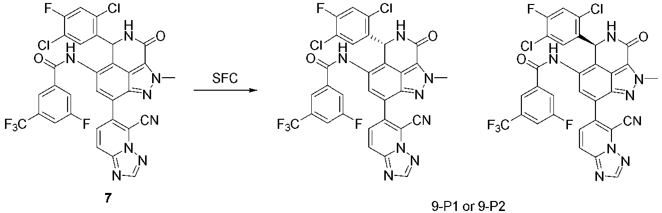

### Synthesis of compound 9-p1 and 9-p2

Compound 7 (75 mg) was subjected to SFC resolution to afford compound 9-p2 (26 mg). Resolution conditions: instrument: SFC-150 (Waters); chromatographic column: 0.46 cm I.D. *5 cm L; mobile phase: CO₂/EtOH = 70/30; flow rate: 2.5 mL/min; back pressure: 2000 psi; detection wavelength: 254 nm; cycle time: 5.0 minutes: The retention time of active compound 9-p2 under this method was 3.379 min. Spectroscopic data for compound 9-p2: LCMS (ESI): m/z 697.2 [M+H]⁺; 1H NMR (400 MHz, DMSO-*d*₆) δ 10.56 (s, 1H), 8.81 (s, 1H), 8.59 (s, 1H), 8.39 (d, J = 9.3 Hz, 1H), 8.19 (d, J = 9.3 Hz, 1H), 7.98 (d, J = 8.4 Hz, 1H), 7.87 (d, J = 9.2 Hz, 1H), 7.76 (s, 1H), 7.66-7.58 (m, 2H), 7.36 (d, J = 7.9 Hz, 1H), 6.42 (s, 1H), 4.48 (s, 3H).

### Example 7 Synthetic route of compound 10

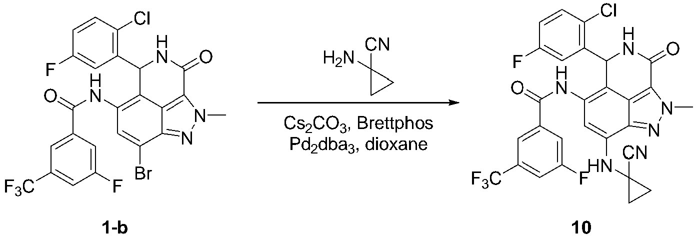

### Synthesis of compound 10

Compound 1-b (180 mg, 0.30 mmol), 1-amino-1-cyclopropyl cyanide hydrochloride (71 mg, 0.60 mmol), cesium carbonate (294 mg, 0.60 mmol), BrettPhos (32 mg, 0.060 mmol), and Pd₂(dba)₃ (27.5 mg, 0.30 mmol) were placed in a sealed tube, followed by addition of 1,4-dioxane (3 mL). The mixture was stirred at 100 °C for 2.5 hours, then extracted with ethyl acetate and water. The organic phase was concentrated under reduced pressure, purified by normal-phase and reversed-phase column chromatography to afford compound 10 (56.5 mg). LCMS (ESI): m/z 601.0 (M+H)⁺;
¹H NMR (400 MHz, DMSO-*d*₆) δ 10.36 (s, 1H), 8.31 (d, J = 2.2 Hz, 1H), 7.93 (dd, J = 8.5, 2.1 Hz, 1H), 7.82 (dt, J = 9.3, 1.9 Hz, 1H), 7.72 (s, 1H), 7.61 (s, 1H), 7.31 (dd, J = 8.8, 5.1 Hz, 1H), 7.06 (td, J = 8.3, 3.1 Hz, 1H), 6.79 (dd, J = 9.2, 3.1 Hz, 1H), 6.39 (s, 1H), 6.25 (d, J = 2.1 Hz, 1H), 4.41 (s, 3H), 1.57 (t, J = 3.7 Hz, 2H), 1.29 (t, J = 4.0 Hz, 2H).

### Example 8 Synthetic route of compound 11 (11-p1 and 11-p2)

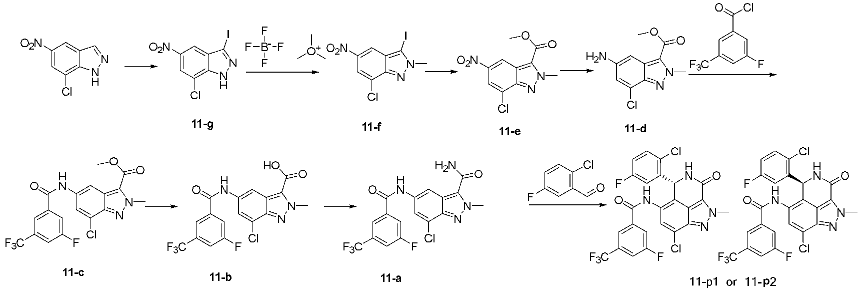

### Synthesis of compound 11-g

7-chloro-5-nitro-1H-benzimidazole (10 g, 50.612 mmol) was dissolved in DMF (100 mL). Potassium hydroxide (5.68 g, 101.225 mmol) was added, and the mixture was purged with nitrogen. Iodine (32.11 g, 126.53 mmol) was added, and the mixture was stirred at 70 °C overnight. The reaction mixture was washed with 5% aqueous sodium thiosulfate solution twice. The aqueous phase was adjusted to weakly acidic with aqueous citric acid solution and extracted with ethyl acetate. The combined organic phases were washed with water and saturated brine, dried over anhydrous Na₂SO₄, filtered, and concentrated under reduced pressure. The residue was slurried with dichloromethane/methanol = 10/1 three times. The solid was collected, and the filtrate was further purified by column chromatography (mobile phase: dichloromethane/methanol 1/0 to 20/1) to afford compound 11-g (14.3 g, 87.35%). ¹H NMR (400 MHz, DMSO-*d*₆) δ 14.70 (s, 1H), 8.31 (d, J = 1.9 Hz, 1H), 8.24 (d, J = 1.9 Hz, 1H).

### Synthesis of compound 11-f

Compound 11-g (14.3 g, 44.208 mmol) was dissolved in ethyl acetate (400 mL). Trimethyloxonium tetrafluoroborate (13.08 g, 88.416 mmol) was added, and the mixture was purged with nitrogen, then stirred at room temperature overnight. A solid precipitated from the reaction mixture, which was filtered off. The solid was slurried with dichloromethane/methanol to afford crude compound 11-f (12.2 g, 81.77%). ESI: (m/z) = 338.0 [M+H]⁺; ¹H NMR (400 MHz, DMSO-*d*₆) δ 8.20 (t, J = 1.4 Hz, 1H), 8.04-7.98 (m, 1H), 4.25 (s, 3H).

### Synthesis of compound 11-e

Compound 11-f (4.5 g, 30.631 mmol) was dissolved in DMF (20 mL) and methanol (20 mL). Triethylamine (5.56 mL, 91.894 mmol) and Pd(dppf)Cl₂ (2.93 g, 4.0 mmol) were added, and the mixture was purged with carbon monoxide gas, then stirred at 70 °C for 3 hours. Methanol was evaporated under reduced pressure, and the reaction mixture was diluted with water and extracted with ethyl acetate. The combined organic phases were washed with water and saturated brine, dried over anhydrous Na₂SO₄, filtered, and concentrated under reduced pressure. Purification by column chromatography (mobile phase: petroleum ether / dichloromethane ~40%/60%) afforded compound 11-e (1.4 g, 38.89%). ESI: (m/z) = 270.2 [M+H]⁺; ¹H NMR (400 MHz, DMSO-*d*₆) δ 8.81 (d, J = 2.0 Hz, 1H), 8.26 (d, J = 1.9 Hz, 1H), 4.52 (s, 3H), 4.06 (s, 3H).

### Synthesis of compound 11-d

Compound 11-e (1.4 g, 5.192 mmol) was dissolved in ethanol (20 mL) and water (4 mL). Ammonium chloride (0.83 g, 15.576 mmol) and iron powder (1.45 g, 25.961 mmol) were added, and the mixture was stirred at 70 °C for 4 hours. The reaction mixture was filtered through celite, washed with dichloromethane, and concentrated under reduced pressure. The residue was extracted with ethyl acetate and water. The organic phase was washed with saturated brine, dried over anhydrous Na₂SO₄, filtered, and evaporated to dryness to afford compound 11-d (1.2 g, 96.77%). ESI: (m/z) = 240.1 [M+H]⁺;

### Synthesis of compound 11-c

Compound 11-d (1.2 g, 5.007 mmol) was dissolved in dichloromethane (15 mL). Triethylamine (2.088 mL, 15.021 mmol) was added, and the mixture was purged with nitrogen and cooled to 0 °C. 3-fluoro-5-(trifluoromethyl)benzoyl chloride (1.70 g, 7.511 mmol) was added, and the mixture was stirred at room temperature for 1 hour. A solid precipitated from the reaction mixture, which was collected by filtration. The solid was slurried with dichloromethane, and the filtrate was purified by column chromatography (mobile phase: petroleum ether/ethyl acetate from 1/0 to 3/1) to afford compound 11-c (1.57 g, 73.02%). ESI: (m/z) = 430.2 [M+H]⁺.

### Synthesis of compound 11-b

Compound 11-c (1.57 g, 3.653 mmol) was dissolved in methanol (10 mL), tetrahydrofuran (10 mL) and water (10 mL). Lithium hydroxide (0.77 g, 18.266 mmol) was added, and the mixture was stirred at 60 °C for 1 hour. The solvent was evaporated under reduced pressure. The residue was adjusted to pH 3-4 with 1 N dilute hydrochloric acid, and a solid precipitated. The mixture was extracted with ethyl acetate and concentrated to dryness to afford crude compound 11-b (1.52 g, crude). ESI: (m/z) = 416.1 [M-H]⁺;

### Synthesis of compound 11-a

Compound 11-b (1.52 g, 3.656 mmol) was dissolved in DMF (15 mL). Ammonium chloride (0.39 g, 7.312 mmol), HATU (2.09 g, 5.484 mmol) and DIEA (1.42 g, 10.969 mmol) were added, and the mixture was stirred at room temperature for 2 hours. The reaction mixture was diluted with ethyl acetate and water. The organic phase was separated, and the aqueous phase was extracted with ethyl acetate. The combined organic phases were washed with water and saturated brine, dried over anhydrous Na₂SO₄, filtered and concentrated under reduced pressure to afford crude compound 11-a (1.2 g, 78.95%). ESI: (m/z) = 415.1 [M+H]⁺.

### Synthesis of compound 11 (11-p1 and 11-p2)

Compound 11-a (600 mg, 1.447 mmol) was dissolved in Eaton's reagent (10 mL). 2-chloro-5-fluorobenzaldehyde (275 mg, 1.736 mmol) was added, and the mixture was stirred at 80 °C for 2 hours. The reaction mixture was added dropwise into ice-cold saturated aqueous sodium bicarbonate solution, and the system was adjusted to basic. The mixture was extracted with ethyl acetate. The combined organic phases were washed with aqueous sodium chloride solution, dried over anhydrous Na₂SO₄, filtered, and concentrated under reduced pressure. Purification by column chromatography (mobile phase: dichloromethane/methanol ~ 10:1) afforded compound 11 (57 mg).

Compound 11 was subjected to chiral resolution to afford compound 11-p1 and 11-p2. Resolution conditions: instrument: SFC-150 (Waters); chromatographic column: 0.46 cm I, D. *5 cm L; mobile phase: CO₂/EtOH = 70/30; flow rate: 2.5 mL/min; back pressure: 2000 psi; detection wavelength: 254 nm; cycle time: 5.0 minutes;
Compound 11-p1 (retention time 1.543 min): ¹H NMR (400 MHz, DMSO-*d*₆) δ 10.37 (s, 1H), 8.60 (d, J = 2.2 Hz, 1H), 7.96 (dt, J = 8.5, 2.0 Hz, 1H), 7.81 (dt, J = 9.2, 1.9 Hz, 1H), 7.76 (s, 1H), 7.36-7.28 (m, 2H), 7.06 (td, J = 8.4, 3.1 Hz, 1H), 6.95 (dd, J = 9.2, 3.1 Hz, 1H), 6.37 (d, J = 1.9 Hz, 1H), 4.47 (s, 3H).
Compound 11-p2 (retention time of active compound 11-p2 under this method was 3.246 min): ¹H NMR (400 MHz, DMSO-*d*₆) δ 10.37 (s, 1H), 8.60 (d, J = 2.2 Hz, 1H), 7.96 (dt, J = 8.5, 2.0 Hz, 1H), 7.81 (dt, J = 9.3, 1.9 Hz, 1H), 7.76 (s, 1H), 7.35-7.28 (m, 2H), 7.06 (ddd, J = 8.9, 8.0, 3.1 Hz, 1H), 6.95 (dd, J = 9.2, 3.1 Hz, 1H), 6.37 (s, 1H), 4.47 (s, 3H).

### Example 9 Synthetic route of compound 12 (12-p1 and 12-p2)

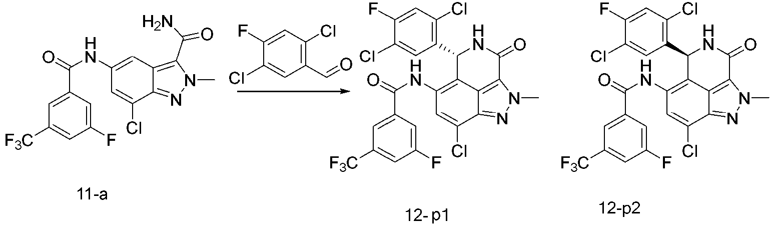

### Synthesis of compound 12-p1 and 12-p2

Compound 11-a (600 mg, 1.447 mmol) was dissolved in Eaton's reagent (10 mL). 2,5-dichloro-4-fluorobenzaldehyde (335 mg, 1.736 mmol) was added, and the mixture was stirred at 80 °C for 2 hours. The reaction mixture was added dropwise into ice-cold saturated aqueous sodium bicarbonate solution, and the system was adjusted to basic. The mixture was extracted with ethyl acetate. The combined organic phases were washed with aqueous sodium chloride solution, dried over anhydrous Na₂SO₄, filtered, and concentrated under reduced pressure. Purification by column chromatography (mobile phase: dichloromethane/methanol ~ 10:1) afforded compound 12.

Compound 12 was subjected to chiral resolution to afford compound 12-p1 (53 mg) and compound 12-p2 (52 mg). Resolution conditions: instrument: SFC-150 (Waters); chromatographic column: 0.46 cm I.D. *5 cm L; mobile phase: CO₂/EtOH = 70/30; flow rate: 2.5 mL/min; back pressure: 2000 psi; detection wavelength: 254 nm; cycle time: 5.0 minutes; the retention time of active compound 12-p2 under this method was 3.833 min.

Compound 12-p1: ESI: (m/z) = 589.0 [M+H]⁺. ¹H NMR (400 MHz, DMSO-*d*₆) δ 10.38 (s, 1H), 8.53 (d, J = 2.0 Hz, 1H), 7.97 (dt, J = 8.5, 1.9 Hz, 1H), 7.83 (dt, J = 9.3, 1.9 Hz, 1H), 7.72 (s, 1H), 7.57 (d, J = 9.1 Hz, 1H), 7.31 (d, J = 8.7 Hz, 2H), 6.49-6.25 (m, 1H), 4.47 (s, 3H).

Compound 12-p2: ESI: (m/z) = 589.0 [M+H]⁺. ¹H NMR (400 MHz, DMSO-*d*₆) δ 10.38 (s, 1H), 8.53 (d, J = 2.1 Hz, 1H), 7.97 (dt, J = 8.5, 1.9 Hz, 1H), 7.83 (dt, J = 9.2, 1.9 Hz, 1H), 7.72 (s, 1H), 7.57 (d, J = 9.0 Hz, 1H), 7.31 (d, J = 9.7 Hz, 2H), 6.36-6.27 (m, 1H), 4.47 (s, 3H).

### Example 10 Synthetic route of compound 13-p1 and 13-p2

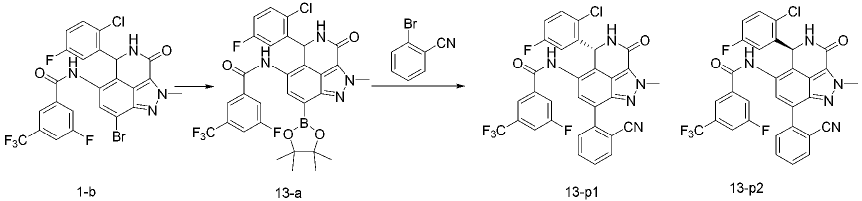

### Synthesis of compound 13-a

Compound 1-b (300 mg, 0.50 mmol) was dissolved in 1,4-dioxane (5 mL). Bis(pinacolato)diboron (190 mg, 0.750 mmol), potassium acetate (147 mg, 1.325 mmol) and Pd(dppf)Cl₂ (73 mg, 0.1 mmol) were added, and the mixture was stirred at 100 °C for 1 hour. The reaction mixture was used directly in the next step.

### Synthesis of compound 13-p1 and 13-p2

Compound 13-a (280 mg, 0.496 mmol), 2-bromobenzonitrile (135 mg, 0.744 mmol), potassium carbonate (205 mg, 1.488 mmol) and Pd(dppf)Cl₂ (36 mg, 0.05 mmol) were added to a reaction flask, followed by addition of 1,4-dioxane (5 mL) and water (1 mL). The mixture was purged with nitrogen and stirred at 100 °C for 1 hour. The mixture was extracted with ethyl acetate and water, concentrated under reduced pressure, and purified by normal-phase and reversed-phase column chromatography, followed by resolution to afford compound 13-p1 (68 mg) and compound 13-p2 (71.5 mg). LCMS (ESI): m/z 622.0 [M+H]⁺;
Resolution conditions: instrument: SFC-150 (Waters); chromatographic column: 0.46 cm I, D. *5 cm L; mobile phase: CO₂/EtOH = 70/30; flow rate: 2.5 mL/min; back pressure: 2000 psi; detection wavelength: 254 nm; cycle time: 5.0 minutes; the retention time of active compound 13-p2 under this method was 3.033 min.

Compound 13-p1 (retention time 0.987 min): ¹H NMR (400 MHz, DMSO-*d*₆) δ 10.49 (s, 1H), 8.59 (d, J = 2.2 Hz, 1H), 8.02 (dt, J = 7.7, 1.0 Hz, 1H), 7.99-7.92 (m, 1H), 7.91-7.80 (m, 3H), 7.79 (s, 1H), 7.66 (ddd, J = 7.8, 6.8, 2.0 Hz, 1H), 7.39-7.30 (m, 2H), 7.09 (ddd, J = 8.8, 8.0, 3.1 Hz, 1H), 6.97 (dd, J = 9.1, 3.1 Hz, 1H), 6.46 (d, J = 2.0 Hz, 1H), 4.45 (s, 3H).

Compound 13-p2 (retention time 3.033 min): ¹H NMR (400 MHz, DMSO-*d*₆) δ 10.49 (s, 1H), 8.59 (d, J = 2.2 Hz, 1H), 8.06-7.99 (m, 1H), 7.99-7.94 (m, 1H), 7.92-7.81 (m, 3H), 7.79 (s, 1H), 7.66 (ddd, J = 7.8, 6.8, 2.0 Hz, 1H), 7.39-7.31 (m, 2H), 7.09 (ddd, J = 8.9, 8.0, 3.1 Hz, 1H), 6.97 (dd, J = 9.1, 3.1 Hz, 1H), 6.51-6.42 (m, 1H), 4.45 (s, 3H).

### Example 11 Synthetic route of compound 14 (14-p1 and 14-p2)

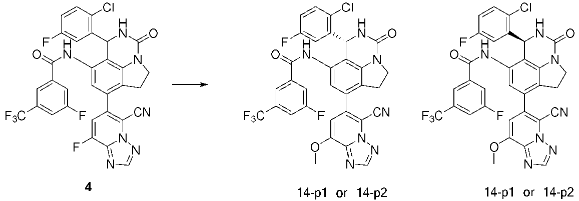

### Synthesis of compound 14-p1 and 14-p2

Compound 4 (1 g, 1.497 mmol), aqueous ammonia (16 mL) and methanol (8 mL) were placed in a reaction flask, and the mixture was stirred at room temperature overnight. Methanol was evaporated under reduced pressure, and the residue was extracted with ethyl acetate and water. The organic phase was concentrated under reduced pressure, purified by normal-phase column chromatography and resolved by SFC to afford compound 14-1 (98 mg) and compound 14-2 (102.5 mg). LCMS (ESI): m/z 680.0 [M+H]⁺; resolution conditions: instrument: SFC-150 (Waters); chromatographic column: 0.46 cm I, D. *5 cm L; mobile phase: CO₂/EtOH = 70/30; flow rate: 2.5 mL/min; back pressure: 2000 psi; detection wavelength: 254 nm; cycle time: 5.0 minutes; the retention time of active compound 14-p2 under this method was 4.026 min;
Compound 14-p1 (retention time 1.235 min): ¹H NMR (400 MHz, DMSO-*d*₆) δ 10.35 (s, 1H), 8.69 (s, 1H), 7.96 (d, J = 8.6 Hz, 1H), 7.90 (d, J = 18.0 Hz, 2H), 7.66 (d, J = 2.7 Hz, 1H), 7.38 (dd, J = 8.9, 5.2 Hz, 1H), 7.33 (s, 1H), 7.14 (ddd, J = 8.9, 8.0, 3.1 Hz, 1H), 6.98 (s, 1H), 6.94 (dd, J = 9.1, 3.1 Hz, 1H), 6.22-6.16 (m, 1H), 4.14 (s, 3H), 4.11-3.97 (m, 1H), 3.39 (d, J = 17.4 Hz, 1H), 3.23 (ddd, J = 16.4, 10.3, 5.9 Hz, 1H).
Compound 14-p2 (retention time 4.026 min): ¹H NMR (400 MHz, DMSO-*d*₆) δ 10.35 (s, 1H), 8.69 (s, 1H), 7.96 (d, J = 8.5 Hz, 1H), 7.94-7.87 (m, 2H), 7.66 (d, J = 2.7 Hz, 1H), 7.38 (dd, J = 8.8, 5.2 Hz, 1H), 7.33 (s, 1H), 7.14 (ddd, J = 8.9, 8.0, 3.1 Hz, 1H), 6.98 (s, 1H), 6.94 (dd, J = 9.1, 3.1 Hz, 1H), 6.23-6.16 (m, 1H), 4.14 (s, 3H), 4.12-3.96 (m, 2H), 3.42-3.36 (m, 1H), 3.23 (ddd, J = 16.5, 10.4, 6.0 Hz, 1H).

### Example 12 Synthetic route of compound 15-p1 and 15-p2

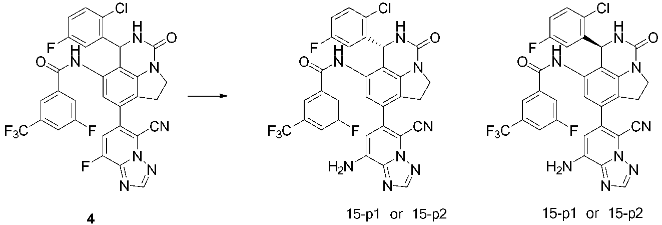

### Synthesis of compound 15-p1 and 15-p2

Compound 4 (1 g, 1.497 mmol), aqueous ammonia (16 mL) and methanol (8 mL) were placed in a reaction flask, and the mixture was stirred at room temperature overnight. Methanol was removed under reduced pressure, and the residue was extracted with ethyl acetate and water. The organic phase was concentrated under reduced pressure, purified by normal-phase column chromatography and resolved by SFC to afford compound 15-1 (196 mg) and compound 15-2 (191 mg). LCMS (ESI): m/z 665.0 [M+H]⁺;
Compound 15-p1: ¹H NMR (400 MHz, DMSO-*d*₆) δ 10.29 (s, 1H), 8.59 (s, 1H), 7.96 (d, J = 8.2 Hz, 1H), 7.93-7.85 (m, 2H), 7.61 (d, J = 2.5 Hz, 1H), 7.45 (s, 2H), 7.37 (dd, J = 8.9, 5.2 Hz, 1H), 7.13 (ddd, J = 8.8, 7.9, 3.1 Hz, 1H), 6.93 (dd, J = 9.2, 3.1 Hz, 1H), 6.87 (s, 1H), 6.59 (s, 1H), 6.20-6.13 (m, 1H), 4.14-3.96 (m, 2H), 3.22 (q, J = 7.8 Hz, 2H).
Compound 15-p2: ¹H NMR (400 MHz, DMSO-*d*₆) δ 10.29 (s, 1H), 8.58 (s, 1H), 7.96 (d, J = 8.4 Hz, 1H), 7.92-7.83 (m, 2H), 7.61 (d, J = 2.6 Hz, 1H), 7.45 (s, 2H), 7.37 (dd, J = 8.9, 5.1 Hz, 1H), 7.13 (ddd, J = 8.9, 7.9, 3.1 Hz, 1H), 6.93 (dd, J = 9.2, 3.1 Hz, 1H), 6.87 (s, 1H), 6.59 (s, 1H), 6.19-6.15 (m, 1H), 4.14-3.94 (m, 2H), 3.27-3.16 (m, 2H).

Resolution conditions: instrument: SFC-150 (Waters); chromatographic column: 0.46 cm I, D. *5 cm L; mobile phase: CO₂/EtOH = 70/30; flow rate: 2.5 mL/min; back pressure: 2000 psi; detection wavelength: 254 nm; cycle time: 5.0 minutes; compound 15-p2, with a retention time of 3.381 min, is the active compound.

### Example 13 Synthetic route of compound 16

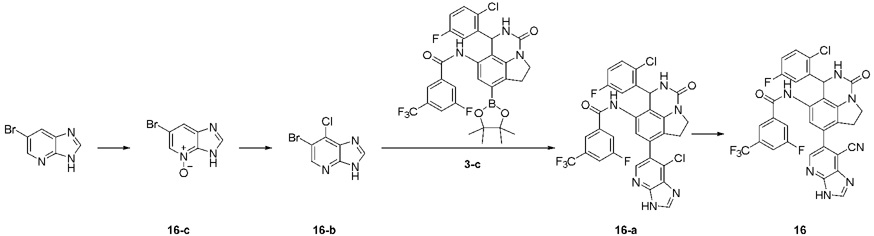

### Synthesis of compound 16-c

To a 100 mL three-necked flask were added 6-bromo-3H-imidazo[5,4-b]pyridine (3000 mg, 15.150 mmol), acetic acid (40 mL) and m-chloroperoxybenzoic acid (5228.56 mg, 30.300 mmol) in sequence. The mixture was stirred at room temperature for 18 hours under nitrogen protection. LC-MS indicated the reaction was complete. The reaction mixture was concentrated to dryness, and 50 mL of ethyl acetate was added. The mixture was heated to 80 °C and stirred for 1 hour, then filtered. The filter cake was washed with ethyl acetate and dried to afford 6-bromoimidazo[5,4-b]pyridine 4-oxide (2900 mg, 13.550 mmol, 89.44%). LC-MS (ESI): m/z 215.9 [M+H]⁺.

### Synthesis of compound 16-b

To a 25 mL microwave tube were added compound 16-c (5000 mg, 23.362 mmol) and phosphorus oxychloride (15 mL) in sequence. The mixture was subjected to microwave reaction at 80 °C for 30 minutes under nitrogen protection. LC-MS indicated the reaction was complete. The reaction mixture was poured into ice water, adjusted to pH approximately 6 with 2 N sodium hydroxide solution, and extracted with ethyl acetate. The organic phase was concentrated and purified by column chromatography (dichloromethane:methanol = 30:1 to 20:1) to afford compound 16-b (310 mg, 1.334 mmol, 5.71%). LC-MS (ESI): m/z 233.8 [M+H]⁺.

### Synthesis of compound 16-a

To a 25 mL microwave tube were added compound 3-c, compound 16-b (220.80 mg, 0.950 mmol), cesium carbonate (928.40 mg, 2.849 mmol), 1,1'-bis(diphenylphosphino)ferrocene]dichloropalladium(II) (69.50 mg, 0.095 mmol), N,N-dimethylformamide (10 mL) and water (2 mL) in sequence. The mixture was reacted at 120 °C under microwave irradiation for 30 minutes under nitrogen protection. LC-MS indicated the reaction was successful. Water and ethyl acetate were added to the reaction mixture, and the organic phase was concentrated and purified by column chromatography (dichloromethane:methanol = 20:1 to 10:1) to afford compound 16-a (102 mg, 0.155 mmol, 16.29%). LC-MS (ESI): m/z 659.1 [M+H]⁺.

### Synthesis of compound 16

To a 10 mL microwave tube were added compound 16-a (80 mg, 0.121 mmol), zinc cyanide (21.37 mg, 0.182 mmol), zinc powder (1.59 mg, 0.024 mmol), 1,1'-bis(diphenylphosphino)ferrocene (20.18 mg, 0.036 mmol), tris(dibenzylideneacetone)dipalladium(O) (16.66 mg, 0.018 mmol) and N,N-dimethylacetamide (4 mL) in sequence. The mixture was subjected to microwave reaction at 170 °C for 1 hour under nitrogen protection. LC-MS indicated the reaction was successful. Water and ethyl acetate were added successively to the reaction mixture. The organic phase was concentrated and purified by column chromatography (dichloromethane:methanol = 30:1 to 20:1) to afford compound 16 (8 mg, 0.012 mmol, 10.14%). LC-MS (ESI): m/z 650.1 [M+H]⁺; ¹H NMR (400 MHz, DMSO-*d*₆) δ 10.32 (s, 1H), 8.70 (s, 1H), 8.54 (s, 1H), 8.10-7.81 (m, 3H), 7.61 (d, J = 2.6 Hz, 1H), 7.37 (dd, J = 8.9, 5.2 Hz, 1H), 7.22-7.08 (m, 1H), 6.95 (dd, J = 9.1, 3.1 Hz, 1H), 6.88 (s, 1H), 6.23-6.13 (m, 1H), 4.46-3.72 (m, 2H), 3.31-3.01 (m, 2H).

### Example 14 Synthetic route of compound 17

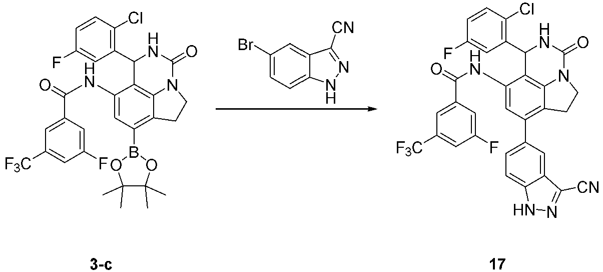

### Synthesis of compound 17

To a 50 mL three-necked flask were added compound 3-c (785.29 mg, 1.239 mmol), 5-bromo-1H-indazole-3-carbonitrile (275 mg, 1.239 mmol), potassium carbonate (513.69 mg, 3.717 mmol), [1,1'-bis(diphenylphosphino)ferrocene]dichloropalladium(II) (90.66 mg, 0.124 mmol), 1,4-dioxane (20 mL) and water (4 mL) in sequence. The mixture was stirred at 100 °C for 2 hours under nitrogen protection. LC-MS indicated the reaction was successful. Ethyl acetate and water were added, and the organic phase was concentrated and purified by column chromatography (dichloromethane:methanol = 20:1 to 10:1) to afford compound 17 (81 mg, 0.125 mmol, 10.07%). LC-MS (ESI): m/z 649.2 [M+H]⁺. ¹H NMR (400 MHz, DMSO-*d*₆) δ 10.24 (s, 1H), 8.04-7.85 (m, 5H), 7.76 (dd, J = 8.8, 1.6 Hz, 1H), 7.58 (d, J = 2.7 Hz, 1H), 7.36 (dd, J = 8.9, 5.2 Hz, 1H), 7.12 (td, J = 8.3, 3.1 Hz, 1H), 7.00 (s, 1H), 6.95 (dd, J = 9.1, 3.1 Hz, 1H), 6.18 (d, J = 2.6 Hz, 1H), 4.02 (dtd, J = 31.1, 10.3, 6.5 Hz, 2H), 3.46 (ddd, J = 17.3, 10.1, 7.2 Hz, 2H).

### Example 15 Synthetic route of compound 18

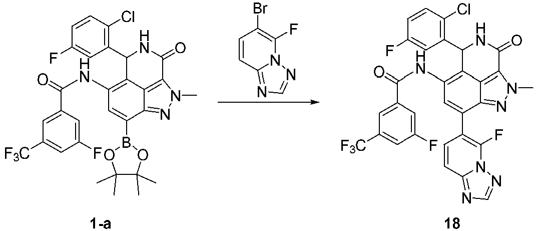

### Synthesis of compound 18

To a 50 mL three-necked flask were added compound 1-a (431 mg, 0.666 mmol), 6-bromo-5-fluoro-[1,2,4]triazolo[1,5-a]pyridine (259.09 mg, 1.199 mmol), potassium carbonate (276.27 mg, 1.999 mmol), [1,1'-bis(diphenylphosphino)ferrocene]dichloropalladium(II) (48.76 mg, 0.067 mmol), 1,4-dioxane (10 mL) and water (2 mL) in sequence. The mixture was stirred at 100 °C for 1 hour under nitrogen protection. LC-MS indicated the reaction was successful. Ethyl acetate and water were added, and the organic phase was concentrated and purified by column chromatography (dichloromethane:methanol = 20:1 to 10:1) to afford compound 18 (62 mg, 0.095 mmol, 14.18%). LC-MS (ESI): m/z 656.1 [M+H]⁺. ¹H NMR (400 MHz, DMSO-*d*₆) δ 10.51 (s, 1H), 8.70 (s, 1H), 8.61 (d, J = 2.2 Hz, 1H), 8.37 (dd, J = 9.2, 7.6 Hz, 1H), 7.95 (t, J = 8.5 Hz, 2H), 7.85 (d, J = 9.2 Hz, 1H), 7.79 (s, 1H), 7.55 (d, J = 1.1 Hz, 1H), 7.35 (dd, J = 8.8, 5.2 Hz, 1H), 7.09 (td, J = 8.4, 3.1 Hz, 1H), 6.98 (dd, J = 9.1, 3.1 Hz, 1H), 6.46 (s, 1H), 4.49 (s, 3H).

### Example 16 Synthetic route of compound 19

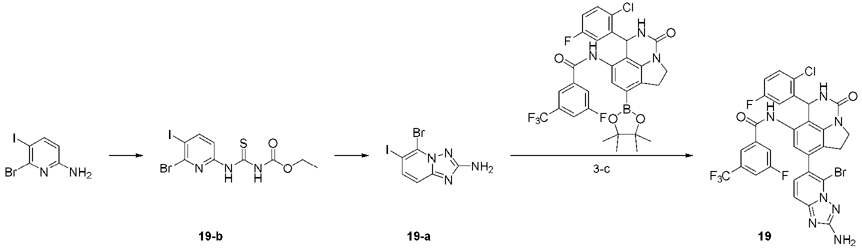

### Synthesis of compound 19-b

To a 50 mL three-necked flask were added 6-bromo-5-iodopyridin-2-amine (1000 mg, 3.345 mmol), ethyl isothiocyanatoformate (526.51 mg, 4.015 mmol) and 1,4-dioxane (15 mL) in sequence. The mixture was stirred at room temperature for 18 hours under nitrogen protection. LC-MS indicated the reaction was successful. The reaction mixture was concentrated and slurried with petroleum ether to afford compound 19-b (1320 mg, 3.069 mmol, 91.75%). LC-MS (ESI): m/z 431.9 [M+H]⁺.

### Synthesis of compound 19-a

To a 100 mL three-necked flask were added hydroxylamine hydrochloride (1066.44 mg, 15.347 mmol), N,N-diisopropylethylamine (1190.14 mg, 9.208 mmol), ethanol (10 mL) and methanol (10 mL) in sequence. The mixture was stirred at room temperature for 1 hour under nitrogen protection. Compound 19-b (1320 mg, 3.069 mmol) was then added, and the mixture was heated to 70 °C and stirred for 18 hours. LC-MS indicated the reaction was complete. After cooling to room temperature, the mixture was filtered, and the filter cake was washed with ethyl acetate and dried to afford compound 19-a (853 mg, 2.517 mmol, 82.00%). LC-MS (ESI): m/z 340.9 [M+H]⁺.

### Synthesis of compound 19

To a 50 mL three-necked flask were added compound 3-c (634 mg, 1.000 mmol), compound 19-a (339.03 mg, 1.000 mmol), potassium carbonate (414.72 mg, 3.001 mmol), [1,1'-bis(diphenylphosphino)ferrocene]dichloropalladium(II) (73.19 mg, 0.100 mmol), 1,4-dioxane (15 mL) and water (3 mL) in sequence. The mixture was stirred at 75 °C for 2 hours under nitrogen protection. LC-MS indicated the reaction was successful. Ethyl acetate and water were added to the reaction mixture, and the organic phase was concentrated and purified by column chromatography (dichloromethane:methanol = 20:1 to 10:1) to afford compound 19 (275 mg, 0.383 mmol, 38.24%). LC-MS (ESI): m/z 720.0 [M+H]⁺; ¹H NMR (400 MHz, DMSO-*d*₆) δ 10.26 (s, 1H), 8.02-7.82 (m, 3H), 7.57 (d, J = 2.6 Hz, 1H), 7.44 (s, 2H), 7.37 (dd, J = 8.9, 5.1 Hz, 1H), 7.13 (td, J = 8.4, 3.1 Hz, 1H), 6.92 (dd, J = 9.1, 3.1 Hz, 1H), 6.74 (s, 1H), 6.35 (s, 2H), 6.19-6.09 (m, 1H), 4.22-3.83 (m, 2H), 3.08 (qt, J = 16.5, 7.5 Hz, 2H).

### Example 17 Synthetic route of compound 20 (20-p1 and 20-p2)

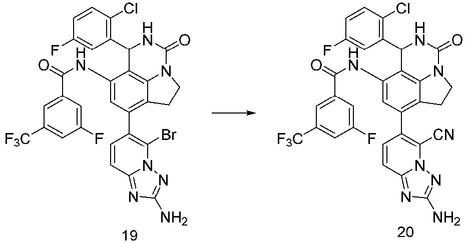

### Synthesis of compound 20, 20-p1 and 20-p2

To a 10 mL microwave tube were added compound 19 (172 mg, 0.239 mmol), zinc cyanide (42.14 mg, 0.359 mmol), zinc powder (1.56 mg, 0.024 mmol), 4,5-bis(diphenylphosphino)-9,9-dimethylxanthene (55.38 mg, 0.096 mmol), palladium acetate (10.74 mg, 0.048 mmol) and N,N-dimethylacetamide (4 mL) in sequence. The mixture was stirred at 120 °C for 6 hours under nitrogen protection. LC-MS indicated the reaction was successful. Water and ethyl acetate were added, and the organic phase was concentrated and purified by column chromatography (dichloromethane:methanol = 20:1 to 10:1) to afford compound 20 (105 mg, 0.158 mmol, 65.99%). LC-MS (ESI): m/z 665.1 [M+H]⁺; ¹H NMR (400 MHz, DMSO-*d*₆) δ 10.32 (s, 1H), 8.00-7.85 (m, 3H), 7.77 (d, J = 9.1 Hz, 1H), 7.70-7.58 (m, 2H), 7.37 (dd, J = 8.9, 5.2 Hz, 1H), 7.13 (td, J = 8.4, 3.1 Hz, 1H), 6.94 (d, J = 9.5 Hz, 2H), 6.58 (s, 2H), 6.24-6.14 (m, 1H), 4.19-3.93 (m, 2H), 3.31-3.08 (m, 2H). Resolved by SFC to give compound 20-p1 (25 mg) and compound 20-2 35.6 mg.

Resolution conditions: instrument: SFC-150 (Waters); chromatographic column: (R, R) Whelk-O1 4.6*100 mm, 5 µm; flow rate: 3.0 mL/min; back pressure: 2000 psi; detection wavelength: 254 nm; cycle time: 5.0 minutes; active compound 20-p1 was eluted at 1.35 min.

### Example 19 Synthetic route of compound 22

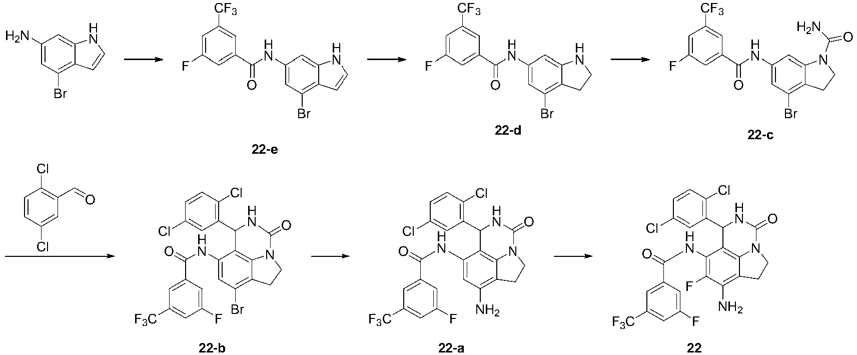

### Synthesis of compound 22-e

To a reaction flask were added 4-bromo-1H-indol-6-amine (13 g, 61.594 mmol), dichloromethane (200 mL) and trifluoroacetic acid (17.123 mL, 123.188 mmol). Under nitrogen atmosphere, 5-fluoro-3-(trifluoromethyl)benzoyl chloride (13 g, 61.594 mmol) was added dropwise slowly in an ice-water bath. The mixture was stirred at room temperature for 2 hours. After completion of the reaction, aqueous solution was added. The organic phase was separated, and the aqueous phase was extracted with dichloromethane. The combined organic phases were washed with saturated aqueous NaCl solution, dried over anhydrous Na₂SO₄, filtered and concentrated under reduced pressure. The resulting crude product was purified by automatic column chromatography (mobile phase: dichloromethane/methanol) to afford the target compound 22-e (23.7 g, 95%) as a yellow solid. LC-MS (ESI): m/z 401.1 [M+H]⁺.

### Synthesis of compound 22-d

To a reaction flask were added compound 22-e (23.7 g, 59.116 mmol) and acetic acid (500 mL). Under nitrogen protection, sodium cyanoborohydride (7.43 g, 118.22 mmol) was added portionwise in an ice-water bath. The mixture was stirred at room temperature overnight. After completion of the reaction, acetic acid was removed under reduced pressure. The residue was dissolved in dichloromethane, water was added, and the organic phase was separated. The aqueous phase was extracted with dichloromethane. The combined organic phases were washed with saturated aqueous NaCl solution, dried over anhydrous Na₂SO₄, filtered and concentrated under reduced pressure. The crude product was purified by automatic column chromatography (mobile phase: petroleum ether/ethyl acetate) to afford target compound 22-d (11 g, 46%). LC-MS (ESI): m/z 403.1 [M+H]⁺.

### Synthesis of compound 22-c

To a reaction flask were added compound 22-d (7.4 g, 18.35 mmol) and acetic acid (80 mL). Under nitrogen protection, potassium cyanate (2.97 g, 36.709 mmol) was added in an ice-water bath. The ice-water bath was maintained, and the mixture was stirred for 30 minutes. Upon completion of the reaction, acetic acid was removed under reduced pressure. Water was added, and the mixture was adjusted to weakly basic with saturated aqueous sodium bicarbonate solution. After thorough stirring, the mixture was filtered. The filter cake was suspended in water and lyophilized to afford the target compound 22-c (6.7 g, 77%). LC-MS (ESI): m/z 446.1 [M+H]⁺.

### Synthesis of compound 22-b

Polyphosphoric acid was added to a reaction flask and heated to 105 °C. Compound 22-c (4.7 g, 10.533 mmol) and 2,5-dichlorobenzaldehyde (3.69 g, 21.066 mmol) were added successively, and the mixture was stirred for 1 hour. After completion of the reaction, the mixture was poured into ice-cold aqueous sodium hydroxide solution and stirred thoroughly until no viscous material remained. The mixture was extracted with dichloromethane. The combined organic phases were washed with saturated aqueous NaCl solution, dried over anhydrous Na₂SO₄, filtered and concentrated under reduced pressure. The crude product was purified by automatic column chromatography (mobile phase: dichloromethane/methanol) to afford the target compound 22-b (4 g, 66%). LC-MS (ESI): m/z 602.19 [M+H]⁺.

### Synthesis of compound 22-a

To a microwave tube were added compound 22-b (800 mg, 1.326 mmol), L-proline (60.95 mg, 0.530 mmol), potassium carbonate (549.76 mg, 3.978 mmol), and CuI (50.51 mg, 0.265 mmol), followed by addition of DMSO (6 mL). The system was purged with nitrogen 3 to 5 times. Aqueous ammonia (25%) (116.19 mg, 3.315 mmol) was injected via syringe, and the mixture was heated to 100 °C and stirred overnight. The reaction was monitored, then extracted with ethyl acetate, and the organic layer was washed with water twice. Purification by normal-phase column chromatography (dichloromethane:methanol = 20:1) afforded the target compound 22-a (530 mg, 74%).

### Synthesis of compound 22

To a 50 mL three-necked flask were added compound 22-a (500 mg, 0.927 mmol), 1-chloromethyl-4-fluoro-1,4-diazoniabicyclo[2.2.2]octane bis(tetrafluoroborate) (361.28 mg, 1.020 mmol) and acetic acid (10 mL) in sequence. The mixture was stirred at room temperature for 18 hours under nitrogen protection. LC-MS indicated the reaction was successful. Excess acetic acid was evaporated under reduced pressure, saturated sodium bicarbonate solution and dichloromethane were added, and the organic phase was concentrated and purified by column chromatography (dichloromethane:methanol = 20:1 to 10:1) to afford compound 22 (70 mg, 0.126 mmol, 13.55%). LC-MS (ESI): m/z 557.2 [M+H]⁺. ¹H NMR (400 MHz, DMSO-*d*₆) δ 9.91 (s, 1H), 8.18-7.71 (m, 3H), 7.43-7.09 (m, 3H), 7.01 (d, J = 2.4 Hz, 1H), 5.85 (d, J = 2.4 Hz, 1H), 5.40 (s, 2H), 4.17-3.55 (m, 2H), 3.02 (t, J = 8.5 Hz, 2H).

### Example 20 Synthetic route of compound 23-p1 and 23-p2

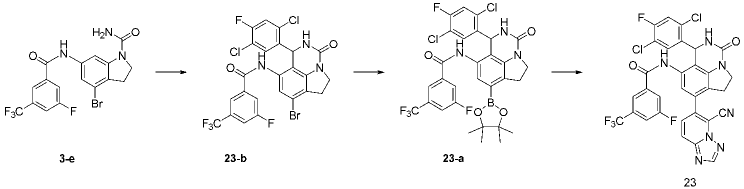

### Synthesis of compound 23-b

To a 100 mL three-necked flask were added compound 3-e (500 mg, 1.121 mmol), 2,5-dichloro-4-fluorobenzaldehyde (324.41 mg, 1.681 mmol) and Eaton's reagent (30 mL) in sequence. The mixture was stirred at 90 °C for 1.5 hours under nitrogen protection. LC-MS indicated the reaction was successful. The reaction mixture was poured into water, filtered, washed with water, and the filter cake was dried to afford compound 23-b (650 mg, 1.046 mmol, 93.38%). LC-MS (ESI): m/z 622.2 [M+H]⁺.

### Synthesis of compound 23-a

To a 50 mL three-necked flask were added compound 23-b (650 mg, 1.046 mmol), bis(pinacolato)diboron (531.44 mg, 2.093 mmol), potassium acetate (308.08 mg, 3.139 mmol), [1,1'-bis(diphenylphosphino)ferrocene]dichloropalladium(II) (76.57 mg, 0.105 mmol) and 1,4-dioxane (15 mL) in sequence. The mixture was stirred at 100 °C for 1.5 hours under nitrogen protection. LC-MS indicated the reaction was successful, and the reaction mixture was used directly in the next step. LC-MS (ESI): m/z 668.3 [M+H]⁺.

### Synthesis of compound 23

To a 50 mL three-necked flask were added compound 23-a (699 mg, 1.046 mmol), 6-bromo-[1,2,4]triazolo[1,5-a]pyridine-5-carbonitrile (513.24 mg, 2.301 mmol), potassium carbonate (433.68 mg, 3.138 mmol), [1,1'-bis(diphenylphosphino)ferrocene]dichloropalladium(II) (76.54 mg, 0.105 mmol), 1,4-dioxane (15 mL) and water (3 mL) in sequence. The mixture was stirred at 100 °C for 1 hour under nitrogen protection. LC-MS indicated the reaction was successful. The reaction mixture was concentrated under reduced pressure and purified by column chromatography (dichloromethane:methanol = 20:1 to 10:1) to afford compound 23 (470 mg, 0.687 mmol, 65.65%). LC-MS (ESI): m/z 684.3 [M+H]⁺. ¹H NMR (400 MHz, DMSO-*d*₆) δ 10.30 (s, 1H), 8.79 (s, 1H), 8.31 (d, J = 9.3 Hz, 1H), 8.03-7.80 (m, 4H), 7.61 (d, J = 9.1 Hz, 1H), 7.52 (d, J = 2.3 Hz, 1H), 7.25 (d, J = 7.8 Hz, 1H), 6.96 (s, 1H), 6.13 (d, J = 2.2 Hz, 1H), 4.03 (qd, J = 7.8, 7.0, 5.6 Hz, 2H), 3.24 (ddd, J = 13.6, 9.7, 7.0 Hz, 2H).

Resolved by SFC to give compound 23-p1 140 mg and compound 23-p2 145 mg.

Resolution conditions: instrument: SFC-150 (Waters); chromatographic column: (R, R) Whelk-O1 4.6*100mm 5µm; mobile phase: CO₂/EtOH = 30/70; flow rate: 3.0 mL/min; back pressure: 2000 psi; detection wavelength: 254 nm; cycle time: 5.0 minutes;
Compound 23-p1 (retention time 2.617 min): LC-MS (ESI): m/z 684.3 [M+H]⁺. ¹H NMR (400 MHz, DMSO-*d*₆) δ 10.32 (s, 1H), 8.74 (s, 1H), 8.34 (d, J = 9.3 Hz, 1H), 8.01-7.81 (m, 4H), 7.61 (d, J = 9.1 Hz, 1H), 7.52 (d, J = 2.3 Hz, 1H), 7.25 (d, J = 7.8 Hz, 1H), 6.96 (s, 1H), 6.13 (d, J = 2.2 Hz, 1H), 4.02 (qd, J = 7.8, 7.0, 5.6 Hz, 2H), 3.24 (ddd, J = 13.6, 9.7, 7.0 Hz, 2H).
Compound 23-p2 (retention time 3.677 min): LC-MS (ESI): m/z 684.3 [M+H]⁺. ¹H NMR (400 MHz, DMSO-*d*₆) δ 10.30 (s, 1H), 8.79 (s, 1H), 8.34 (d, J = 9.3 Hz, 1H), 8.03-7.80 (m, 4H), 7.61 (d, J = 9.1 Hz, 1H), 7.52 (d, J = 2.3 Hz, 1H), 7.26 (d, J = 7.8 Hz, 1H), 6.96 (s, 1H), 6.13 (d, J = 2.2 Hz, 1H), 4.03 (qd, J = 7.8, 7.0, 5.6 Hz, 2H), 3.26 (m, 2H).

### Example 21 Synthetic route of compound 24

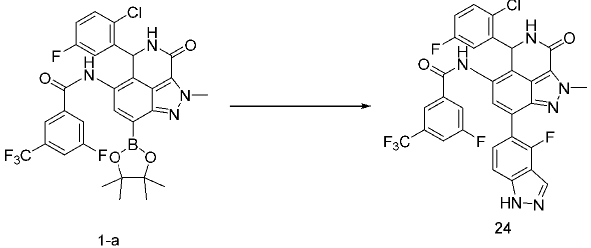

### Synthesis of compound 24

To a 50 mL three-necked flask were added compound 1-a (377 mg, 0.583 mmol), 5-bromo-4-fluoro-1H-indazole (213.12 mg, 0.991 mmol), potassium carbonate (241.65 mg, 1.749 mmol), [1,1'-bis(diphenylphosphino)ferrocene]dichloropalladium(II) (42.65 mg, 0.058 mmol), 1,4-dioxane (10 mL) and water (2 mL) in sequence. The mixture was stirred at 100 °C for 1 hour under nitrogen protection. LC-MS indicated the reaction was successful. Ethyl acetate and water were added, and the organic phase was concentrated and purified by column chromatography (dichloromethane:methanol = 20:1 to 10:1) to afford compound 24 (93 mg, 0.142 mmol, 24.35%). LC-MS (ESI): m/z 655.1 [M+H]⁺.

¹H NMR (400 MHz, DMSO-*d*₆) δ 13.52 (s, 1H), 10.45 (s, 1H), 8.54 (d, J = 2.2 Hz, 1H), 8.28 (t, J = 1.2 Hz, 1H), 8.04-7.82 (m, 3H), 7.79 (s, 1H), 7.55 (dd, J = 8.6, 1.1 Hz, 1H), 7.42-7.28 (m, 2H), 7.09 (ddd, J = 8.8, 8.0, 3.1 Hz, 1H), 6.96 (dd, J = 9.1, 3.1 Hz, 1H), 6.45 (d, J = 2.0 Hz, 1H), 4.47 (s, 3H).

### Example 22 Synthetic route of compound 25

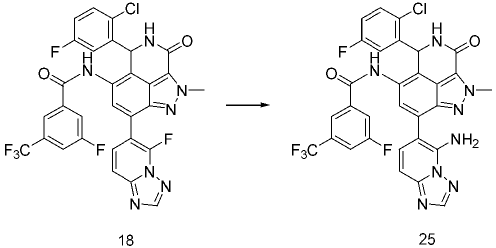

### Synthesis of compound 25

To a 50 mL three-necked flask were added compound 18 (200 mg, 0.305 mmol) and ammonia solution in 1,4-dioxane (7 mL) in sequence. The mixture was stirred at room temperature for 18 hours under nitrogen protection. LC-MS indicated the reaction was successful. The reaction mixture was concentrated and purified by column chromatography (dichloromethane:methanol = 20:1 to 10:1) to afford compound 25 (112 mg, 0.172 mmol, 56.26%). LC-MS (ESI): m/z 653.3 [M+H]⁺. ¹H NMR (400 MHz, DMSO-*d*₆) δ 10.44 (s, 1H), 8.56 (d, J = 2.3 Hz, 1H), 8.49 (s, 1H), 7.95 (dt, J = 8.5, 1.9 Hz, 1H), 7.84 (dt, J = 9.4, 1.9 Hz, 1H), 7.80-7.72 (m, 2H), 7.38-7.27 (m, 2H), 7.15 (d, J = 8.9 Hz, 1H), 7.12-7.05 (m, 1H), 7.00-6.89 (m, 3H), 6.51 (d, J = 2.1 Hz, 1H), 4.46 (s, 3H).

### Example 23 Synthetic route of compound 26

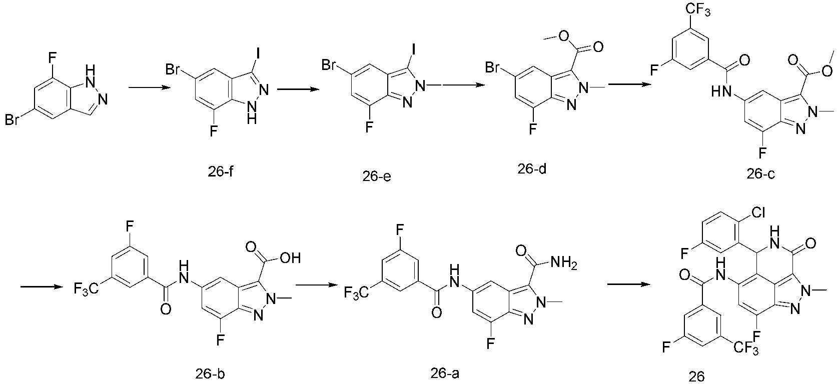

### Synthesis of compound 26-f

To a 250 mL three-necked flask were added 5-bromo-7-fluoro-1H-indazole (5075 mg, 23.601 mmol), iodine (11980.52 mg, 47.203 mmol), potassium hydroxide (5297.09 mg, 94.405 mmol) and N,N-dimethylformamide (60 mL) in sequence. The mixture was stirred at 70 °C for 2 hours under nitrogen protection. LC-MS indicated the reaction was successful. The reaction mixture was adjusted to acidic with 10% citric acid solution, followed by addition of a large amount of ethyl acetate. The organic phase was washed with 5% aqueous sodium thiosulfate solution, concentrated and purified by column chromatography (petroleum ether:ethyl acetate = 20:1 to 10:1) to afford compound 26-f (4400 mg, 12.906 mmol, 54.68%). LC-MS (ESI) M⁻: m/z 338.8.

### Synthesis of compound 26-e

To a 50 mL three-necked flask were added compound 26-f (3700 mg, 10.853 mmol), trimethyloxonium tetrafluoroborate (1765.79 mg, 11.938 mmol) and ethyl acetate (50 mL) in sequence. The mixture was stirred at room temperature overnight under nitrogen protection. LC-MS indicated the reaction was successful. The reaction mixture was filtered to afford compound 26-e (2300 mg, 6.480 mmol, 59.71%).

LC-MS (ESI): m/z 354.9 [M+H]⁺.

### Synthesis of compound 26-d

To a 100 mL three-necked flask were added compound 26-e (2650 mg, 7.466 mmol), triethylamine (3.113 mL, 22.398 mmol), [1,1'-bis(diphenylphosphino)ferrocene]dichloropalladium(II) (546.28 mg, 0.747 mmol), N,N-dimethylformamide (15 mL) and methanol (15 mL) in sequence. The mixture was stirred at 70 °C for 3 hours under carbon monoxide protection. LC-MS indicated the reaction was successful. Methanol was evaporated under reduced pressure, ethyl acetate and water were added, and the organic phase was concentrated and purified by column chromatography (petroleum ether:ethyl acetate = 20:1 to 10:1) to afford compound 26-d (1660 mg, 5.782 mmol, 77.45%). LC-MS (ESI): m/z 287.0 [M+H]⁺.

### Synthesis of compound 26-c

To a 50 mL three-necked flask were added compound 26-d (1060 mg, 3.692 mmol), 5-fluoro-3-(trifluoromethyl)benzamide (917.72 mg, 4.431 mmol), cesium carbonate (2406.00 mg, 7.384 mmol), methanesulfonato(2-(di-tert-butylphosphino)-3,6-dimethoxy-2',4',6'-triisopropyl-1,1'-biphenyl)(2'-amino-1,1'-biphenyl-2-yl)palladium(II) (315.85 mg, 0.369 mmol) and 1,4-dioxane (20 mL) in sequence. The mixture was stirred at 90 °C for 3 hours under nitrogen protection. LC-MS indicated the reaction was successful. The reaction mixture was directly concentrated and purified by column chromatography (petroleum ether:ethyl acetate = 10:1 to 5:1) to afford compound 26-c (1420 mg, 3.436 mmol, 93.05%). LC-MS (ESI): m/z 414.1 [M+H]⁺.

### Synthesis of compound 26-b

To a 100 mL three-necked flask were added compound 26-c (1680 mg, 4.065 mmol), lithium hydroxide (487.78 mg, 20.324 mmol), methanol (15 mL), tetrahydrofuran (15 mL) and water (7 mL) in sequence. The mixture was stirred at room temperature for 1 hour under nitrogen protection. LC-MS indicated the reaction was complete. Excess methanol and tetrahydrofuran were evaporated under reduced pressure, and the mixture was adjusted to pH approximately 4 with 1 N hydrochloric acid. Ethyl acetate was added, and the organic phase was concentrated to afford compound 26-b (1600 mg, 4.01 mmol, 98.6%). LC-MS (ESI): m/z 400.1 [M+H]⁺.

### Synthesis of compound 26-a

To a 50 mL three-necked flask were added compound 26-b (1600 mg, 4.007 mmol), ammonium chloride (321.52 mg, 6.011 mmol), HATU (1828.44 mg, 4.809 mmol), N,N-diisopropylethylamine (1553.80 mg, 12.022 mmol) and N,N-dimethylformamide (20 mL) in sequence. The mixture was stirred at room temperature for 1 hour under nitrogen protection. LC-MS indicated the reaction was successful. Water and ethyl acetate were added, and the organic phase was concentrated to afford compound 26-a (1100 mg, 2.762 mmol, 68.92%). LC-MS (ESI): m/z 399.2 [M+H]⁺.

### Synthesis of compound 26

To a 50 mL three-necked flask were added compound 26-a (398 mg, 0.999 mmol), 2-chloro-5-fluorobenzaldehyde (237.67 mg, 1.499 mmol) and Eaton's reagent (10 mL) in sequence. The mixture was stirred at 80 °C for 1.5 hours under nitrogen protection. LC-MS indicated the reaction was successful. The reaction mixture was poured into a large amount of cold saturated sodium bicarbonate solution, and ethyl acetate was added. The organic phase was concentrated and purified by column chromatography (petroleum ether: ethyl acetate = 5:1 to 3:1) to afford compound 26 (220 mg, 0.408 mmol, 40.86%). LC-MS (ESI): m/z 539.2 [M+H]⁺.

### Example 25 Synthetic route of compound 28 (28-p1 and 28-p2)

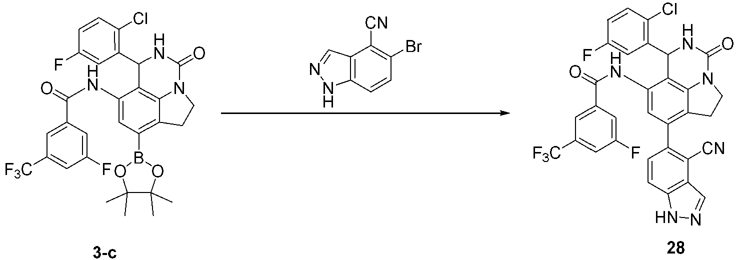

Compound 3-c (800 mg, 1.262 mmol), 5-bromo-1H-indazole-4-carbonitrile (308.29 mg, 1.388 mmol), potassium carbonate (523.31 mg, 3.787 mmol) and Pd(dppf)Cl₂ (138.54 mg, 0.189 mmol) were added to a reaction flask, followed by addition of 1,4-dioxane (85 mL) and water (14 mL). The system was purged with nitrogen and then stirred at 100 °C for 1.5 hours, then diluted with ethyl acetate and water. The organic phase was separated, and the aqueous phase was extracted with ethyl acetate. The combined organic phases were washed with saturated aqueous NaCl solution, dried over anhydrous Na₂SO₄, filtered, and concentrated under reduced pressure. Purification by automatic column chromatography (Biotage) (mobile phase: dichloromethane/methanol = 100/6 to 100/7) afforded the target compound 28 as a solid (180 mg, purity: 93.1%, yield: 20.46%). ESI: (m/z) = 649.2 [M+H]⁺.

Chiral resolution was performed (instrument SFC-150 (Waters); chromatographic column: AD-3, 0.46 cm I.D. *5 cm L; 2 µL(injection); mobile phase: CO₂/EtOH = 70/30; flow rate: 2.5 mL/min; back pressure: 100 bar; detection wavelength: 254 nm; cycle time: 13.8 min) to give compound 28-p1 (retention time: 1.344 min, 70 mg, purity: 97.2%, yield: 8.31%), ¹H NMR (400 MHz, DMSO-*d*₆) δ 13.82 (s, 1H), 10.29 (s, 1H), 8.33 (s, 1H), 7.95 (dt, J = 36.3, 13.1 Hz, 4H), 7.65-7.57 (m, 2H), 7.37 (dd, J = 8.9, 5.1 Hz, 1H), 7.13 (dd, J = 9.7, 6.7 Hz, 1H), 6.94 (dd, J = 9.0, 3.1 Hz, 1H), 6.85 (s, 1H), 6.22-6.15 (m, 1H), 4.01 (dq, J = 18.6, 10.0, 8.4 Hz, 2H), 3.26-3.09 (m, 2H). ESI: (m/z) = 649.2 [M+H]⁺.

compound 28-p2 (retention time: 3.123 min, 65 mg, purity: 99.2%, yield: 7.87%), ¹H NMR (400 MHz, DMSO-*d*₆) δ 13.82 (s, 1H), 10.29 (s, 1H), 8.33 (s, 1H), 8.08-7.84 (m, 4H), 7.65-7.55 (m, 2H), 7.37 (dd, J = 8.9, 5.1 Hz, 1H), 7.13 (td, J = 8.4, 3.1 Hz, 1H), 6.94 (dd, J = 9.2, 3.1 Hz, 1H), 6.85 (s, 1H), 6.19 (d, J = 2.6 Hz, 1H), 4.12-3.93 (m, 2H), 3.07-3.26 (m, 2H). ESI: (m/z) = 649.2 [M+H]⁺.

### Example 26 Synthetic route of compound 29 (29-p1 and 29-p2)

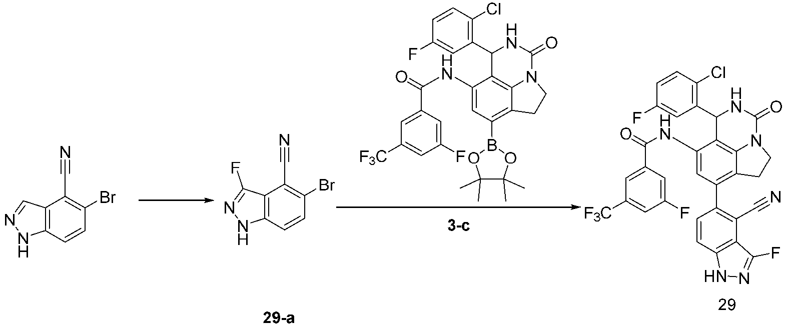

### Synthesis of compound 29-a

To a solution of 5-bromo-1H-indazole-4-carbonitrile (500 mg, 2.252 mmol) in acetonitrile (170 mL) were added 1-chloromethyl-4-fluoro-1,4-diazoniabicyclo[2.2.2]octane bis(tetrafluoroborate) (Selectfluor, 1595.48 mg, 4.504 mmol) and acetic acid (6 mL) at room temperature. The reaction mixture was heated to 90 °C under nitrogen protection and stirred for 16 hours. Additional 1-chloromethyl-4-fluoro-1,4-diazoniabicyclo[2.2.2]octane bis(tetrafluoroborate) (Selectfluor, 1595.48 mg, 4.504 mmol) and acetic acid (6 mL) were added, and the reaction was continued for a total of 48 hours. After completion of the reaction, the mixture was extracted with ethyl acetate. The combined organic phases were washed with water and saturated aqueous NaCl solution, dried over anhydrous Na₂SO₄, filtered, and concentrated under reduced pressure. Purification by column chromatography (mobile phase: methanol/dichloromethane from 1% to 2%) afforded compound 29-a (310 mg, yield: 48.75%, purity: 85%). LC-MS (ESI): m/z 241.0 [M+H]⁺, ¹H NMR (400 MHz, DMSO-*d*₆) δ 13.38 (s, 1H), 7.84-7.77 (m, 2H).

### Synthesis of compound 29

To a reaction flask were added compound 3-c (600 mg, 0.947 mmol), compound 29-a, potassium carbonate (392.48 mg, 2.840 mmol) and Pd(dppf)Cl₂ (103.90 mg, 0.142 mmol), followed by addition of 1,4-dioxane (90 mL) and water (18 mL). The system was purged with nitrogen, and the mixture was stirred at 100 °C for 1.5 hours, then diluted with ethyl acetate and water. The organic phase was separated, and the aqueous phase was extracted with ethyl acetate. The combined organic phases were washed with saturated aqueous NaCl solution, dried over anhydrous Na₂SO₄, filtered, and concentrated under reduced pressure. The crude product was purified by automatic column chromatography (Biotage, mobile phase: dichloromethane/methanol = 100/5 to 100/6), then further purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm). The resulting product was lyophilized to afford the target compound 29 as a solid (80 mg, purity: 96.6%). ESI: (m/z) = 667.2 [M+H]⁺. ¹H NMR (400 MHz, DMSO-*d*₆) δ 10.32 (s, 1H), 7.92 (dt, J = 16.9, 9.2 Hz, 4H), 7.63 (dd, J = 15.3, 5.8 Hz, 2H), 7.37 (dd, J = 8.9, 5.1 Hz, 1H), 7.13 (dt, J = 8.7, 4.4 Hz, 1H), 6.94 (dd, J = 9.1, 3.2 Hz, 1H), 6.86 (s, 1H), 6.19 (s, 1H), 4.11-3.94 (m, 2H), 3.17 (dt, J = 27.2, 7.0 Hz, 2H).

Compound 29 was subjected to SFC resolution (instrument: SFC-150 (Waters); chromatographic column: AD-3, 0.46 cm I.D. * 5 cm. L; 2 µL(injection); mobile phase: CO₂/EtOH = 70/30; flow rate: 2.5 mL/min; back pressure: 100 bar; detection wavelength: 254 nm; cycle time: 13.8 min) to give target compound 29-p2 (retention time: 2.456 min, 38 mg, 0.056 mmol, 5.94%), ¹H NMR (400 MHz, DMSO-*d*₆) δ 13.32 (s, 1H), 10.30 (s, 1H), 7.99-7.85 (m, 4H), 7.69-7.59 (m, 2H), 7.37 (dd, J = 8.9, 5.1 Hz, 1H), 7.13 (td, J = 8.3, 3.1 Hz, 1H), 6.94 (dd, J = 9.1, 3.1 Hz, 1H), 6.86 (s, 1H), 6.18 (d, J = 2.6 Hz, 1H), 4.03 (dtd, J = 20.9, 10.5, 4.9 Hz, 2H), 3.26-3.11 (m, 2H). LC-MS (ESI): m/z 667.2 [M+H]⁺.

### Example 27 Synthetic route of compound 30

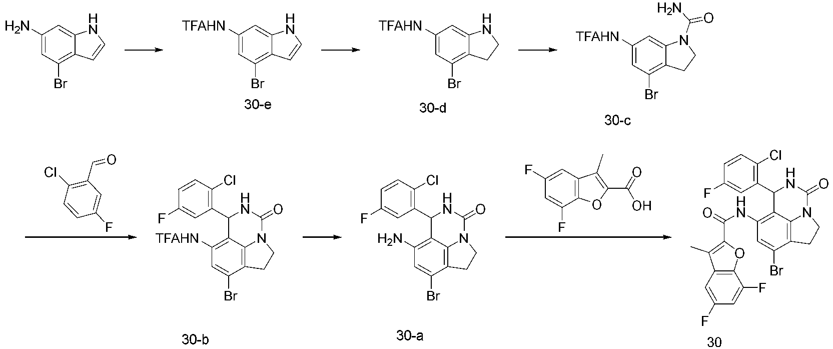

### Synthesis of compound 30-e

4-bromo-1H-indol-6-amine (10 g, 47.380 mmol) was dissolved in dichloromethane (150 mL), and triethylamine (19.757 mL, 142.140 mmol) was added. The system was purged with nitrogen, and trifluoroacetic anhydride (19.90 g, 94.760 mmol) was added at 0 °C. The reaction mixture was warmed to room temperature and stirred for 3 hours. The mixture was diluted with ethyl acetate and water. The organic phase was separated, and the aqueous phase was extracted with ethyl acetate. The combined organic phases were washed with water and saturated aqueous NaCl solution, dried over anhydrous Na₂SO₄, filtered, and concentrated under reduced pressure to afford compound 30-e directly (14.2 g, purity 87.16%, yield 82.15%). ESI: (m/z) = 308.7 [M+H]⁺.

### Synthesis of compound 30-d

Compound 30-e (14.2 g, 46.244 mmol) was dissolved in acetic acid (250 mL). Sodium cyanoborohydride (5.81 g, 92.487 mmol) was added, and the mixture was stirred at room temperature for 16 hours. The reaction mixture was concentrated under reduced pressure, poured into ice-cold saturated aqueous sodium bicarbonate solution, and diluted with dichloromethane and water. The organic phase was separated, and the aqueous phase was extracted with dichloromethane. The combined organic phases were washed with water and saturated aqueous NaCl solution, dried over anhydrous Na₂SO₄, filtered, and evaporated to dryness. Purification by column chromatography (mobile phase: petroleum ether/ethyl acetate = 100/13 to 100/14) afforded compound 30-d (4.6 g, purity 96%, yield 30.90%). ESI: (m/z) = 310.6 [M+H]⁺.

### Synthesis of compound 30-c

Compound 30-d (4.5 g, 14.559 mmol) was dissolved in acetic acid (100 mL). The temperature was cooled to 10 °C, and potassium cyanate (2.36 g, 29.118 mmol) was added. The mixture was stirred at 10 °C for 0.5 h. The reaction mixture was concentrated under reduced pressure, basified with saturated aqueous sodium bicarbonate solution, diluted with water, and filtered to afford the crude product compound 30-c (5.15 g, purity 74.4 %, yield 74.74 %). ESI: (m/z) = 353.8 [M+H]⁺.

### Synthesis of compound 30-b

Compound 30-c (5.1 g, 14.484 mmol) was dissolved in Eaton's reagent (45 mL). 2-chloro-5-fluorobenzaldehyde (4.59 g, 28.968 mmol) was added, and the mixture was stirred at 80 °C for 2 h. The reaction mixture was diluted with ethyl acetate and water. The organic layer was separated, and the aqueous layer was extracted with ethyl acetate. The combined organic layers were washed with water and saturated aqueous NaCl solution, dried over anhydrous Na₂SO₄, filtered, and concentrated under reduced pressure. Purification by column chromatography (eluent: dichloromethane/methanol = 100/3 to 100/5) afforded compound 30-b (6.8 g, purity 70 %, yield 66.71 %). ESI: (m/z) = 493.8 [M+H]⁺.

### Synthesis of compound 30-a

Compound 30-b (6.8 g, 9.662 mmol) was dissolved in methanol (150 mL) and water (50 mL). Lithium hydroxide (10.14 g, 241.551 mmol) was added, and the mixture was stirred at 80 °C for 7 h. The reaction mixture was concentrated under reduced pressure, then diluted with dichloromethane and water. The organic layer was separated, and the aqueous layer was extracted with dichloromethane. The combined organic layers were washed with water and saturated aqueous NaCl solution, dried over anhydrous Na₂SO₄, filtered, and concentrated under reduced pressure to afford crude compound 30-a (3.7 g, purity 63.8 %, yield 61.60 %). ESI: (m/z) = 397.8 [M+H]⁺.

### Synthesis of compound 30

To a reaction flask were added compound 30-a (113.11 mg, 0.285 mmol), 5,7-difluoro-3-methyl-1-benzofuran-2-carboxylic acid (55 mg, 0.259 mmol), HATU (147.87 mg, 0.389 mmol), DMF (25 mL) and triethylamine (78.70 mg, 0.778 mmol). The mixture was stirred at room temperature for 72 h. Although the product was formed, the starting material was not completely consumed, and the reaction was then stopped. The reaction solvent was evaporated under reduced pressure, and the residue was directly loaded and purified by automatic flash chromatography (Biotage, eluent: methanol/dichloromethane = 100/1 to 100/2) to afford crude compound 30 (180 mg, purity: 32.3%). The crude product was further purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm), and then lyophilized to give the target compound 30 as a solid (10 mg, purity: 92%, yield: 6.01%). ESI: (m/z) = 590.0/592.0 [M+H]⁺. ¹H NMR (400 MHz, DMSO-*d*₆) δ 10.06 (s, 1H), 7.57-7.49 (m, 3H), 7.29 (dd, J = 8.8, 5.1 Hz, 1H), 7.05 (td, J = 8.4, 3.1 Hz, 1H), 7.02-6.93 (m, 2H), 6.17 (d, J = 2.5 Hz, 1H), 4.06-3.97 (m, 2H), 3.16 (t, J = 8.1 Hz, 2H), 2.40 (s, 3H).

### Example 28 Synthetic route of compound 31

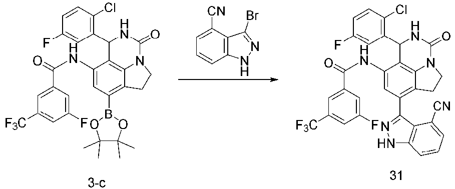

### Synthesis of compound 31

Compound 3-c (500 mg, 0.789 mmol), 5-bromo-3-fluoro-1H-indazole-4-carbonitrile (227.24 mg, 0.947 mmol), potassium carbonate (109.02 mg, 0.789 mmol), and Pd(dppf)Cl₂ (577.23 mg, 0.789 mmol) were added to a reaction flask. 1,4-Dioxane (75 mL) and water (15 mL) were added, and the mixture was degassed and purged with nitrogen. The reaction was stirred at 100 °C for 1.5 h, then diluted with ethyl acetate and water. The organic layer was separated, and the aqueous layer was extracted with ethyl acetate. The combined organic layers were washed with saturated aqueous NaCl solution, dried over anhydrous Na₂SO₄, filtered, and concentrated under reduced pressure. The crude product was purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm), and lyophilized to afford the target compound 31 as a solid (28 mg, 0.043 mmol, 5.41%). ESI: (m/z) = 649.2 [M+H]⁺. ¹H NMR (400 MHz, DMSO-*d*₆) δ 10.32 (s, 1H), 8.01 (d, J = 8.5 Hz, 1H), 7.94 (d, J = 11.5 Hz, 2H), 7.88 (d, J = 9.2 Hz, 1H), 7.77 (d, J = 7.1 Hz, 1H), 7.62-7.54 (m, 2H), 7.36 (dd, J = 8.8, 5.2 Hz, 1H), 7.11 (td, J = 8.4, 3.1 Hz, 1H), 6.98 (s, 1H), 6.92 (dd, J = 9.2, 3.1 Hz, 1H), 6.24 (d, J = 2.6 Hz, 1H), 4.13-3.92 (m, 2H), 3.05 (ddd, J = 16.1, 10.2, 5.4 Hz, 2H).

### Example 29 Synthetic route of compound 32

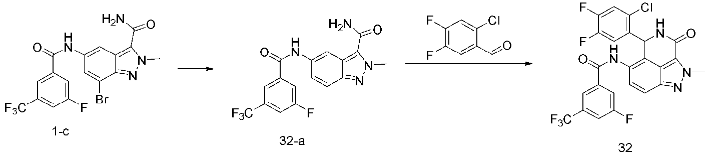

### Synthesis of compound 32-a

To a reaction flask were added compound 1-c (6.7 g, 14.591 mmol), 10% Pd/C (6.21 g, 58.362 mmol) and methanol (515 mL). The mixture was stirred under hydrogen atmosphere at room temperature overnight. After completion of the reaction, the mixture was filtered. Most of the product remained in the filter cake, which was then washed with a large amount of ethyl acetate/methanol mixed solvent. The filtrate was concentrated and purified by automatic flash chromatography (Biotage, eluent: dichloromethane/methanol = 100/5 to 100/6) to afford compound 32-a (3.05 g, 8.020 mmol, 54.97%). ESI: (m/z) = 381.1 [M+H]⁺.

### Synthesis of compound 32

Compound 32-a (558 mg, 1.467 mmol) was dissolved in Eaton's reagent (65 mL). 2-chloro-4,5-difluorobenzaldehyde (388.57 mg, 2.201 mmol) was added, and the mixture was stirred at 90 °C for 3 h under nitrogen atmosphere. Upon completion, the reaction mixture was poured dropwise into ice-cold saturated aqueous sodium bicarbonate solution, then diluted with ethyl acetate and water. The organic layer was separated, and the aqueous layer was extracted with ethyl acetate. The combined organic layers were washed with water and saturated aqueous NaCl solution, dried over anhydrous Na₂SO₄, filtered, and concentrated under reduced pressure. Purification by column chromatography (eluent: dichloromethane/methanol = 100/3 to 100/4) afforded compound 32 (111 mg, purity: 90%), ESI: (m/z) = 539.1 [M+H]⁺. ¹H NMR (400 MHz, DMSO-*d*₆) δ 10.33 (s, 1H), 8.43 (d, J = 2.2 Hz, 1H), 7.97 (dt, J = 8.6, 1.9 Hz, 1H), 7.85 (dt, J = 9.3, 1.9 Hz, 1H), 7.74 (s, 1H), 7.67 (dd, J = 8.9, 1.0 Hz, 1H), 7.56 (dd, J = 10.4, 7.3 Hz, 1H), 7.21-7.10 (m, 2H), 6.36 (s, 1H), 4.44 (s, 3H).

### Example 30 Synthetic route of compound 33

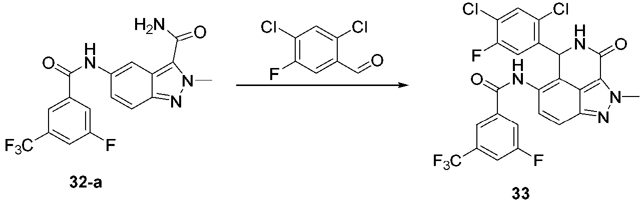

### Synthesis of compound 33

Compound 32-a (675 mg, 1.775 mmol) was dissolved in Eaton's reagent (65 mL). 2,4-dichloro-5-fluorobenzaldehyde (513.84 mg, 2.662 mmol) was added, and the mixture was stirred at 90 °C for 3 h under nitrogen atmosphere. Upon completion, the reaction mixture was added dropwise into ice-cold saturated aqueous sodium bicarbonate solution and diluted with ethyl acetate and water. The organic layer was separated, and the aqueous layer was extracted with ethyl acetate. The combined organic layers were washed with water and saturated aqueous NaCl solution, dried over anhydrous Na₂SO₄, filtered, and concentrated under reduced pressure. Purification by column chromatography (eluent: dichloromethane/methanol = 100/3 to 100/4) afforded compound 33 (210 mg). The crude product was further purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm), and lyophilized to give compound 33 as a solid (68 mg, yield 7%). ¹H NMR (400 MHz, DMSO-*d*₆) δ 10.33 (s, 1H), 8.45 (s, 1H), 7.97 (d, J = 8.4 Hz, 1H), 7.87-7.74 (m, 2H), 7.64 (dd, J = 18.3, 7.8 Hz, 2H), 7.14 (dd, J = 22.5, 9.2 Hz, 2H), 6.36 (s, 1H), 4.43 (s, 3H). LC-MS (ESI): m/z 555.0 [M+H]⁺.

### Example 31 Synthetic route of compound 34

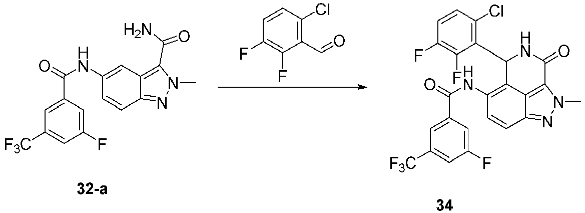

Compound 32-a (849 mg, 2.232 mmol) was dissolved in Eaton's reagent (65 mL), and 2-chloro-5,6-difluorobenzaldehyde (591.21 mg, 3.349 mmol) was added. The mixture was stirred at 90 °C under nitrogen protection for 3 hours. Upon completion, the reaction mixture was poured dropwise into ice-cold saturated aqueous sodium bicarbonate solution, and diluted with ethyl acetate and water. The organic layer was separated, and the aqueous layer was extracted with ethyl acetate. The combined organic layers were washed with water and saturated aqueous NaCl solution, dried over anhydrous Na₂SO₄, filtered, and concentrated under reduced pressure. The residue was purified by column chromatography (eluent: dichloromethane/methanol = 100:3 to 100:4) to afford compound 34 (420 mg). The crude product was further purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm), and lyophilized to give compound 34 as a solid (60 mg). ¹H NMR (400 MHz, DMSO-*d*₆) δ 10.32 (d, J = 35.9 Hz, 1H), 8.56 (d, J = 82.4 Hz, 1H), 7.96 (d, J = 8.4 Hz, 1H), 7.91-7.61 (m, 3H), 7.44-7.04 (m, 3H), 6.65 (d, J = 11.3 Hz, 1H), 4.44 (s, 3H). LC-MS (ESI): m/z 539.1 [M+H]⁺.

### Example 32 Synthetic route of compound 35

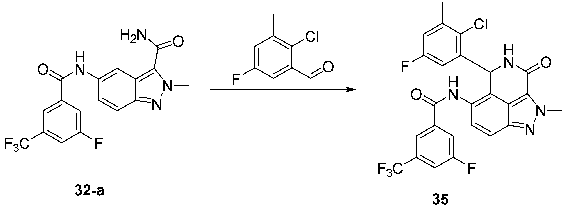

### Synthesis of compound 35

Compound 32-a (775 mg, 2.038 mmol) was dissolved in Eaton's reagent (65 mL), and 2-chloro-5-fluoro-3-methylbenzaldehyde (351.69 mg, 2.038 mmol) was added. The mixture was stirred at 90 °C under nitrogen protection for 3 hours. Upon completion, the reaction mixture was added dropwise into ice-cold saturated aqueous sodium bicarbonate solution and diluted with ethyl acetate and water. The organic layer was separated, and the aqueous layer was extracted with ethyl acetate. The combined organic layers were washed with water and saturated aqueous NaCl solution, dried over anhydrous Na₂SO₄, filtered, and concentrated under reduced pressure. The residue was purified by column chromatography (eluent: dichloromethane/methanol = 100:3 to 100:4) to afford compound 35 (340 mg). The crude product was further purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm), and lyophilized to give solid compound 35 as a solid (70 mg). ¹H NMR (400 MHz, DMSO-*d*₆) δ 10.31 (s, 1H), 8.42 (s, 1H), 8.06-7.87 (m, 1H), 7.86-7.62 (m, 3H), 7.13 (d, J = 22.6 Hz, 2H), 6.56 (d, J = 100.3 Hz, 2H), 4.44 (s, 3H), 2.09 (s, 3H). LC-MS (ESI): m/z 535.2 [M+H]⁺.

### Example 33 Synthetic route of compound 36 (36-p1 and 36-p2)

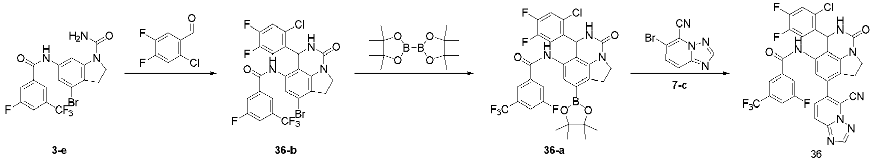

### Synthesis of compound 36-b

Compound 3-e (360 mg, 0.807 mmol) was dissolved in Eaton's reagent (30 mL), and 2-chloro-4,5-difluorobenzaldehyde (213.66 mg, 1.210 mmol) was added. The mixture was stirred at 90 °C for 2.5 hours. After cooling to room temperature, the reaction mixture was slowly added dropwise into ice-water, followed by addition of a large amount of water. The solid product precipitated out and was collected by suction filtration. The filter cake was washed with water and saturated aqueous NaCl solution, then concentrated and lyophilized to afford compound 36-b (510 mg, 0.628 mmol, 77.85%), ESI: (m/z) = 605.8 [M+H]⁺.

### Synthesis of compound 36-a

To a reaction flask were added compound 36-b (500 mg, 0.827 mmol), bis(pinacolato)diboron (629.88 mg, 2.480 mmol), potassium acetate (324.57 mg, 3.307 mmol), and [1,1'-bis(diphenylphosphino)ferrocene]dichloropalladium(II) (121.00 mg, 0.165 mmol). The mixture was dissolved in 1,4-dioxane (30 mL), then heated to 110 °C and stirred for 2.5 hours under nitrogen protection. After cooling to room temperature, the reaction mixture was diluted with ethyl acetate and water. The organic layer was separated, and the aqueous layer was extracted with ethyl acetate. The combined organic layers were washed with saturated aqueous NaCl solution, dried over anhydrous Na₂SO₄, filtered, and concentrated under reduced pressure. Purification by Biotage automatic column chromatography (eluent: petroleum ether/ethyl acetate = 100:4 to 100:5) afforded compound 36-a (660 mg, 0.836 mmol, 101.16%) as a brownish semi-solid. LC-MS (ESI): m/z 652.0 [M+H]⁺.

### Synthesis of compound 36

Compound 36-a (350 mg, 0.537 mmol) and compound 7-c (155.69 mg, 0.698 mmol) were dissolved in dioxane (15 mL) and water (3 mL). Then, [1,1'-bis(diphenylphosphino)ferrocene]dichloropalladium(II) (78.58 mg, 0.107 mmol) and potassium carbonate (222.63 mg, 1.611 mmol) were added. The mixture was stirred at 100 °C for 1.5 hours under nitrogen protection, then cooled to room temperature. The mixture was diluted with water (30 mL) and extracted with ethyl acetate three times. The combined organic layers were dried over anhydrous Na₂SO₄ and filtered. The filtrate was concentrated under reduced pressure. The crude product was purified by preparative chromatography (Welch Xtimate C18, 21.2 * 250 mm, 10 µm; mobile phase: water (10 mM ammonium bicarbonate)/acetonitrile; flow rate: 30 mL/min; column temperature: 25 °C; detection wavelength: 254 nm) to afford target compound 36.

Compound 36 was separated by SFC (instrument: SFC-150 (Waters); chromatographic column: AD-3, 0.46 cm I.D. * 5cm L; 2 µL(injection); mobile phase: CO₂/EtOH = 70/30; flow rate: 2.5 mL/min; back pressure: 100 bar; detection wavelength: 254 nm; cycle time: 13.8 min). After lyophilization, compound 36-p1 (retention time 1.567 min, 26 mg, 0.038 mmol, 7.05%) was obtained as a solid, ¹H NMR (400 MHz, DMSO-*d*₆) δ 10.31 (s, 1H), 8.79 (s, 1H), 8.32 (d, J = 9.3 Hz, 1H), 7.99-7.88 (m, 4H), 7.65-7.58 (m, 2H), 7.19 (dd, J = 10.9, 8.4 Hz, 1H), 7.00 (s, 1H), 6.15 (d, J = 2.3 Hz, 1H), 4.12-3.98 (m, 2H), 3.30-3.16 (m, 2H).

Compound 36-p2 (retention time 2.434 min, 28 mg, 0.041 mmol, 7.70%), ¹H NMR (400 MHz, DMSO-*d*₆) δ 10.30 (s, 1H), 8.79 (s, 1H), 8.32 (d, J = 9.2 Hz, 1H), 7.99-7.88 (m, 4H), 7.65-7.57 (m, 2H), 7.19 (dd, J = 10.9, 8.3 Hz, 1H), 6.99 (s, 1H), 6.15 (d, J = 2.3 Hz, 1H), 4.11-3.98 (m, 2H), 3.24 (qd, J = 10.2, 5.3 Hz, 2H). LC-MS (ESI): m/z 668.3 [M+H]⁺.

### Example 34 Synthetic route of compound 37

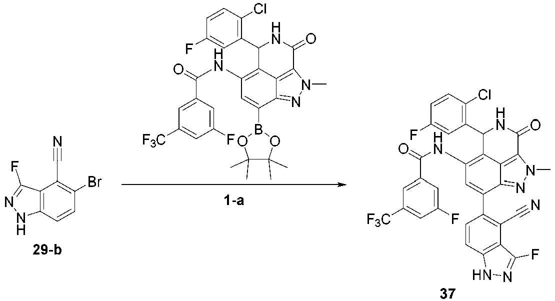

### Synthesis of compound 37

Compound 1-a (160 mg, 0.247 mmol), compound 29-b (59.38 mg, 0.247 mmol), potassium carbonate (102.56 mg, 0.742 mmol) and [1,1'-bis(diphenylphosphino)ferrocene]dichloropalladium(II) (36.20 mg, 0.049 mmol) were added to a reaction flask. 1,4-Dioxane (30 mL) and water (6 mL) were added, and the atmosphere was replaced with nitrogen. The mixture was stirred at 100 °C for 2 hours, then diluted with ethyl acetate and water. The organic layer was separated, and the aqueous layer was extracted with ethyl acetate. The combined organic layers were washed with saturated aqueous NaCl solution, dried over anhydrous Na₂SO₄, filtered, and concentrated under reduced pressure. The crude product was purified by reverse-phase column chromatography (Welch Xtimate C18, 20 g; mobile phase: water (0.05% ammonium bicarbonate)/acetonitrile; flow rate: 20 mL/min; room temperature; detection wavelength: 254 nm). After lyophilization, the target compound 37 (6 mg, 0.009 mmol, 3.48%) was obtained as a solid. ESI: (m/z) = 680.3 [M+H]⁺. ¹H NMR (400 MHz, DMSO-*d*₆) δ 13.37 (s, 1H), 10.53 (s, 1H), 8.68-8.54 (m, 1H), 8.02 (dd, J = 8.9, 2.1 Hz, 1H), 7.95 (dd, J = 14.0, 8.8 Hz, 2H), 7.88-7.77 (m, 2H), 7.43 (s, 1H), 7.35 (dd, J = 8.8, 5.1 Hz, 1H), 7.10 (td, J = 8.4, 3.0 Hz, 1H), 6.99 (dd, J = 9.2, 3.2 Hz, 1H), 6.47 (s, 1H), 4.46 (s, 3H).

### Example 37 Synthetic route of compound 41 (41-p1 and 41-p2)

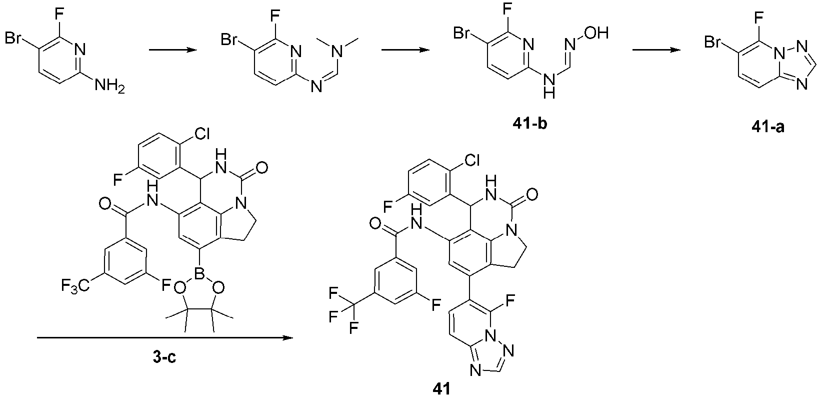

### Synthesis of compound 41-b

DMF-DMA (N,N-dimethylformamide dimethyl acetal) (8.11 g, 68.063 mmol) was added to a solution of 5-bromo-6-fluoropyridin-2-amine (10.00 g, 52.356 mmol) in isopropanol (20 mL). The mixture was heated to 90 °C and stirred for 3 hours. TLC (ethyl acetate/petroleum ether (v/v) = 1:3) showed the formation of the intermediate and complete consumption of the starting material. After cooling to 50 °C, hydroxylamine hydrochloride (4.73 g, 68.063 mmol) was added, and the mixture was stirred at 50 °C for 16 hours. The reaction mixture was concentrated to give the crude product, which was purified by silica gel column chromatography (ethyl acetate/petroleum ether (v/v) = 50% to 90%) to afford compound 41-b (11.00 g, yield 89.80%). ESI: (m/z) = 233.7, 235.8 [M+H]⁺; tR = 1.891 min. ¹H NMR (400 MHz, DMSO-*d*₆) δ 10.38 (s, 1H), 9.88 (d, J = 9.8 Hz, 1H), 8.01 (dd, J = 9.3, 8.5 Hz, 1H), 7.61 (d, J = 9.8 Hz, 1H), 6.94 (dd, J = 8.5, 1.2 Hz, 1H).

### Synthesis of compound 41-a

Compound 41-b (6.00 g, 25.638 mmol) was added to a solution of Eaton's reagent (50 mL). The mixture was heated to 80 °C and stirred for 2 hours. The reaction mixture was diluted with ethyl acetate, then quenched with saturated aqueous sodium bicarbonate solution to neutral under ice-water bath. The organic layer was separated and concentrated, then purified by silica gel column chromatography (ethyl acetate/petroleum ether (v/v) = 0 to 30%) to afford compound 41-a (3.00 g, yield 54.15%). ESI: (m/z) = 216.0, 218.0 [M+H]⁺; tR = 1.605 min. ¹H NMR (400 MHz, DMSO-*d*₆) δ 8.64 (s, 1H), 7.99 (dd, J = 9.4, 7.2 Hz, 1H), 7.76 (d, J = 9.4 Hz, 1H).

### Synthesis of compound 41

Under nitrogen protection, Pd(dppf)Cl₂ (69 mg, 0.095 mmol) was added to a mixture of compound 41-a (23 mg, 0.118 mmol), compound 3-c (300 mg, 0.473 mmol) and potassium carbonate (131 mg, 0.947 mmol) in 1,4-dioxane (15 mL) and water (3 mL). The mixture was heated to 90 °C and stirred for 2 hours. The reaction was quenched with water, extracted with ethyl acetate, and the organic layer was separated. The organic layer was quickly filtered through a celite pad, and the filtrate was concentrated. The residue was purified by preparative HPLC (basic conditions) to afford compound 41 (40 mg, yield 13.14%). (ESI): m/z 643.1 [M+H]⁺; tR = 4.664 min. ¹H NMR (400 MHz, DMSO-*d*₆) δ 10.28 (s, 1H), 8.68 (s, 1H), 7.96 (d, J = 8.4 Hz, 1H), 7.90 (d, J = 6.5 Hz, 2H), 7.89-7.84 (m, 2H), 7.61 (d, J = 2.6 Hz, 1H), 7.36 (dd, J = 8.9, 5.1 Hz, 1H), 7.13 (td, J = 8.4, 3.0 Hz, 1H), 7.01-6.95 (m, 1H), 6.94 (s, 1H), 6.24-6.12 (m, 1H), 4.11-4.04 (m, 1H), 4.04-3.95 (m, 1H), 3.25 (t, J = 8.8 Hz, 2H).

A solid sample of compound 41 (105 mg, 0.163 mmol) was subjected to chiral SFC separation (instrument: SFC-150 (Waters); chromatographic column: AD-3, 0.46 cm I.D. *5 cm L; 2 µL(injection); mobile phase: CO₂/EtOH = 70/30; flow rate: 2.5 mL/min; back pressure: 100 bar; detection wavelength: 254 nm; cycle time: 13.8 min) to afford compound 41-p1 (retention time: 1.486 min, 36 mg, yield 68.57%) and compound 41-p2 (retention time: 3.136 min, 34 mg, yield 64.76%). (ESI):m/z 643.0 [M+H]⁺; tR=2.122min. 1: ee=99.92%, 2: ee=100%.

Compound 41-p1: (ESI): m/z 643.1 [M+H]⁺; tR=4.664 min. ¹H NMR (400 MHz, DMSO-*d*₆) δ 10.28 (s, 1H), 8.68 (s, 1H), 7.96 (d, J = 8.4 Hz, 1H), 7.90 (d, J = 6.5 Hz, 2H), 7.89-7.84 (m, 2H), 7.61 (d, J = 2.6 Hz, 1H), 7.36 (dd, J = 8.9, 5.1 Hz, 1H), 7.13 (td, J = 8.4, 3.0 Hz, 1H), 7.01-6.95 (m, 1H), 6.94 (s, 1H), 6.24-6.12 (m, 1H), 4.11-4.04 (m, 1H), 4.04-3.95 (m, 1H), 3.25 (t, J = 8.8 Hz, 2H).

Compound 41-p2: (ESI): m/z 643.1 [M+H]⁺; tR=4.664 min. ¹H NMR (400 MHz, DMSO-*d*₆) δ 10.28 (s, 1H), 8.68 (s, 1H), 7.96 (d, J = 8.4 Hz, 1H), 7.90 (d, J = 6.5 Hz, 2H), 7.89-7.84 (m, 2H), 7.61 (d, J = 2.6 Hz, 1H), 7.36 (dd, J = 8.9, 5.1 Hz, 1H), 7.13 (td, J = 8.4, 3.0 Hz, 1H), 7.01-6.95 (m, 1H), 6.94 (s, 1H), 6.24-6.12 (m, 1H), 4.11-4.04 (m, 1H), 4.04-3.95 (m, 1H), 3.25 (t, J = 8.8 Hz, 2H).

### Example 39 Synthetic route of compound 43

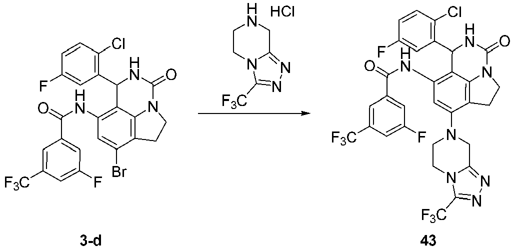

### Synthesis of compound 43

Under nitrogen protection, Pd₂(dba)₃ (31 mg, 0.034 mmol) was added to a mixture of compound 3-d (200 mg, 0.341 mmol), 3-(trifluoromethyl)-5,6,7,8-tetrahydro-[1,2,4]triazolo[4,3-a]pyrazine hydrochloride (117 mg, 0.511 mmol), cesium carbonate (389 mg, 1.193 mmol) and RuPhos (2-dicyclohexylphosphino-2',6'-diisopropoxy-1,1'-biphenyl) (32 mg, 0.068 mmol) in 1,4-dioxane (8 mL). The mixture was heated to 110 °C and stirred for 6 hours. The reaction was quenched with water, extracted with ethyl acetate, and the organic layer was separated. After concentration, the organic layer was purified by silica gel column chromatography (methanol/dichloromethane (v/v) = 0 to 10%) to give the crude product, which was further purified by preparative HPLC (basic conditions) to afford compound 43 (55 mg, yield 23.11 %). (ESI): m/z 698.2 [M+H]⁺; ¹H NMR (400 MHz, DMSO-*d*₆) δ 10.15 (s, 1H), 7.95 (d, J = 8.4 Hz, 1H), 7.88 (s, 1H), 7.86 (d, J = 8.6 Hz, 1H), 7.48 (d, J = 2.6 Hz, 1H), 7.32 (dd, J = 8.8, 5.1 Hz, 1H), 7.09 (td, J = 8.3, 3.1 Hz, 1H), 6.84 (dd, J = 9.2, 3.1 Hz, 1H), 6.45 (s, 1H), 6.09-6.00 (m, 1H), 4.64-4.44 (m, 2H), 4.28 (t, J = 5.3 Hz, 2H), 4.00 (dp, J = 19.4, 9.9, 9.0 Hz, 2H), 3.64 (t, J = 5.4 Hz, 2H), 3.26 (t, J = 8.5 Hz, 2H).

### Example 40 Synthetic route of compound 44

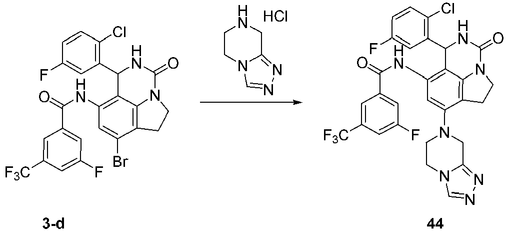

### Synthesis of compound 44

Under nitrogen protection, Pd₂(dba)₃ (31 mg, 0.034 mmol) was added to a mixture of compound 3-d (200 mg, 0.341 mmol), 5,6,7,8-tetrahydro-[1,2,4]triazolo[4,3-a]pyrazine hydrochloride (82 mg, 0.511 mmol), cesium carbonate (389 mg, 1.193 mmol) and RuPhos (32 mg, 0.068 mmol) in 1,4-dioxane (8 mL). The mixture was heated to 110 °C and stirred for 6 hours. The reaction was quenched with water, extracted with ethyl acetate, and the organic layer was separated. After concentration, the organic layer was purified by silica gel column chromatography (methanol/dichloromethane (v/v) = 0 to 10%) to afford the crude product, which was further purified by preparative HPLC (basic conditions) to give compound 44 (35 mg, yield 16.30%). (ESI): m/z 630.2 [M+H]⁺; ¹H NMR (400 MHz, DMSO-*d*₆) δ 10.15 (s, 1H), 8.51 (s, 1H), 7.95 (d, J = 8.4 Hz, 1H), 7.91-7.79 (m, 2H), 7.46 (s, 1H), 7.19-7.02 (m, 1H), 7.09 (dd, J = 10.8, 5.3 Hz, 1H), 6.97-6.78 (m, 1H), 6.41 (s, 1H), 6.02 (s, 1H), 4.53-4.35 (m, 2H), 4.15 (t, J = 5.4 Hz, 2H), 3.99 (q, J = 8.8 Hz, 2H), 3.59-3.56 (m, 2H), 3.28-3.23 (m, 2H).

### Example 41 Synthetic route of compound 45

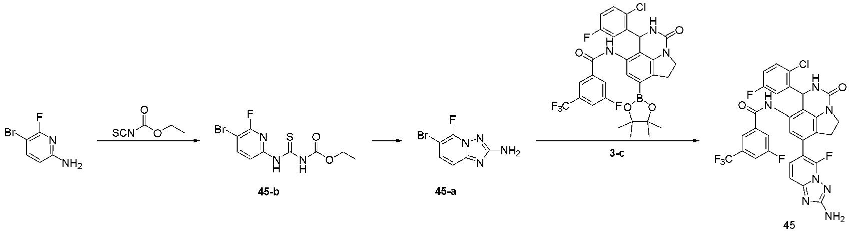

### Synthesis of compound 45-b

Ethyl isothiocyanatoformate (377.66 mg, 2.880 mmol) was added to a solution of 5-bromo-6-fluoropyridin-2-amine (500 mg, 2.618 mmol) in 1,4-dioxane (20 mL), and the mixture was stirred at room temperature for 16 hours. The reaction mixture was concentrated to afford compound 45-b (900 mg, crude). (ESI): m/z 322.0 [M(⁷⁹Br)-FH]⁺, 324.0 [M(⁸¹Br)+H]⁺.

### Synthesis of compound 45-a

Hydroxylamine hydrochloride (970.78 mg, 13.970 mmol) was added to a mixture of compound 45-b (900 mg, 2.794 mmol) and N,N-diisopropylethylamine (1083 mg, 8.382 mmol) in methanol (5 mL) and ethanol (10 mL). The mixture was stirred at room temperature for 1 hour, then heated to 70 °C and stirred for 17 hours. The reaction mixture was diluted with water and extracted with ethyl acetate. The organic layer was dried over anhydrous sodium sulfate, filtered, and concentrated. The residue was purified by silica gel column chromatography (methanol/dichloromethane (v/v) = 1:20) to afford compound 45-a (100 mg, yield 15.49%). (ESI): m/z 231.0 [M(⁷⁹Br)+H]⁺, 233.0 [M(⁸¹Br)+H]⁺; ¹H NMR (400 MHz, DMSO-*d*₆) δ 7.71 (dd, J = 9.3, 7.5 Hz, 1H), 7.23 (d, J = 9.3 Hz, 1H), 6.39 (s, 2H).

### Synthesis of compound 45

Under nitrogen protection, Pd(dppf)Cl₂ (17 mg, 0.024 mmol) was added to a mixture of compound 3-c (150 mg, 0.237 mmol), compound 45-a (60 mg, 0.260 mmol), and potassium carbonate (65 mg, 0.473 mmol) in water (2 mL) and 1,4-dioxane (10 mL). The mixture was heated to 80 °C and stirred for 1 hour. The reaction was quenched with water, extracted with ethyl acetate, and the organic layer was separated. After concentration, the organic layer was purified by silica gel column chromatography (methanol/dichloromethane (v/v) = 1:20) to give an off-white solid. The off-white solid was further purified by C18 chromatography (basic conditions) to afford the target compound 45 (65 mg, yield 41.74%). (ESI): m/z 658.0 [M+H]⁺; ¹H NMR (400 MHz, DMSO-*d*₆) δ 10.27 (s, 1H), 7.96 (d, J = 8.5 Hz, 1H), 7.91 (s, 1H), 7.88 (d, J = 8.9 Hz, 1H), 7.63 (d, J = 8.7 Hz, 1H), 7.61-7.54 (m, 1H), 7.35 (td, J = 8.9, 5.6 Hz, 2H), 7.13 (td, J = 8.4, 3.0 Hz, 1H), 6.95 (dd, J = 9.3, 3.1 Hz, 1H), 6.87 (s, 1H), 6.38 (s, 2H), 6.16 (d, J = 2.5 Hz, 1H), 4.14-3.93 (m, 2H), 3.27-3.11 (m, 2H).

### Example 42 Synthetic route of compound 45-p1 and 45-p2

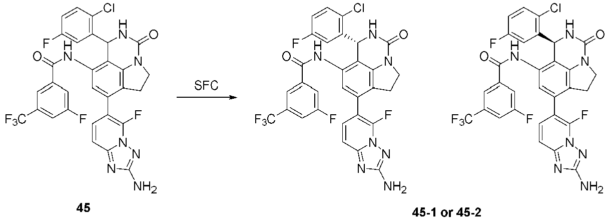

Compound 45 (60 mg, 0.091 mmol) was subjected to chiral SFC separation (conditions: instrument: SFC-150 (Waters); chromatographic column: AS,20*250 mm, 10 µm (Daicel); mobile phase: CO₂/MeOH[0.2%NH₃(7 M in MeOH)] = 45/55; flow rate: 120 g/min; back pressure: 100 bar; detection wavelength: 214 nm; cycle time: 13.8 min) to afford compound 45-p1 (14.7 mg, yield 24.50%) and 45-p2 (17.3 mg, yield 28.83%).

### Example 43 Synthetic route of compound 47

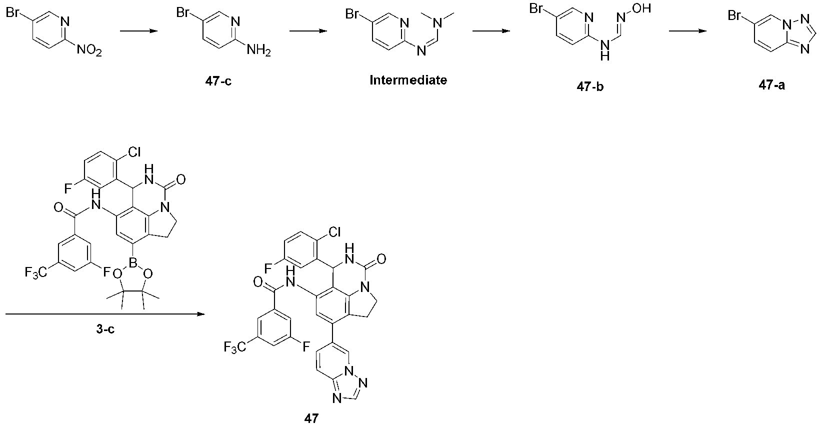

### Synthesis of compound 47-c

Under nitrogen protection, iron powder (13.75 g, 246.305 mmol) was added to a mixture of 5-bromo-2-nitropyridine (10.00 g, 49.261 mmol) and ammonium chloride (7.90 g, 147.783 mmol) in ethanol (100 mL) and water (20 mL). The mixture was heated to 70 °C and stirred for 10 hours. The reaction mixture was diluted with dichloromethane, filtered through a celite pad, and the filtrate was concentrated to remove organic solvents. Then it was diluted with ethyl acetate and water, and the organic layer was separated. The organic layer was dried over anhydrous sodium sulfate and filtered again. The filtrate was concentrated and purified by silica gel column chromatography (ethyl acetate/petroleum ether (v/v) = 30% to 90%) to afford compound 47-c (6.50 g, yield 76.29%). (ESI): m/z 173.0, 175.0 [M+H]⁺.

### Synthesis of compound 47-b

DMF-DMA (0.90 g, 7.514 mmol) was added to a solution of compound 47-c (1.00 g, 5.780 mmol) in isopropanol (2 mL). The mixture was heated to 90 °C and stirred for 3 hours. LCMS analysis indicated the formation of the intermediate (LC-MS (ESI): m/z 227.8, 229.8 [M+H]⁺, tR = 1.737 min). After cooling to 50 °C, hydroxylamine hydrochloride (0.52 g, 7.514 mmol) was added, and the mixture was stirred at 50 °C for 16 hours. The reaction mixture was concentrated to give the crude product, which was purified by silica gel column chromatography (ethyl acetate/petroleum ether (v/v) = 50% to 90%) to afford compound 47-b (1.00 g, yield 80.00%). (ESI): m/z 215.8, 217.8 [M+H]⁺, tR = 1.270 min. ¹H NMR (400 MHz, DMSO-*d*₆) δ 10.19 (s, 1H), 9.56 (d, J = 9.9 Hz, 1H), 8.23 (dd, J = 2.5, 0.6 Hz, 1H), 7.80 (dd, J = 8.8, 2.5 Hz, 1H), 7.77 (d, J = 9.9 Hz, 1H), 7.05 (dd, J = 8.9, 0.7 Hz, 1H).

### Synthesis of compound 47-a

Trifluoroacetic anhydride (1.26 g, 6.017 mmol) was added dropwise to a solution of compound 47-b (1.00 g, 4.629 mmol) in tetrahydrofuran (20 mL). The mixture was heated to 80 °C and stirred for 4 hours. The reaction was quenched with saturated aqueous sodium bicarbonate solution to neutral, extracted with ethyl acetate, and the organic layer was separated. The organic layer was dried over anhydrous sodium sulfate and filtered. The filtrate was concentrated and purified by silica gel column chromatography (ethyl acetate/petroleum ether (v/v) = 0 to 50%) to afford compound 47-a (700 mg, yield 76.09%). (ESI): m/z 197.8, 199.8 [M+H]⁺, tR = 1.603 min.

### Synthesis of compound 47

Under nitrogen protection, Pd(dppf)Cl₂ (69 mg, 0.095 mmol) was added to a mixture of compound 47-a (141 mg, 0.710 mmol), compound 3-c (300 mg, 0.473 mmol) and potassium carbonate (196 mg, 1.420 mmol) in 1,4-dioxane (18 mL) and water (3 mL). The mixture was heated to 90 °C and stirred for 2 hours. The reaction was quenched with water, extracted with ethyl acetate, and the organic layer was separated. The organic layer was quickly filtered through a celite pad, and the filtrate was concentrated. The residue was purified by preparative HPLC (basic conditions) to afford compound 47 (70 mg, yield 23.66%). (ESI): m/z 625.0 [M+H]⁺, tR = 1.603 min. ¹H NMR (400 MHz, DMSO-*d*₆) δ 10.27 (s, 1H), 9.17 (s, 1H), 8.57 (s, 1H), 7.98 (s, 1H), 7.95 (d, J = 3.6 Hz, 2H), 7.92-7.83 (m, 2H), 7.62 (d, J = 2.7 Hz, 1H), 7.36 (dd, J = 8.9, 5.1 Hz, 1H), 7.12 (td, J = 8.4, 3.1 Hz, 1H), 7.03 (s, 1H), 6.95 (dd, J = 9.2, 3.1 Hz, 1H), 6.19 (d, J = 2.6 Hz, 1H), 4.06 (dd, J = 10.3, 5.7 Hz, 1H), 3.99 (q, J = 9.8 Hz, 1H), 3.51 (dt, J = 17.2, 9.0 Hz, 1H).

### Example 44 Synthetic route of compound 48 (48-p1 and 48-p2)

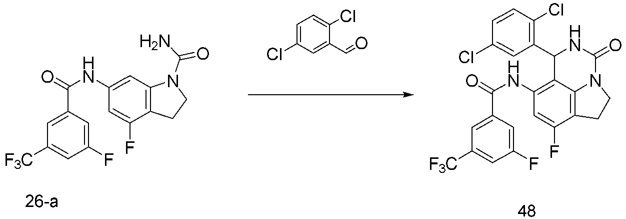

To a 50 mL three-necked flask were added compound 26-a (200 mg, 0.519 mmol), Eaton's reagent (10 mL) and 2,5-dichlorobenzaldehyde (136 mg, 0.779 mmol) successively. The mixture was heated to 80 °C and stirred for 2 hours under nitrogen protection. After cooling to room temperature, the reaction mixture was poured into ice water, and saturated aqueous sodium bicarbonate solution was added dropwise to adjust the pH to 7-8. The mixture was extracted with ethyl acetate, and the layers were separated. The organic layer was washed with brine, dried over anhydrous sodium sulfate, filtered, and concentrated under reduced pressure. The crude product was purified by preparative chromatography to afford compound 48 (151 mg, 0.278 mmol, 53.64%). LC-MS (ESI): m/z 542.2 [M+H]⁺.

¹H NMR (400 MHz, Chloroform-d) δ 7.50 (m, J = 8.1, 2.3 Hz, 2H), 7.39 (s, 1H), 7.34 (d, J = 8.6 Hz, 1H), 7.28 (d, J = 2.4 Hz, 1H), 7.25-7.18 (m, 2H), 7.05 (d, J = 2.4 Hz, 1H), 6.25-6.19 (m, 1H), 5.19 (d, J = 2.4 Hz, 1H), 4.16 (tt, J = 6.9, 3.1 Hz, 2H), 3.32 (dd, J = 9.8, 7.4 Hz, 2H).

The product was separated by chiral SFC (conditions: instrument: SFC-150 (Waters); chromatographic column: AD-3; mobile phase: CO₂/MeOH[0.2%NH₃(7 M in MeOH)] = 30/70; flow rate: 3.0 mL/min; detection wavelength: 214 nm) to afford compound 48-p1 and 48-p2; compound 48-p1 46 mg, retention time: 1.305 min, compound 48-p2 40 mg (RT = 1.712 min).

### Example 45 Synthetic route of compound 49 (49-p1 and 49-p2)

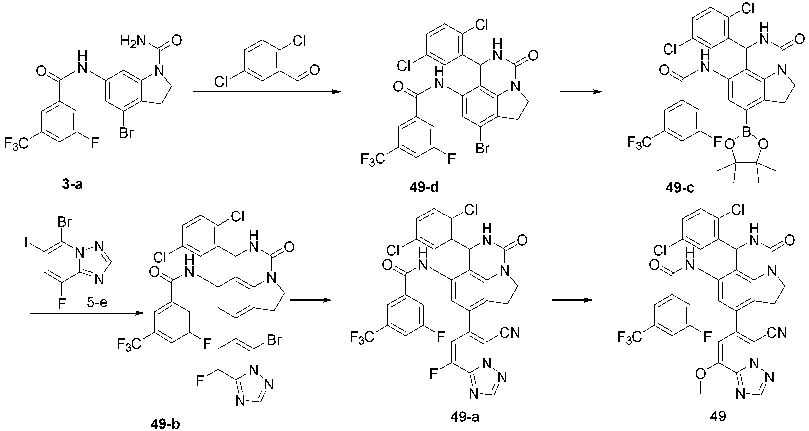

### Synthesis of compound 49-d

Compound 3-a (8 g, 17.929 mmol) was dissolved in Eaton's reagent (80 mL), and 2,5-dichlorobenzaldehyde (3.77 g, 21.515 mmol) was added. The mixture was stirred at 80 °C for 2 hours. The reaction mixture was poured into ice water, adjusted to pH ~7 with 1 N aqueous sodium hydroxide solution, and extracted with ethyl acetate. The aqueous phase was extracted with ethyl acetate three times. The combined organic phases were washed with water and saturated aqueous sodium chloride solution, dried over anhydrous sodium sulfate, filtered, and evaporated to dryness. Purification by column chromatography (dichloromethane/methanol ~20/1) afforded compound 49-d (2.2 g, 20.35%). ESI: (m/z) = 604.1 [M+H]⁺.

### Synthesis of compound 49-c

Compound 49-d (2.2 g, 3.647 mmol) was dissolved in 1,4-dioxane (25 mL). Bis(pinacolato)diboron (1.39 g, 5.471 mmol), potassium acetate (1.07 g, 10.942 mmol), and Pd(dppf)Cl₂ (530 mg, 0.729 mmol) were added, and the mixture was stirred at 100 °C for 1 hour to afford compound 49-c (2.37 g), which was used directly in the next step.

### Synthesis of compound 49-b

Compound 49-c (2.37 g, 3.739 mmol), compound 5-e (1.28 g, 3.739 mmol), potassium carbonate (1.55 g, 11.218 mmol), and Pd(dppf)Cl₂ (0.27 g, 0.374 mmol) were added to a reaction flask. 1,4-Dioxane (25 mL) and water (5 mL) were added, and the atmosphere was replaced with nitrogen. The mixture was stirred at 100 °C for 1 hour. The mixture was extracted with ethyl acetate and water three times. The combined organic layers were dried over anhydrous sodium sulfate and concentrated under reduced pressure. Purification by normal-phase column chromatography (dichloromethane/methanol = 20/1) afforded compound 49-b (1.8 g, 66.6%). LC-MS (ESI): m/z 739.2 [M+H]⁺;

### Synthesis of compound 49-a

Compound 49-b (1.7 g, 2.303 mmol), zinc cyanide (0.41 g, 3.454 mmol), zinc powder (0.02 g, 0.230 mmol), Xantphos (0.53 g, 0.921 mmol), and palladium acetate (0.10 g, 0.461 mmol) were added to a reaction flask. DMAC (20 mL) was added, and the atmosphere was replaced with nitrogen. The mixture was stirred at 120 °C for 1 hour. The mixture was extracted with ethyl acetate and water. The organic phase was concentrated under reduced pressure, and purified by normal-phase column chromatography (dichloromethane/methanol = 20/1) to afford compound 49-a (1 g, 63.3%). LC-MS (ESI): m/z 684.3 [M+H]⁺;

### Synthesis of compound 49 (49-p1 and 49-p2)

Compound 49-a (200 mg, 0.292 mmol) was added to a reaction flask, followed by methanol (5 mL) and sodium methoxide (32 mg, 0.584 mmol). The atmosphere was replaced with nitrogen, and the mixture was stirred at room temperature overnight. The reaction mixture was concentrated under reduced pressure and purified by normal-phase column chromatography (dichloromethane/methanol = 20/1) to afford compound 49. LC-MS (ESI): m/z 696.3 [M+H]⁺; the crude product was separated by SFC to give compound 49-p1 (42 mg, 21%) and compound 49-p2 (43 mg, 21.1%).

SFC conditions: instrument: SFC-150 (Waters); chromatographic column: OD-3 4.6*100 mm 3 µm; mobile phase: MeOH[0.2%NH₃(7 M in MeOH)]; flow rate: 3.0 mL/min; back pressure: 2000 psi; detection wavelength: 214 nm; cycle time: 2.0 min.

Compound 49-p1: Retention time: 0.938 min; ¹H NMR (400 MHz, DMSO-*d*₆) δ 10.34 (s, 1H), 8.69 (s, 1H), 7.96 (d, J = 8.4 Hz, 1H), 7.89 (d, J = 16.2 Hz, 2H), 7.57 (d, J = 2.4 Hz, 1H), 7.38 (d, J = 8.6 Hz, 1H), 7.34-7.28 (m, 2H), 7.11 (d, J = 2.5 Hz, 1H), 6.95 (s, 1H), 6.16 (d, J = 2.4 Hz, 1H), 4.14 (s, 3H), 4.06 (dq, J = 18.9, 7.4, 5.8 Hz, 2H), 3.42-3.35 (m, 1H), 3.28-3.17 (m, 1H).

Compound 49-p2: Retention time: 1.306 min, ¹H NMR (400 MHz, DMSO-*d*₆) δ 10.34 (s, 1H), 8.69 (s, 1H), 7.96 (d, J = 8.5 Hz, 1H), 7.93-7.85 (m, 2H), 7.57 (d, J = 2.4 Hz, 1H), 7.38 (d, J = 8.5 Hz, 1H), 7.34-7.29 (m, 2H), 7.11 (d, J = 2.5 Hz, 1H), 6.95 (s, 1H), 6.16 (d, J = 2.4 Hz, 1H), 4.14 (s, 3H), 4.10-3.99 (m, 2H), 3.37 (d, J = 10.0 Hz, 1H), 3.22 (ddd, J = 16.6, 10.2, 6.3 Hz, 1H).

### Example 46 Synthetic route of compound 50 (50-p1 and 50-p2)

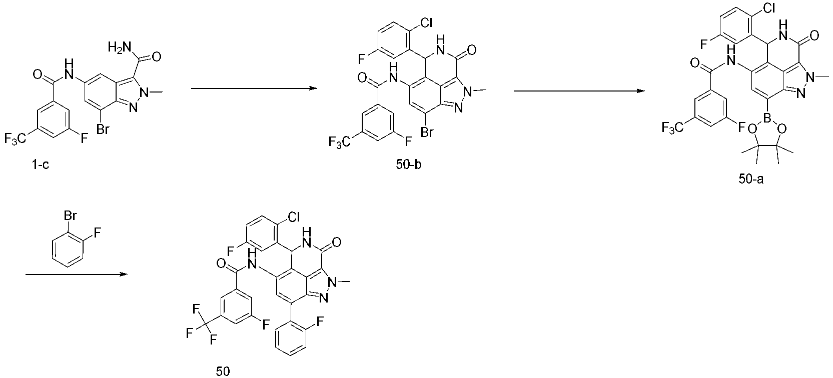

### Synthesis of compound 50-b

To a 100 mL three-necked flask were added compound 1-c (3.5 g, 7.622 mmol), Eaton's reagent (70 mL) and 2-chloro-5-fluorobenzaldehyde (2.18 g, 13.720 mmol) successively. The mixture was heated to 70 °C and stirred for 2 hours under nitrogen protection. The reaction mixture was added dropwise to water at 0 °C to quench, extracted with ethyl acetate, and the layers were separated. The organic layer was concentrated under reduced pressure, dissolved in dichloromethane, and adjusted to strongly basic with 1 M aqueous sodium hydroxide solution, then stirred for 30 min. The layers were separated, and the aqueous layer was extracted with dichloromethane. The combined dichloromethane layers were dried over anhydrous sodium sulfate, filtered, concentrated under reduced pressure, and purified by column chromatography (methanol/dichloromethane = 0 to 2%) to afford compound 50-b (955 mg, 1.592 mmol, 20.89%). LC-MS (ESI): m/z 599.1, 601.1 [M+H]⁺.

### Synthesis of compound 50-a

To a 50 mL three-necked flask were added compound 50-b (300 mg, 0.487 mmol), potassium acetate (147 mg, 1.501 mmol), bis(pinacolato)diboron (190 mg, 0.750 mmol), [1,1'-bis(diphenylphosphino)ferrocene]dichloropalladium(II) (36 mg, 0.050 mmol) and 1,4-dioxane (8 mL) successively. The mixture was heated to 100 °C and stirred for 1 hour under nitrogen protection. LC-MS monitoring showed 90% formation of the target compound 50-a, which was used directly in the next step. LC-MS (ESI): m/z 565.1 [M+H]⁺.

### Synthesis of compound 50 (50-p1 and 50-p2)

To a 50 mL three-necked flask were added compound 50-a (282 mg, 0.487 mmol), 1-bromo-2-fluorobenzene (131 mg, 0.749 mmol), potassium carbonate (207 mg, 1.498 mmol), [1,1'-bis(diphenylphosphino)ferrocene]dichloropalladium(II) (36 mg, 0.050 mmol), 1,4-dioxane (8 mL) and water (1 mL) successively. The mixture was heated to 100 °C and stirred for 1 hour under nitrogen protection. The reaction mixture was diluted with water and ethyl acetate, filtered, and the filter cake was washed with ethyl acetate. The filtrate was separated, and the organic phase was dried over anhydrous sodium sulfate, filtered and concentrated under reduced pressure. Purification by column chromatography (methanol/dichloromethane = 0 to 3%) followed by reversed-phase column chromatography (acetonitrile/water = 5 to 65%) afforded compound 50 (145 mg, 0.236 mmol, 47.22%). LC-MS (ESI): m/z 615.2 [M+H]⁺

Compound 50 was separated by SFC (conditions: instrument: SFC-150 (Waters); chromatographic column: AD-3; mobile phase: CO₂/EtOH = 70/30; flow rate: 2.5 mL/min; detection wavelength: 254 nm) to give compound 50-p1 53 mg (retention time 2.002 min) and compound 50-p2 58 mg (retention time 3.317 min) ¹H NMR (400 MHz, DMSO-*d*₆) δ 10.45 (s, 1H), 8.56 (d, J = 2.2 Hz, 1H), 7.92-8.03 (m, 2H), 7.84 (dt, J = 9.3, 2.0 Hz, 1H), 7.78 (s, 1H), 7.45-7.56 (m, 1H), 7.30-7.43 (m, 4H), 7.09 (m, J = 8.4, 3.1 Hz, 1H), 6.95 (dd, J = 9.2, 3.1 Hz, 1H), 6.44 (d, J = 2.1 Hz, 1H), 4.47 (s, 3H).

### Example 47 Synthetic route of compound 51 (51-p1 and 51-p2)

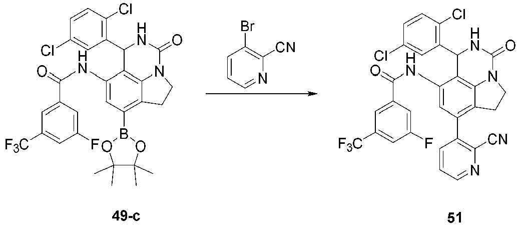

Compound 49-c (320 mg, 0.492 mmol), 3-bromo-2-cyanopyridine (135 mg, 0.738 mmol), potassium carbonate (204 mg, 1.476 mmol) and Pd(dppf)Cl₂ (36 mg, 0.049 mmol) were added to a reaction flask. 1,4-Dioxane (10 mL) and water (2 mL) were added, and the atmosphere was replaced with nitrogen. The mixture was stirred at 100 °C for 1 hour, then extracted with ethyl acetate and water, and concentrated under reduced pressure. The crude product was purified by normal-phase chromatography and separated by SFC, separation conditions: instrument: SFC-150 (Waters); chromatographic column: AD-3; mobile phase: CO₂/MeOH[0.2%NH₃(7 M in MeOH)] = 70/30; flow rate: 3.0 mL/min; detection wavelength: 214 nm) to afford compound 51-p1 (73 mg) and compound 51-p2 (76.5 mg). LC-MS (ESI): m/z 626.3 [M+H]⁺;
SFC conditions: instrument: SFC-150 (Waters); chromatographic column: AD-3; mobile phase: CO₂/EtOH = 70/30; flow rate: 2.5 mL/min; back pressure: 100 bar; detection wavelength: 254 nm; cycle time: 5 min. Compound 51-p1: retention time is 2.387 min; compound 51-p2: retention time: is 4.086 min
Compound 51-p1: ¹H NMR (400 MHz, DMSO-*d*₆) δ 10.30 (s, 1H), 8.81 (dd, J = 4.7, 1.5 Hz, 1H), 8.16 (dd, J = 8.1, 1.6 Hz, 1H), 7.96 (d, J = 8.1 Hz, 1H), 7.80-7.92 (m, 3H), 7.55 (d, J = 2.4 Hz, 1H), 7.37 (d, J = 8.6 Hz, 1H), 7.30 (dd, J = 8.6, 2.5 Hz, 1H), 7.10 (d, J = 2.5 Hz, 1H), 6.85 (s, 1H), 6.14 (d, J = 2.4 Hz, 1H), 4.03 (pd, J = 10.4, 6.7 Hz, 2H), 3.04-3.29 (m, 2H).
Compound 51-p2: ¹H NMR (400 MHz, DMSO-*d*₆) δ 10.30 (s, 1H), 8.81 (dd, J = 4.7, 1.5 Hz, 1H), 8.16 (dd, J = 8.1, 1.6 Hz, 1H), 7.96 (d, J = 8.4 Hz, 1H), 7.81-7.92 (m, 3H), 7.55 (d, J = 2.4 Hz, 1H), 7.37 (d, J = 8.6 Hz, 1H), 7.30 (dd, J = 8.6, 2.5 Hz, 1H), 7.09 (d, J = 2.5 Hz, 1H), 6.85 (s, 1H), 6.14 (d, J = 2.4 Hz, 1H), 3.94-4.13 (m, 2H), 3.02-3.29 (m, 2H).

### Example 48 Synthetic route of compound 52 (52-p1 and 52-p2)

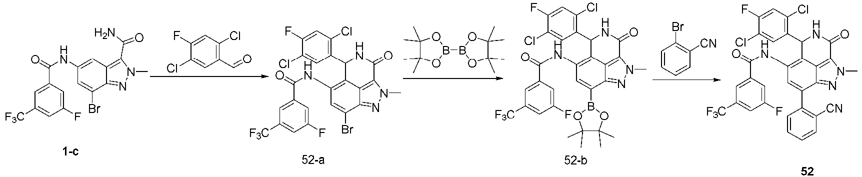

### Synthesis of compound 52-a

Compound 1-c (2 g, 4.355 mmol) was dissolved in Eaton's reagent (20 mL), and 2,5-dichloro-4-fluorobenzaldehyde (1.01 g, 5.226 mmol) was added. The mixture was stirred at 80 °C for 2 hours. The reaction mixture was poured into ice water, adjusted to pH ~7 with saturated aqueous sodium bicarbonate solution, and extracted with ethyl acetate. The aqueous phase was extracted with ethyl acetate three times. The combined organic phases were washed with water and saturated aqueous sodium chloride solution, dried over anhydrous Na₂SO₄, filtered, and evaporated to dryness. Purification by column chromatography (dichloromethane/methanol ~20/1) afforded compound 52-a (360 mg, 13%). ESI: (m/z) = 635.0 [M+H]⁺.

### Synthesis of compound 52-b

Compound 52-a (360 mg, 0.568 mmol) was dissolved in 1,4-dioxane (10 mL). Bis(pinacolato)diboron (216 mg, 0.851 mmol), potassium acetate (167 mg, 1.703 mmol), and Pd(dppf)Cl₂ (83 mg, 0.114 mmol) were added, and the mixture was stirred at 100 °C for 1 hour to afford compound 52-b (390 mg), which was used directly in the next step.

### Synthesis of compound 52 (52-p1 and 52-p2)

Compound 52-b (390 mg, 0.572 mmol), 2-bromobenzonitrile (156 mg, 0.859 mmol), potassium carbonate (237 mg, 1.717 mmol) and Pd(dppf)Cl₂ (42 mg, 0.057 mmol) were added to a reaction flask. 1,4-Dioxane (10 mL) and water (2 mL) were added, and the atmosphere was replaced with nitrogen. The mixture was stirred at 100 °C for 1 hour, extracted with ethyl acetate and water, and concentrated under reduced pressure. The crude product was purified by normal-phase chromatography and separated by SFC (conditions: instrument: SFC-150 (Waters); chromatographic column: AD-3; mobile phase: CO₂/MeOH[0.2%NH₃(7 M in MeOH)] = 70/30; flow rate: 3.0 mL/min; back pressure: 2000 psi; detection wavelength: 214 nm) to afford compound 52-p1 (64 mg) and compound 52-p2 (52 mg). LC-MS (ESI): m/z 656.2 [M+H]⁺;
SFC conditions: instrument: SFC-150 (Waters); chromatographic column: AD-3; mobile phase: CO₂/EtOH = 70/30; flow rate: 2.5 mL/min; back pressure: 100 bar; detection wavelength: 254 nm; cycle time: 5 min; Compound 52-p1: retention time is 2.223 min; compound 52-p2: retention time is 3.679 min
Compound 52-p1: ¹H NMR (400 MHz, DMSO-*d*₆) δ 10.50 (s, 1H), 8.51 (s, 1H), 8.03 (d, J = 7.8 Hz, 1H), 7.97 (d, J = 8.4 Hz, 1H), 7.92-7.84 (m, 3H), 7.75 (s, 1H), 7.66 (ddd, J = 8.6, 6.7, 2.2 Hz, 1H), 7.60 (d, J = 9.0 Hz, 1H), 6.40 (s, 1H), 4.45 (s, 3H).
Compound 52-p2: ¹H NMR (400 MHz, DMSO-*d*₆) δ 10.50 (s, 1H), 8.51 (s, 1H), 8.02 (d, J = 7.7 Hz, 1H), 7.97 (d, J = 8.5 Hz, 1H), 7.87 (m, 3H), 7.75 (s, 1H), 7.66 (ddd, J = 8.4, 6.6, 2.1 Hz, 1H), 7.60 (d, J = 9.0 Hz, 1H), 7.33 (t, J = 4.0 Hz, 2H), 6.40 (s, 1H), 4.45 (s, 3H).

### Effect Example IC50 of PI3Kα and PI3KαH1047R enzyme activity inhibition evaluation assay

The ADP-Glo Kinase Assay Kit was used for the experiments. The following buffer solutions were prepared: 50 mM HEPES, pH 7.5, 3 mM MgCl2, 1 mM EGTA, 100 mM NaCl, 0.03% CHAPS, 2 mM DTT. The tested compound samples were dissolved in DMSO and added to the screening system at a certain starting concentration of, for example, 10 µM, diluted 3-fold, while setting a DMSO control and an unkinase control. The optimal concentrations of PI3Kα, PI3Kα H1047R, substrate (PIP2) and ATP were prepared with buffers. The enzyme reaction system included: buffer, ATP 25µM, kinase substrate (PIP2, 50µg/mL), kinase PI3Kα (0.15µg/mL), PI3Kδ H1047R (0.05µg/mL), etc. The reaction system was reacted at room temperature for 1 hour, then terminated by adding a terminal reagent (ADP-Glo reagent, 5µL), and the ADP content in the system was detected using a detection reagent (Kinase Detection Reagent, 10µL). Signal data was collected using the Envision instrument. Calculated the % inhibition according to the following formula: % inhibition = (DMSO control signal value - sample signal value)/ (DMSO control signal value - unkinase control signal value). Inhibition curves were plotted using the formula Y=Bottom + (Top-Bottom)/(1+(IC50/X)^HillSlope) to obtain IC50 values and the results are as shown in Table 1.

Result + represents IC50 <= 250nM ++ represents 250 nM < IC50 < = 10µM +++ represents IC50 > 10 µM

**Table 1**

| Compound No. | PI3KαH10 47RIC₅₀ | PI3Kα IC₅₀ | Compound No. | PI3KαH1047RI C₅₀ | PI3Kα IC₅₀ |
|---|---|---|---|---|---|
| Compound 1-p1 | + | ++ | Compound 1-p2 | ++ | +++ |
| Compound 3 | + | ++ | Compound 2 | ++ | ++ |
| Compound 5 | + | +++ | Compound 6 | + | ++ |
| Compound 7 | + | ++ | Compound 8 | + | ++ |
| | | | Compound 9-p2 | + | ++ |
| Compound 10 | ++ | ++ | Compound 11-p1 | + | ++ |
| Compound 11-p2 | + | ++ | Compound 12-p1 | ++ | +++ |
| Compound 12-p2 | + | +++ | Compound 13-p1 | ++ | +++ |
| Compound 13-p2 | + | ++ | Compound 14-p1 | + | ++ |
| Compound 14-p2 | + | ++ | Compound 15-p1 | ++ | +++ |
| Compound 15-p2 | + | ++ | Compound 16 | + | ++ |
| Compound 17 | ++ | +++ | Compound 18 | + | ++ |
| Compound 19 | + | ++ | Compound 20 | + | ++ |
| Compound 20-p1 | + | ++ | Compound 20-p2 | ++ | +++ |
| Compound 23-p1 | ++ | +++ | Compound 22 | + | ++ |
| Compound 24 | + | ++ | Compound 23-p2 | + | ++ |
| Compound 25 | + | +++ | Compound 26 | + | ++ |
| Compound 28-p1 | ++ | +++ | Compound 28-p2 | + | ++ |
| Compound 29-p1 | ++ | +++ | Compound 29-p2 | + | ++ |
| Compound 30 | ++ | ++ | Compound 31 | ++ | ++ |
| Compound 32 | + | ++ | Compound 33 | + | ++ |
| Compound 34 | + | ++ | Compound 35 | + | ++ |
| Compound 36 | + | ++ | | | |
| Compound 36-p1 | ++ | +++ | Compound 36-p2 | + | ++ |
| Compound 37 | + | ++ | Compound 43 | ++ | +++ |
| Compound 44 | ++ | +++ | Compound 45 | + | ++ |
| Compound 45-p1 | + | ++ | Compound 45-p2 | ++ | ++ |
| Compound 48-p1 | ++ | +++ | Compound 48-p2 | ++ | ++ |
| Compound 49-p1 | ++ | ++ | Compound 49-p2 | + | ++ |
| Compound 50-p1 | ++ | ++ | Compound 50-p2 | + | ++ |
| Compound 51-p1 | ++ | +++ | Compound 51-p2 | + | ++ |
| Compound 52-p1 | ++ | +++ | Compound 52-p2 | + | ++ |

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A compound with a tricyclic fused ring structure as shown in formula I, a pharmaceutically acceptable salt thereof, a solvate thereof, a solvate of the pharmaceutically acceptable salt thereof, a crystal form thereof, a stereoisomer thereof, a tautomer thereof or an isotopically labeled compound thereof: 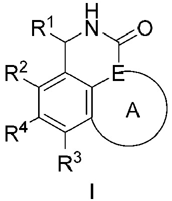 **characterized in that**,
E is C or N;
ring A is a 4- to 10-membered cycloalkene containing 1-3 heteroatoms each independently selected from O, N and S, or a 5- to 12-membered heteroaromatic ring containing 1-3 heteroatoms each independently selected from O, N and S substituted by one or more C₁₋₆ alkyl;
R¹ is phenyl substituted by at least two R¹⁻¹; R¹⁻¹ is independently halogen or C₁₋₆ alkyl;
R² is C₆₋₁₀ aryl substituted by one or more R²⁻¹, or benzofuranyl substituted by one or more R²⁻²; each R²⁻¹ and R²⁻² is independently halogen, C₁₋₆ alkyl, or C₁₋₆ alkyl substituted by one or more halogen;
R³ is 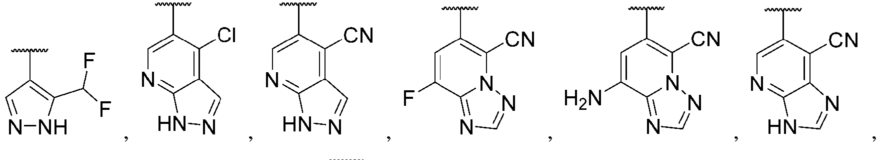 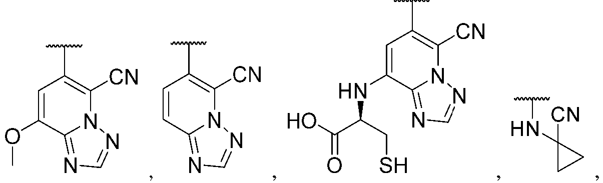 Cl, F, Br, -NH₂, H, 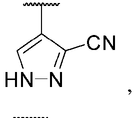 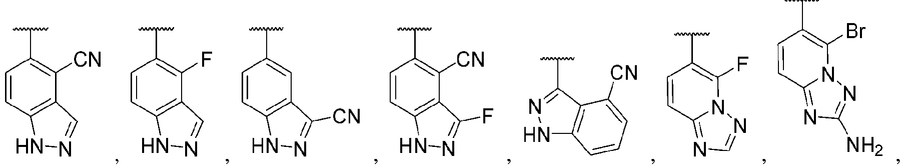 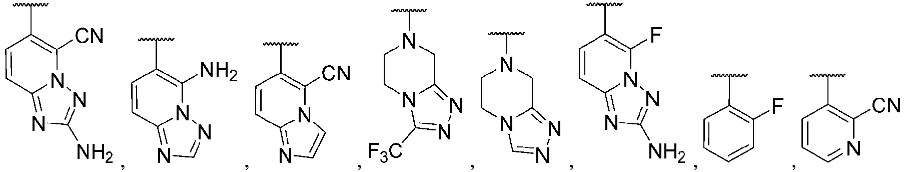 or 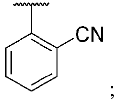
R⁴ is hydrogen or halogen;
said compound with a tricyclic fused ring structure as shown in formula I is not any one of the following compounds:
N-(7-bromo-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
N-(7-amino-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
N-(7-bromo-1-(2-bromo-5-fluorophenyl)-3-oxo-2,3,5,6-tetrahydro-1H-pyrrolo[3,2,1-ij]quinazolin-9-yl)-3-fluoro-5-(trifluoromethyl)benzamide;
N-(7-bromo-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
N-(7-amino-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
N-(7-amino-1-(2-chloro-5-(trifluoromethyl)phenyl)-3-oxo-2,3,5,6-tetrahydro-1H-pyrrolo[3,2,1-ij]quinazolin-9-yl)-3-fluoro-5-(trifluoromethyl)benzamide;
N-(7-amino-1-(2,5-dichloro-4-fluorophenyl)-3-oxo-2,3,5,6-tetrahydro-1H-pyrrolo[3,2,1-ij]quinazolin-9-yl)-3-fluoro-5-(trifluoromethyl)benzamide;
N-(5-(2-chloro-5-fluorophenyl)-2-methyl-3-oxo-2,3,4,5-tetrahydropyrazolo[3,4,5-de]isoquinolin-6-yl)-3-fluoro-5-(trifluoromethyl)benzamide;
N-(5-(2,5-dichlorophenyl)-2-methyl-3-oxo-2,3,4,5-tetrahydropyrazolo[3,4,5-de]isoquinolin-6-yl)-3-fluoro-5-(trifluoromethyl)benzamide;
N-(5-(2-chloro-5-fluorophenyl)-8-(5-cyano-[1,2,4]triazolo[1,5-a]pyridin-6-yl)-2-methyl-3-oxo-2,3,4,5-tetrahydropyrazolo[3,4,5-de]isoquinolin-6-yl)-3-fluoro-5-(trifluoromethyl)benzamide;
N-(8-(5-cyano-[1,2,4]triazolo[1,5-a]pyridin-6-yl)-5-(2,5-dichlorophenyl)-2-methyl-3-oxo-2,3,4,5-tetrahydropyrazolo[3,4,5-de]isoquinolin-6-yl)-3-fluoro-5-(trifluoromethyl)benzamide;
N-(1-(2-chloro-5-fluorophenyl)-7-(5-cyano-[1,2,4]triazolo[1,5-a]pyridin-6-yl)-3-oxo-2,3,5,6-tetrahydro-1H-pyrrolo[3,2,1-ij]quinazolin-9-yl)-3-fluoro-5-(trifluoromethyl)benzamide;
N-(7-(5-cyano-[1,2,4]triazolo[1,5-a]pyridin-6-yl)-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
N-(7-(5-cyano-[1,2,4]triazolo[1,5-a]pyridin-6-yl)-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
N-(1-(2-chloro-5-fluorophenyl)-7-(5-cyano-[1,2,4]triazolo[1,5-a]pyridin-6-yl)-3-oxo-2,3,5,6-tetrahydro-1H-pyrrolo[3,2,1-ij]quinazolin-9-yl)-3-fluoro-5-(trifluoromethyl)benzamide;
N-(5-(2,5-dichloro-4-fluorophenyl)-2-methyl-3-oxo-2,3,4,5-tetrahydropyrazolo[3,4,5-de]isoquinolin-6-yl)-3-fluoro-5-(trifluoromethyl)benzamide;
N-(1-(2-chloro-5-fluorophenyl)-7-(3-cyano-1H-pyrazol-4-yl)-3-oxo-2,3,5,6-tetrahydro-1H-pyrrolo[3,2,1-ij]quinazolin-9-yl)-3-fluoro-5-(trifluoromethyl)benzamide;
N-(1-(2-chloro-5-fluorophenyl)-7-(5-fluoro-[1,2,4]triazolo[1,5-a]pyridin-6-yl)-3-oxo-2,3,5,6-tetrahydro-1H-pyrrolo[3,2,1-ij]quinazolin-9-yl)-3-fluoro-5-(trifluoromethyl)benzamide;
N-(7-(2-cyanopyridin-3-yl)-1-(2,5-dichlorophenyl)-3-oxo-2,3,5,6-tetrahydro-1H-pyrrolo[3,2,1-ij]quinazolin-9-yl)-3-fluoro-5-(trifluoromethyl)benzamide;
N-(1-(2-chloro-5-fluorophenyl)-7-(2-cyanopyridin-3-yl)-3-oxo-2,3,5,6-tetrahydro-1H-pyrrolo[3,2,1-ij]quinazolin-9-yl)-3-fluoro-5-(trifluoromethyl)benzamide;
N-(7-(5-amino-[1,2,4]triazolo[1,5-a]pyridin-6-yl)-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
N-(1-(2-chloro-5-fluorophenyl)-7-(3-cyano-1H-pyrazol-4-yl)-3-oxo-2,3,5,6-tetrahydro-1H-pyrrolo[3,2,1-ij]quinazolin-9-yl)-3-fluoro-5-(trifluoromethyl)benzamide;
N-(7-(5-amino-[1,2,4]triazolo[1,5-a]pyridin-6-yl)-1-(2-chloro-5-fluorophenyl)-3-oxo-2,3,5,6-tetrahydro-1H-pyrrolo[3,2,1-ij]quinazolin-9-yl)-3-fluoro-5-(trifluoromethyl)benzamide;
N-(1-(2-chloro-5-fluorophenyl)-7-(4-cyano-1H-indazol-5-yl)-3-oxo-2,3,5,6-tetrahydro-1H-pyrrolo[3,2,1-ij]quinazolin-9-yl)-3-fluoro-5-(trifluoromethyl)benzamide.

2. The compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that** the structure of the compound with a tricyclic fused ring structure as shown in formula I is as shown in formula II: 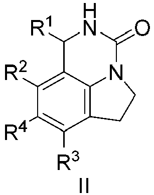 wherein,
R¹ is phenyl substituted with at least two R¹⁻¹; R¹⁻¹ is independently halogen or C₁₋₆ alkyl;
R² is C₆₋₁₀ aryl substituted by one or more R²⁻¹, or benzofuranyl substituted by one or more R²⁻²; each R²⁻¹ and R²⁻² is independently halogen, C₁₋₆ alkyl, or C₁₋₆ alkyl substituted by one or more halogen;
R³ is 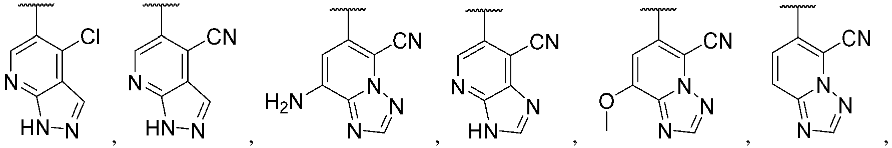 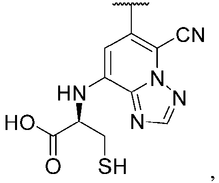 F, Br, -NH₂, 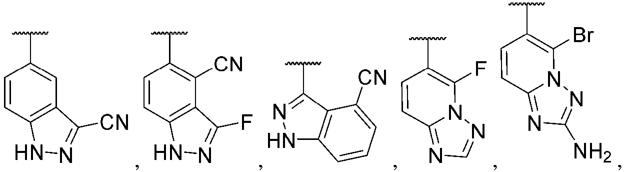 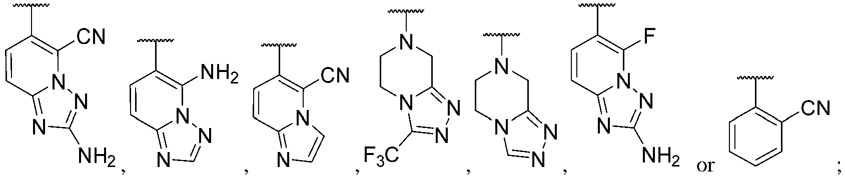
R⁴ is hydrogen or halogen.

3. The compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that** the structure of the compound with a tricyclic fused ring structure as shown in formula I is as shown in formula III: 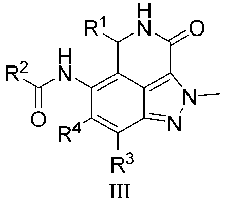 wherein,
R¹ is phenyl substituted by at least two R¹⁻¹; R¹⁻¹ is independently halogen or C₁₋₆ alkyl;
R² is C₆₋₁₀ aryl substituted by one or more R²⁻¹, or benzofuranyl substituted by one or more R²⁻²; each R²⁻¹ and R²⁻² is independently halogen, C₁₋₆ alkyl, or C₁₋₆ alkyl substituted by one or more halogen;
R³ is 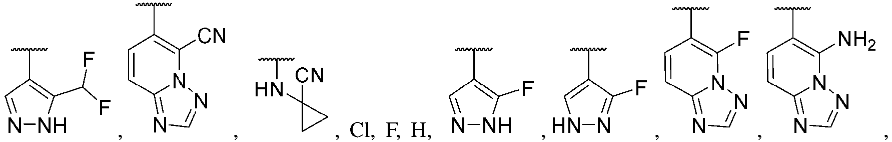 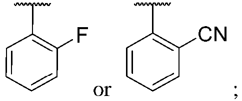
R⁴ is hydrogen or halogen.

4. The compound with a tricyclic fused ring structure as shown in formula **I,** the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that**,
when the definition of R¹⁻¹, R²⁻¹, R²⁻² and R⁴ refers to "halogen", said halogen is fluorine, chlorine, bromine or iodine;
and/or, when the definition of ring A, R¹⁻¹, R²⁻¹ and R²⁻² refers to "C₁₋₆ alkyl", said C₁₋₆ alkyl is C₁₋₄ alkyl, further selected from methyl, ethyl, n-propyl, isopropyl, n-butyl, sec-butyl, isobutyl or tert-butyl;
and/or, when ring A is "a 4- to 10-membered cycloalkene containing 1-3 heteroatoms each independently selected from O, N and S", said ring A is "a 4- to 6-membered cycloalkene containing 1-3 N heteroatoms", for example, "a 5-membered cycloalkene containing one N heteroatom";
and/or, when ring A is "a 5- to 12-membered heteroaromatic ring containing 1-3 heteroatoms each independently selected from O, N and S" substituted by one or more C₁₋₆ alkyl, said ring A is "a 5- to 6-membered heteroaromatic ring containing 1-3 heteroatoms each independently selected from O, N and S" substituted by one or more C₁₋₆ alkyl.

5. The compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that**,
R¹ is 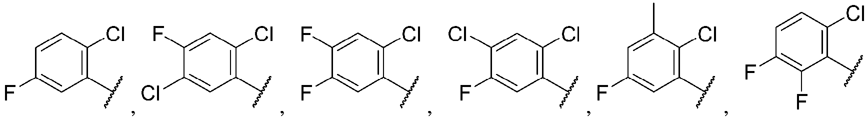 or 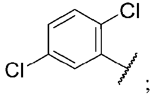
and/or, R² is 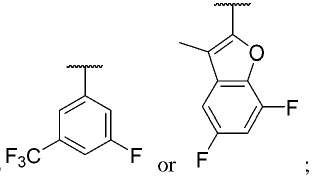
and/or, R⁴ is hydrogen or fluorine.

6. The compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that** said compound with a tricyclic fused ring structure as shown in formula I is any one of the following structures:

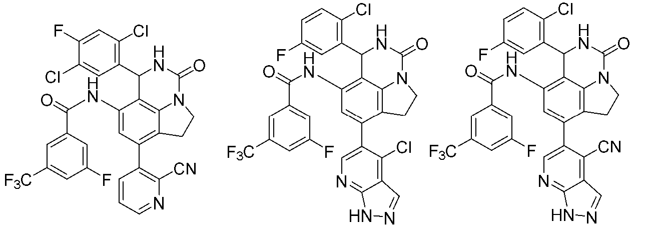

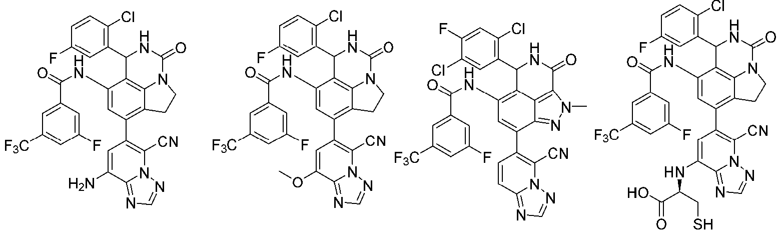

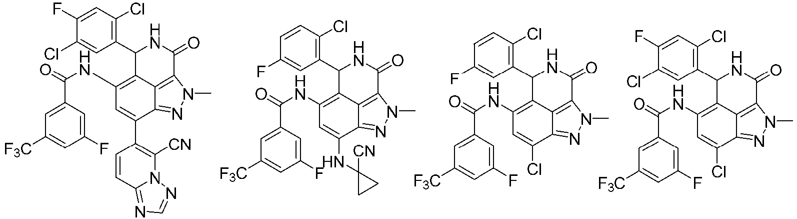

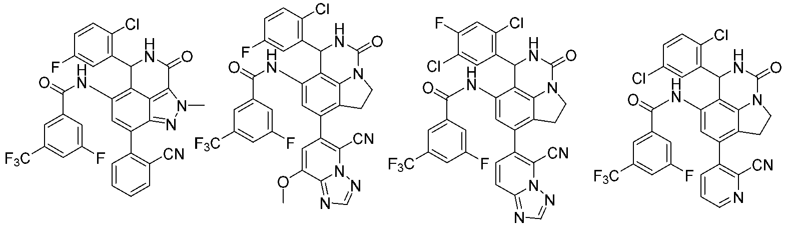

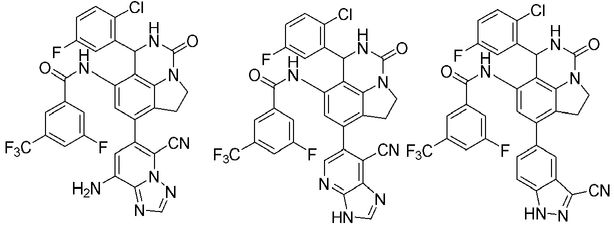

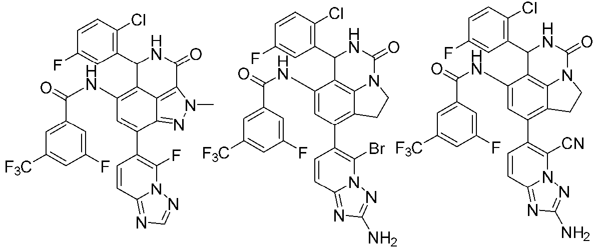

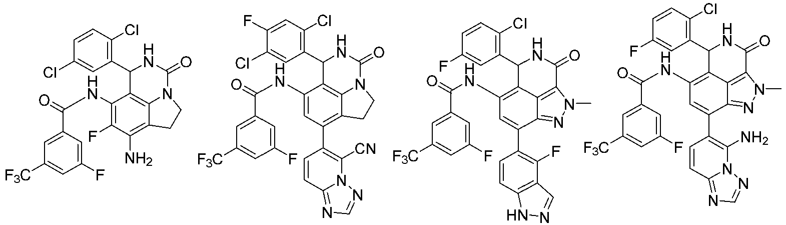

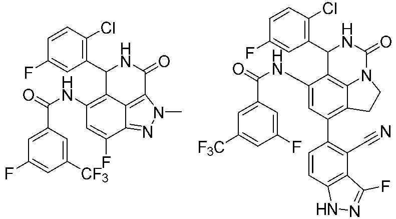

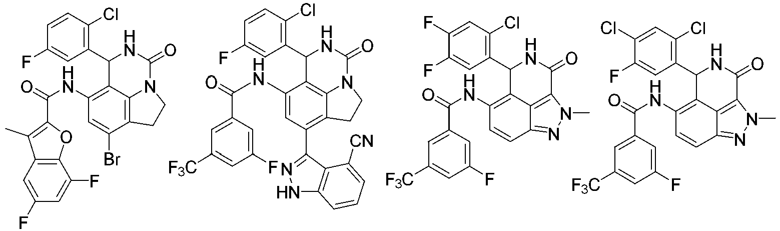

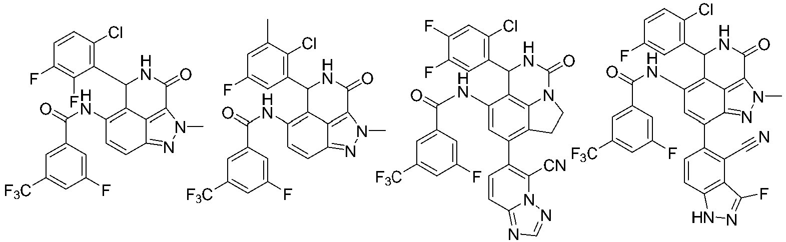

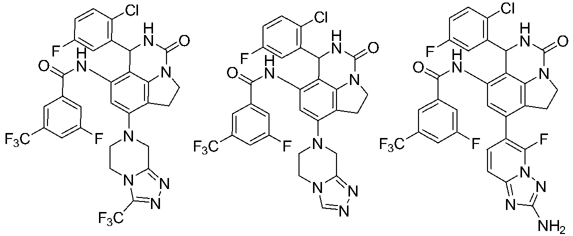

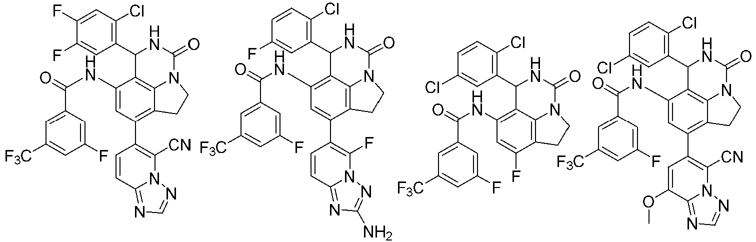

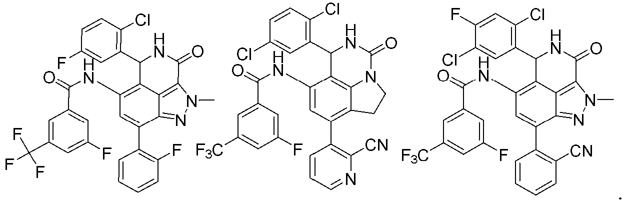

7. The compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that** said compound with a tricyclic fused ring structure as shown in formula I is any one of the following structures:
compound 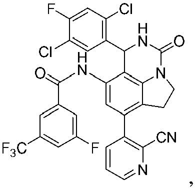 which has a retention time of 0.693 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 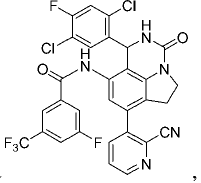 which has a retention time of 1.326 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; detection wavelength: 214 nm;
compound 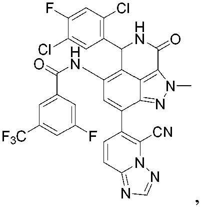 which has a retention time of 3.379 min under the following conditions: instrument: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 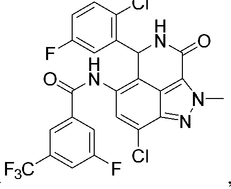 which has a retention time of 1.543 min under the following conditions: instrument: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 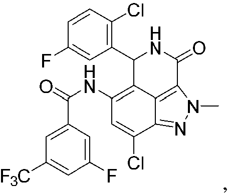 which has a retention time of 3.246 min under the following conditions: instrument: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 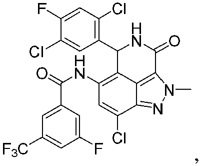 which has a retention time of 3.833 min under the following conditions: instrument: SFC-150 (Waters); chromatographic column: 0.46 cm I.D.*5 cm L; mobile phase: CO₂/EtOH = 70/30; flow rate: 2.5 mL/min; back pressure: 2000 psi; detection wavelength: 254 nm;
compound 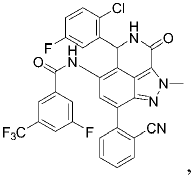 which has a retention time of 0.987 min under the following conditions: instrument: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 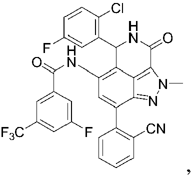 which has a retention time of 3.033 min under the following conditions: instrument: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 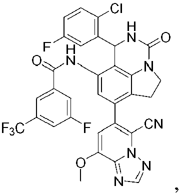 which has a retention time of 1.235 min under the following conditions: instrument: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 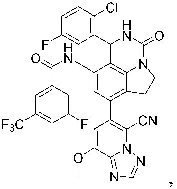 which has a retention time of 4.026 min under the following conditions: instrument: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 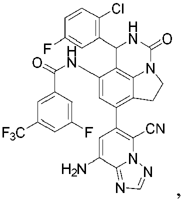 which has a retention time of 3.381 min under the following conditions: instrument: SFC-150 (Waters); chromatographic column: 0.46 cm I, D.*5 cm L; mobile phase: CO₂/EtOH = 70/30; flow rate: 2.5 mL/min; back pressure: 2000 psi; detection wavelength: 254 nm;
compound 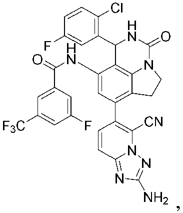 which has a retention time of 1.35 min under the following conditions: instrument: SFC-150 (Waters); chromatographic column: (R, R) Whelk-O1 4.6*100 mm 5 µm; mobile phase: CO₂/EtOH = 70/30; flow rate: 3.0 mL/min; back pressure: 2000 psi; detection wavelength: 254 nm;
compound 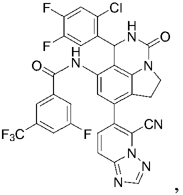 which has a retention time of 2.617 min under the following conditions: instrument: SFC-150 (Waters); chromatographic column: (R, R)Whelk-O1 4.6*100mm 5µm; mobile phase: CO₂/EtOH = 70/30; flow rate: 2.5 mL/min; back pressure: 100 bar; detection wavelength: 254 nm;
compound 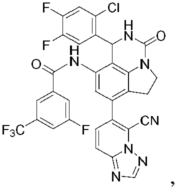 which has a retention time of 3.677 min under the following conditions: instrument: AD-3, 0.46 cm I.D. * 5 cm. L; mobile phase: CO₂/EtOH = 70/30; flow rate: 2.5 mL/min; back pressure: 100 bar; detection wavelength: 254 nm;
compound 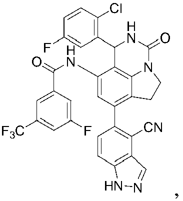 which has a retention time of 1.344 min under the following conditions: instrument: SFC-150 (Waters); chromatographic column: AD-3 0.46 cm I.D. * 5 cm. L; mobile phase: CO₂/EtOH = 70/30; flow rate: 2.5 mL/min; back pressure: 100 bar; detection wavelength: 254 nm;
compound 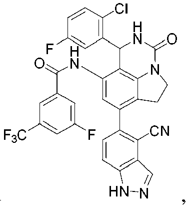 which has a retention time of 3.123 min under the following conditions: instrument: SFC-150 (Waters); chromatographic column: AD-3 0.46 cm I.D. * 5 cm. L; mobile phase: CO₂/EtOH = 70/30; flow rate: 2.5 mL/min; back pressure: 100 bar; detection wavelength: 254 nm;
compound 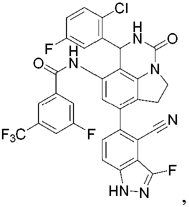 which has a retention time of 2.456 min under the following conditions: instrument: SFC-150 (Waters); chromatographic column: AD-3 0.46 cm I.D.* 5 cm. L; mobile phase: CO₂/EtOH = 70/30; flow rate: 2.5 mL/min; back pressure: 100 bar; detection wavelength: 254 nm;
compound 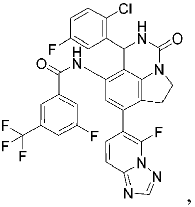 which has a retention time of 1.486 min under the following conditions: instrument: AD-3 0.46 cm I.D.* 5 cm. L; mobile phase: MeOH [0.2%NH₃(7M in MeOH)]; flow rate: 2.5 mL/min; back pressure: 100 bar; detection wavelength: 254 nm;
compound 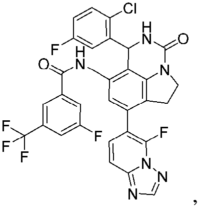 which has a retention time of 3.136 min under the following conditions: instrument: AD-3 0.46cm I.D.* 5cm. L; mobile phase: MeOH [0.2%NH₃(7M in MeOH)]; flow rate: 2.5 mL/min; back pressure: 100 bar; detection wavelength: 254 nm;
Compound 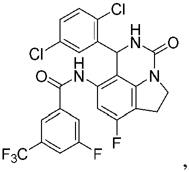 which has a retention time of 1.305 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; wavelength: 214 nm;
compound 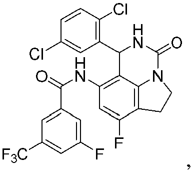 which has a retention time of 1.712 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; wavelength: 214 nm;
compound 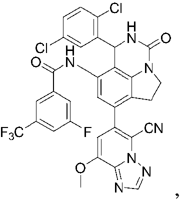 which has a retention time of 0.938 min under the following conditions: instrument: SFC-150 (Waters); chromatographic column: OD-3 4.6*100 mm 3µm; mobile phase: MeOH[0.2%NH₃(7M in MeOH)]; flow rate: 3.0 mL/min; back pressure: 2000 psi; detection wavelength: 254 nm;
compound 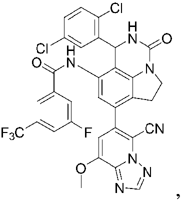 which has a retention time of 1.306 min under the following conditions: instrument: SFC-150 (Waters); chromatographic column: OD-3 4.6*100 mm 3µm; mobile phase: MeOH[0.2%NH₃(7M in MeOH)]; flow rate: 3.0 mL/min; back pressure: 2000 psi; detection wavelength: 254 nm;
compound 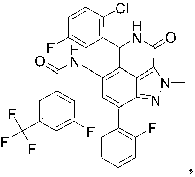 which has a retention time of 2.002 min under the following conditions: instrument: SFC-150 (Waters); chromatographic column: AD-3; mobile phase: CO₂/EtOH = 70/30; flow rate: 2.5 mL/min; back pressure: 2000 psi; wavelength: 254 nm;
compound 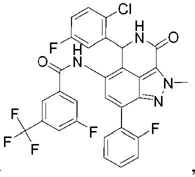 which has a retention time of 3.317 min under the following conditions: instrument: SFC-150 (Waters); chromatographic column: AD-3; mobile phase: CO₂/EtOH = 70/30; flow rate: 2.5 mL/min; back pressure: 2000 psi; wavelength: 254 nm;
compound 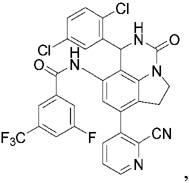 which has a retention time of 2.387 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; wavelength: 214 nm;
compound 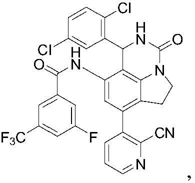 which has a retention time of 4.086 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; wavelength: 214 nm;
Compound 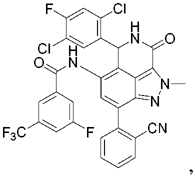 which has a retention time of 2.223 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; wavelength: 214 nm;
Compound 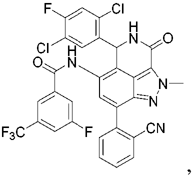 which has a retention time of 3.679 min under the following conditions: instrument: SFC-150 (Waters); chromatographic column: AD-3; mobile phase: CO₂/MeOH[0.2%NH₃(7M in MeOH)] = 30/70; flow rate: 3.0 mL/min; back pressure: 2000 psi; wavelength: 214 nm.

8. The compound with a tricyclic fused ring structure as shown in formula I, the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to claim 1, **characterized in that** said compound with a tricyclic fused ring structure as shown in formula I is any one of the following structures:

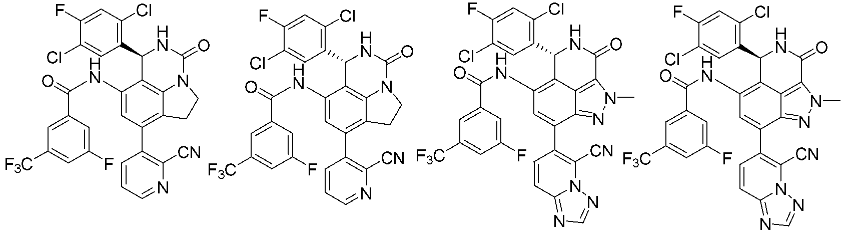

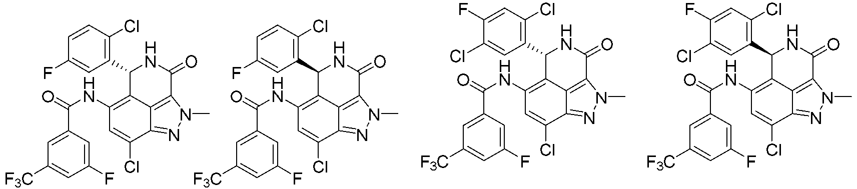

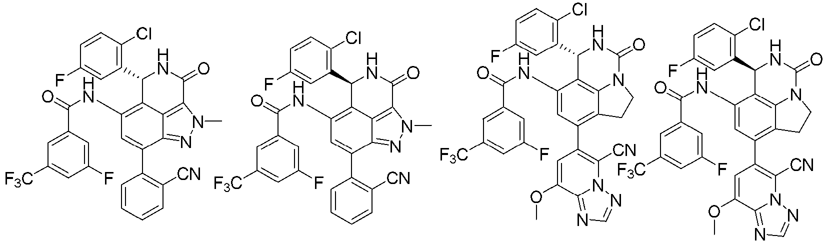

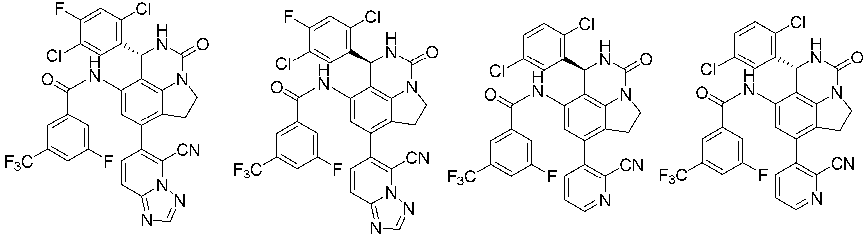

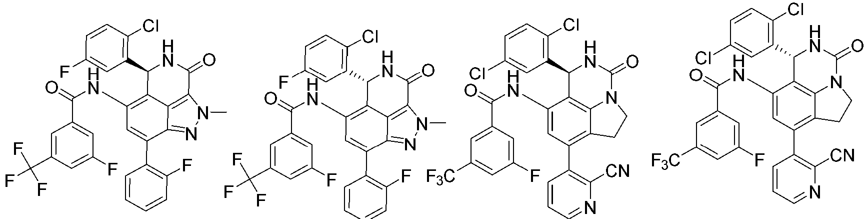

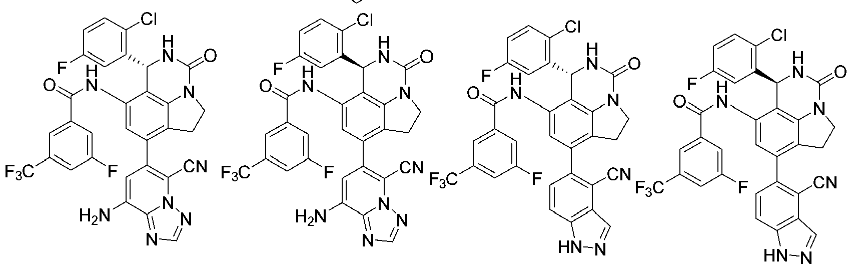

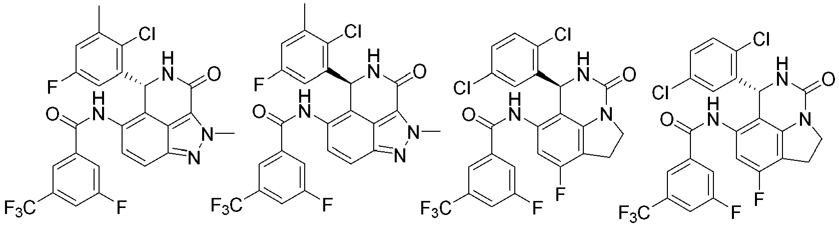

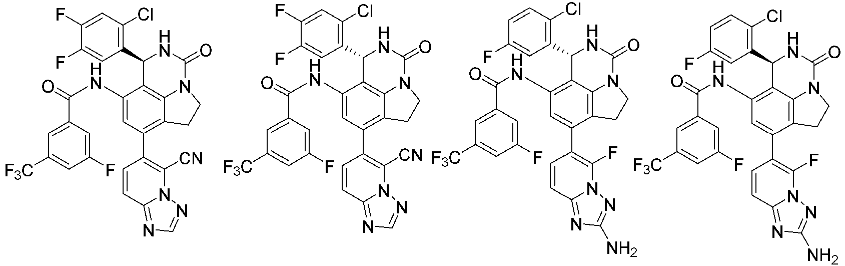

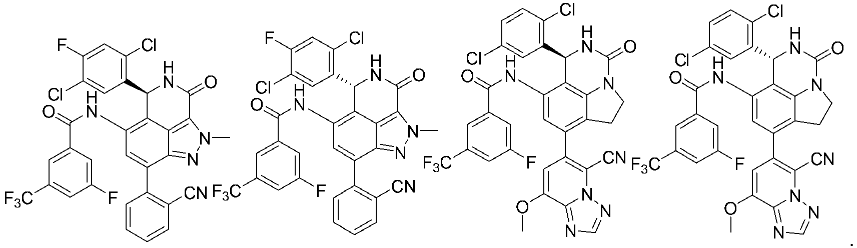

9. A pharmaceutical composition, comprising substance A and pharmaceutical adjuvant, said substance A is the therapeutically effective amount of the compound with a tricyclic fused ring structure as shown in formula **I,** the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to any one of claims 1-8.

10. Use of a substance A in preparation of a PI3K inhibitor, wherein said substance A is the compound with a tricyclic fused ring structure as shown in formula **I,** the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to any one of claims 1-8.

11. Use of a substance A in the preparation of a medicament for treating or preventing a PI3K-mediated disease; wherein said substance A is the compound with a tricyclic fused ring structure as shown in formula **I,** the pharmaceutically acceptable salt thereof, the solvate thereof, the solvate of the pharmaceutically acceptable salt thereof, the crystal form thereof, the stereoisomer thereof, the tautomer thereof or the isotopically labeled compound thereof according to any one of claims 1-8.
